(19) Europäisches Patentamt
European Patent Office
Office européen des brevets

(11) Veröffentlichungsnummer: **0 301 394 B1**

(12) # EUROPÄISCHE PATENTSCHRIFT

(45) Veröffentlichungstag der Patentschrift: **06.10.93**

(21) Anmeldenummer: **88111670.1**

(22) Anmeldetag: **20.07.88**

Die Akte enthält technische Angaben, die nach dem Eingang der Anmeldung eingereicht wurden und die nicht in dieser Patentschrift enthalten sind.

(51) Int. Cl.5: **C07D 498/04**, C07D 205/08, C07D 403/12, A61K 31/42, //(C07D498/04,263:00,205:00)

(54) **Stabile Oxapenem- 3-carbonsäuren.**

(30) Priorität: **31.07.87 DE 3725375**

(43) Veröffentlichungstag der Anmeldung: **01.02.89 Patentblatt 89/05**

(45) Bekanntmachung des Hinweises auf die Patenterteilung: **06.10.93 Patentblatt 93/40**

(84) Benannte Vertragsstaaten: **AT BE CH DE ES FR GB GR IT LI NL SE**

(56) Entgegenhaltungen:
**EP-A- 0 018 305**
**DE-A- 2 747 350**
**DE-A- 2 808 116**

(73) Patentinhaber: **Pfaendler, Hans Rudolf, Prof. Dr.**
**Pippinplatz 1**
**D-81475 München(DE)**

(72) Erfinder: **Pfändler, Hans Rudolf, Prof. Dr.**
**Pippinplatz 1**
**D-8000 München 71(DE)**
Erfinder: **Hendel, Wolfram, Dr.**
**Koblenzer Strasse 6**
**D-5000 Köln 51(DE)**

(74) Vertreter: **Dänner, Klaus, Dr.**
**Bayer AG**
**Konzernverwaltung RP**
**Patente Konzern**
**D-51368 Leverkusen (DE)**

EP 0 301 394 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.6/3.3.1)

## Beschreibung

Die Erfindung betrifft 1-Oxapenem-3-carbonsäuren der folgenden Strukturen

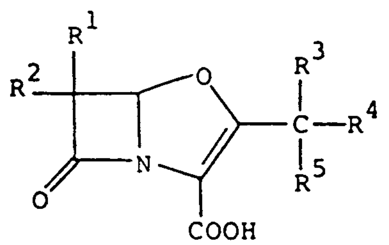

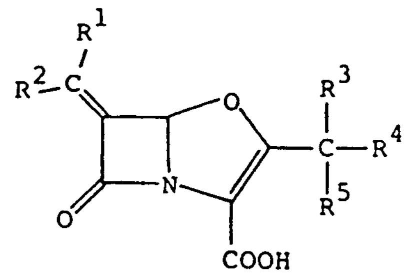

in der $R^1$ und $R^2$ unabhängig voneinander Wasserstoff oder via Kohlenstoffatome gebundene pharmazeutisch annehmbare Gruppen bedeuten und $R^3$, $R^4$ und $R^5$ unabhängig voneinander pharmazeutisch annehmbare Gruppen bedeuten, die via Kohlenstoffatome an das exocyclische, allylische Kohlenstoffatom gebunden sind. Diese Verbindungen wie auch ihre pharmazeutisch annehmbaren Salze und Ester sind nützliche Antibiotika. Gegenstand der Erfindung sind weiterhin Verfahren zur Herstellung solcher Verbindungen, diese Verbindungen enthaltende pharmazeutische Zubereitungen und Behandlungsverfahren, bei denen diese Verbindungen und Zubereitungen verabreicht werden, wenn eine antibiotische Wirkung indiziert ist.

Die Erfindung betrifft 6-unsubstituierte, 6-mono oder 6,6-disubstituierte 1-Oxapen-2-em-3-carbonsäuren, die in 2-Stellung mit besonderen Resten versehen sind. Diese Reste sind dadurch gekennzeichnet, dass sie ein zentrales Kohlenstoffatom besitzen, welches direkt an den Oxapenem-Nucleus gebunden ist und welches drei weitere, über C-Atome verbundene Gruppen gebunden hält. Diese Verbindungen sind nützliche Antibiotika und sie können durch die allgemeinen Strukturformeln

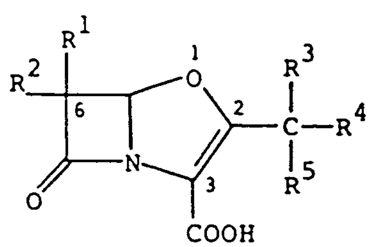

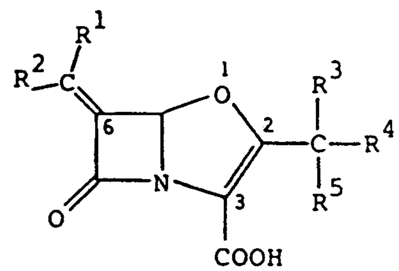

dargestellt werden, in der $R^1$ und $R^2$ unabhängig voneinander Wasserstoff oder über Kohlenstoff-Kohlenstoff-Einfachbindungen mit dem übrigen Molekülteil gebundene pharmazeutisch annehmbare Gruppen bedeuten, die ausgewählt werden aus substituiertem oder unsubstituiertem Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl-, Alkenyl- oder Alkinyl-Molelülteile 1 bis 6 Kohlenstoffatome, die Cycloalkyl-oder die Cycloalkenyl-Molekülteile 3 bis 6 und die Aryl-Molekülteile 6 bis 10 Kohlenstoffatome enthalten, Heteroaralkyl, Heteroaralkenyl, Heteroaralkinyl, Alkylheteroaryl, Heterocyclyl, Heterocyclylalkyl, Heterocyclylalkenyl, Heterocyclalkinyl, Alkylheterocyclyl, worin die vorhergehenden Alkyl-, Alkenyl- oder die Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können:
Hydroxy, Hydroxyalkyloxy, Aminoalkyloxy, Amidinoalkyloxy, Alkyloxy, Acyloxy, Aryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, Amino oder Monoalkylamino, Dialkylamino, Oxo, Oximino oder Alkylimino, Tetraalkylammonium, Cycloalkylamino, Arylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino,

Nitro, Chlor, Brom, Fluor, Iod, Azido, Cyano, Alkylsulfinyl, Alkylsulfonyl, Sulfonamido, Sulfamoyloxy, Alkylsulfonyloxy oder Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und dadurch gekennzeichnet, dass $R^3$, $R^4$ und $R^5$ unabhängig voneinander ausgewählt werden aus den vorhergehend genannten, über Kohlenstoff-Kohlenstoff-Einfachbindungen an den übrigen Molekülteil gebundenen pharmazeutisch annehmbaren Gruppen.

Die Schutzgruppen der oben erwähnten, geschützten Substituenten sind an sich bekannte, leicht entfernbare Reste, wie sie üblicherweise in der organischen Synthese zu diesen Zweck Verwendung finden. Solche Schutzgruppen finden sich beispielsweise in T. W. Greene, "Protective Groups in Organic Synthesis", Wiley, New York, 1981.

Weiterhin können zwei der Gruppen $R^3$, $R^4$ oder $R^5$ über Kohlenstoff, Sauerstoff, Stickstoff und Schwefel enthaltende Molekülteile miteinander verbrückt sein; sie sind dann Bestandteil eines carbocyclischen oder heterocyclischen Ringes, der drei-, vier-, fünf- oder sechsgliedrig sein kann.

Weiterhin können die beiden Gruppen $R^1$ und $R^2$ über Kohlenstoff, Sauerstoff, Stickstoff und Schwefel enthaltende Molekülteile miteinander verbrückt sein; sie sind dann Bestandteil eines drei-, vier-, fünf- oder sechsgliedrigen carbo- oder heterocyclischen Ringes.

Beispiele für verbrückende Molekülteile für $R^1$ und $R^2$ bzw. für $R^3$ und $R^4$ sind Methylen, Dimethylen, Trimethylen, Tetramethylen, Oxamethylen, Oxadimethylen, Dioxamethylen, Azadimethylen, Diazamethylen o. ä.

Pharmazeutisch annehmbare Gruppen $R^1$, $R^2$, $R^3$, $R^4$ und $R^5$, die über C-C-Einfachbindungen gebunden sind, sind Gruppen, wie sie beispielsweise bei den $\beta$-Lactamantibiotika üblich sind. Solche Gruppen findet man z. B. in M.L. Sassiver, A. Lewis in "Advances in Applied Microbiology, ed. D. Perlman, Academic Press, N. Y. (1970).

Die Erfindung betrifft weiterhin die pharmazeutisch annehmbaren Salze und Ester der erfindungsgemäßen Verbindungen (1) und (11).

Die Erfindung betrifft weiterhin Verfahren zur Herstellung solcher Verbindungen (I) und (II), solche Verbindungen enthaltende pharmazeutische Zubereitungen und Verfahren zur Behandlung, bei denen diese Verbindungen und Zubereitungen verabreicht werden, wenn eine antibiotische Wirkung indiziert ist.

Es besteht ein kontinuierlicher Bedarf an neuen Antibiotika. Unglücklicherweise gibt es bei irgendeinem gegebenen Antibiotikum keine statische Wirksamkeit, da bei der kontinuierlichen Verwendung im grossen Massstab selektiv resistente Stämme von pathogenen Bakterien neu gebildet werden. Dementsprechend geht die Suche nach neuen Antibiotika weiter.

Neben den klassischen $\beta$-Lactamantibiotika, d.h. den Penicillinen und Cephalosporinen werden heute auch die sogenannten nichtklassischen oder nichttraditionellen $\beta$-Lactamantibiotika gegen bakterielle Infektionskrankheiten eingesetzt. Die wichtigsten heute angewandten Verbindungen dieses Typs sind die Peneme und die Carbapeneme. Ein kürzlich erschienenes Buch behandelt die Synthese und Pharmakologie dieser neuen Wirkstoffe: Chemistry and Biology of $\beta$-Lactam Antibiotics, Vol. 2 (Nontraditional $\beta$-Lactam Antibiotics), ed. by R. B. Morin and M. Gorman, Academic Press, New York (1982).

Auf Grund der engen strukturellen Verwandtschaft der Oxapenemcarbonsäuren mit den schwefelhaltigen Penemcarbonsäuren oder mit den Carbapenemcarbonsäuren konnte vermutet werden, dass Oxapenem-3-carbonsäuren auch antibakteriell wirksam seien. (Tetrahedron 38 (16) 2489-2504 (1982), Seite 2489).

Obwohl eine antibakterielle Wirksamkeit von Oxapenem-3-carbonsäuren erwähnt wurde, z.B. in US 4, 172, 895 oder EP-A-0 018 305 (80710008.6), ist sie niemals durch experimentelle Daten belegt worden. Die einzigen verfügbaren Messdaten über deren antibakterielle Wirksamkeit finden sich in "Chemistry and Biology of $\beta$-Lactam Antibiotics Vol. 2 Nontraditional $\beta$-Lactam Antibiotics" ed. by R. B. Morin and M. Gorman, Seite 383:

"(Das Kaliumsalz von 2-Ethyl-1-oxapenem-carbonsäure) war zu instabil für die Prüfung der antibakteriellen Aktivität oder des Synergismus mit Ampicillin gegenüber intakten Bakterien."

Eine in früheren Patentanmeldungen als wirksam vorgestellte Verbindung, die 2-Ethyl-1-oxapen-2-em-3-carbonsäure,war also in Wirklichkeit viel zu wenig stabil in wässrigem Medium für eine antibakterielle Prüfung und damit als Antibiotikum praktisch unwirksam. Lediglich eine Hemmung von isolierten bakteriellen Enzymen ($\beta$-Laktamasen) war nachweisbar.

Die Instabilität früher bekanntgewordener Oxapenem-3-carbonsäuren, auch Clavem-carbonsäuren genannt, äusserte sich auch bei der Herstellung der Methylester z.B. in J.C.S. Chem. Commun. 1977, 720. Auch diese waren instabil.

Die geringe Bedeutung der antibakteriell praktisch unwirksamen oder gering wirksamen Oxapenem-3-carbonsäuren mag man auch daran ermessen, dass ihnen in einem 402 Seiten umfassenden Buch über nichtklassische β-Laktamantibiotika (Chemistry and Biology of β-Lactam Antibiotics, Vol 2, ed. by R.B. Morin and M. Gorman Academic Press, New York 1982) lediglich 5 Seiten gewidmet sind (Seiten 381-385).

Noch viel geringeres Interesse wurde den Oxapenem-3-carbonsäuren in den folgenden Jahren (1982-1986) entgegengebracht, was durch eine vollständige Literatursuche in Chemical Abstracts bestätigt wurde. Unter dem systematischen Namen 4-Oxa-1-azabicyclo[3.2.0]hept-2-en-2-carbonsäure wurde gefunden, dass die Forschung auf diesem Gebiet ständig abnahm: 1977: 3, 1978: 9, 1979: 2, 1980: 6, 1981: 9, 1982: 2, 1983: 5, 1984: 2, 1985: 0, 1986: 0 Publikationen. Die Oxapenem-3-carbonsäuren waren also wegen ihrer geringen Stabilität und wegen ihrer geringen antibakteriellen Wirksamkeit für die Fachwelt uninteressant geworden. Dieses geringe Interesse an den Oxapenem-3-carbonsäuren verglichen mit demjenigen an anderen nichtklassischen β-Laktamantibiotika zeigt, dass ein Vorurteil der Fachwelt gegen die Brauchbarkeit und Wirksamkeit der Stoffklasse der Oxapenem-3-carbonsäuren z. Zt. besteht.

Die Stabilität von β-Laktamantibiotika ist seit jeher ein zentrales Problem dieser Wirkstoffklasse. So sind beispielsweise im Zweiten Weltkrieg hunderttausende von Soldaten an Wundinfektionen gestorben, weil wegen der Instabilität des Penicillins nicht genügend Material hergestellt werden konnte, um die Erkrankten zu heilen. Erst später mit der Entdeckung der stabileren, kristallinen Penicilline (Penicillin V und Penicillin G) gelang die Produktion aus Schimmelpilzen im Tausend-Tonnen-Massstab.

Auch bei den nichtklassischen β-Laktamantibiotika spielt die Stabilität eine wichtige Rolle: Thienamycin, das z. Zt. wirksamste natürliche Antibiotikum "in vitro" ist sehr hydrolyseempfindlich und deshalb als Therapeutikum nicht anwendbar. Später erst ist ein geeignetes stabileres Derivat (Formiminothienamycin = MK-0787) hergestellt worden (Lit.: Recent Advances in the Chemistry of β-Lactam Antibiotics ed. by G.I. Gregory, The Royal Society, London, Seite 249 (1981)).

Die herkömmlichen Oxapenem-3-carbonsäuren sind sehr instabile Stoffe. Es bestand deshalb ein Bedarf auch in dieser Stoffklasse, stabile Derivate herzustellen mit stark verbesserter antibakterieller Wirkung, die sich im wässrigen Medium lange genug halten, damit sie den Wirkungsort unzersetzt erreichen, um die pathogenen Bakterien abzutöten.

Es wurde nun gefunden, dass Oxapenem-3-carbonsäuren der Formeln I und II viel stabiler sind als die früher bekanntgewordenen Verbindungen. Exakte Messungen bei physiologischen Bedingungen, d.h. im wässrigen Phosphatpuffer bei pH 7.4 und 37 °C mithilfe der Uv-Spektroskopie zeigten eine überraschende Abhängigkeit der Stabilität der Verbindungen III von den Substituenten $R^1$, $R^2$, $R^3$, $R^4$, und $R^5$.

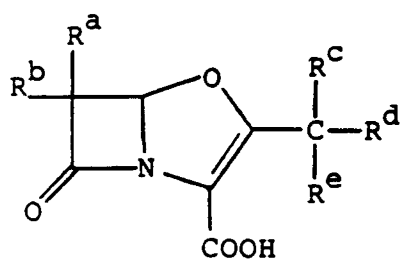

III

| Verbindung (III) | | Hydrolyse-Halbwertszeit bei pH 7.4, 37 °C (Mass für die Stabilität) |
|---|---|---|
| (a) | $R^a$, $R^b$, $R^c$, $R^d$, $R^e$ = $CH_3$ | 30 Stunden |
| (b) | $R^a$, $R^b$, $R^c$, $R^d$ = $CH_3$; $R^e$ = H | 2 Stunden |
| (c) | $R^a$, $R^b$, $R^c$ = $CH_3$; $R^d$, $R^e$ = H | 70 Minuten |
| (d) | $R^a$, $R^b$ = $CH_3$; $R^c$, $R^d$, $R^e$ = H | 50 Minuten |
| (e) | $R^a$, $R^b$, $R^c$, $R^d$, $R^e$ = H | wenige Minuten |

Verbindung IIIa ist identisch mit I ($R^1$, $R^2$, $R^3$, $R^4$, $R^5$ = $CH_3$).

Mit diesen Messungen wird erstmals bewiesen, dass über Kohlenstoff gebundene Gruppen $R^c$, $R^d$, $R^e$ eine bedeutende Stabilisierung der Oxapenem-3-carbonsäuren bewirken. Schon eine einzige Gruppe $R^c$, $R^d$ oder $R^e$ = H führt zu einer drastischen Erniedrigung der Stabilität!

Die in früheren Patentanmeldungen (z.B. in EP 18305) als bevorzugt bezeichnete Verbindung IIIe hydrolysiert in wenigen Minuten und könnte durch die Blutbahn (pH 7.4, 37 °C) niemals effizient an den Wirkort unbeschadet transportiert werden. Aber auch in vitro ist IIIe wegen sofortiger Hydrolyse antibakteriell praktisch unwirksam. Bei Verwendung von Staphylococcus aureus DSM 1104 wurde nach Auftragen von 250 µg IIIe nur ein Hemmhof von wenigen mm festgestellt im Agar-Diffusionstest.

Es wurde weiter gefunden, dass die Verbindungen der Formeln I und II eine hohe Wirksamkeit gegen Staphylococcus aureus besitzen. Gewisse Vertreter sind ebenso wirksam gegen grampositive wie gegen gramnegative Keime und resistente Bakterien. So ergibt die Verbindung (I) ($R^1$, $R^2$ = H; $R^3$, $R^4$, $R^5$ = $CH_3$), die sich vom antibakteriell praktisch unwirksamen IIIe nur durch den Mehrbesitz von drei Methylgruppen auszeichnet, folgende Hemmhof-Durchmesser nach Auftragen von 200 µg Substanz im Agar-Diffusionstest:

| | |
|---|---|
| Staph. aureus DSM 1104 | 45 mm |
| Staph. aureus 012484/77 (Penicillin- und Cephalosporin-resistent) | 47 mm |
| Escherichia coli DSM 1103 | 41 mm |

Durch geeignete Substitution konnte die Wirksamkeit gegenüber gewissen Keimen erheblich gesteigert werden. So zeigt beispielsweise die Verbindung (I) ($R^1$ = H; $R^2$ = $CH_2OH$; $R^3$, $R^4$, $R^5$ = $CH_3$) Hemmhof-Durchmesser nach Auftragen von nur 10 µg Substanz:

| | |
|---|---|
| Staph. aureus DSM 1104 | 30 mm |
| Staph. aureus 012484/77 | 32 mm |
| Escherichia coli DSM 1103 | 30 mm |
| Escherichia coli W 3110 R6K (TEM 1) (β-lactamase bildend) | 29 mm |

Die obigen Daten zeigen, dass aufgrund der erfindungsmässigen Verbindungen die bisher als antibakteriell praktisch unwirksam und folglich als uninteressant betrachtete Klasse der Oxapenem-3-carbonsäuren erstmals unter die wirksamsten antibakteriellen Mittel überhaupt aufrückt. Penicillin V (130 µg) zeigte nur eine starke Hemmwirkung gegen Staph. aureus 1104 (42 mm) und eine minimale Wirkung gegen E. coli DSM 1103 (13 mm). Die beiden anderen Keime wurden nicht gehemmt. Vergleichbare Daten über die antibakterielle Wirksamkeit des natürlichen Antibiotikums Thienamycin finden sich in Journ. Amer. Chem. Soc. 100, 8004 (1978): Die Hemmhof-Durchmesser nach Auftragen von 25 µg Substanz betrugen hier bei Verwendung von ähnlichen Keimen 28-41 mm.

Der vorliegenden Erfindung liegt daher die Aufgabe zugrunde, eine neue Klasse von Antibiotika zur Verfügung zu stellen, die in der Tier- und Menschentherapie und bei unbelebten Systemen wichtig ist.

Diese Antibiotika sind gegenüber vielen grampositiven, gramnegativen, Penicillin-resistenten und Cephalosporin-resistenten Bakterien wirksam. Die Voraussetzung für diese hohe Wirksamkeit und Anwendbarkeit wird geschaffen durch die Trisubstitution des exocyclischen, allylischen Kohlenstoffatoms von I bzw. II mit drei via Kohlenstoffatome gebundenen Gruppen $R^3$, $R^4$ und $R^5$ Die überlegene antibakterielle Wirksamkeit der erfindungsmässigen Oxapenem-3-carbonsäuren konnte nach dem Stand der Wissenschaft nicht in diesem Masse erwartet werden. Erfindungsgemäss sollen weiterhin chemische Verfahren zur Herstellung dieser Antibiotika und ihrer nichttoxischen pharmazeutisch annehmbaren Salze; diese Antibiotika enthaltende, pharmazeutische Zubereitungen; Behandlungsverfahren, bei denen diese Antibiotika und die Zubereitungen verabreicht werden, wenn eine antibiotische Wirkung indiziert ist, geschaffen werden.

Die erfindungsgemässen Verbindungen der obigen Formeln I und II werden zweckdienlich nach dem folgenden Schema hergestellt:

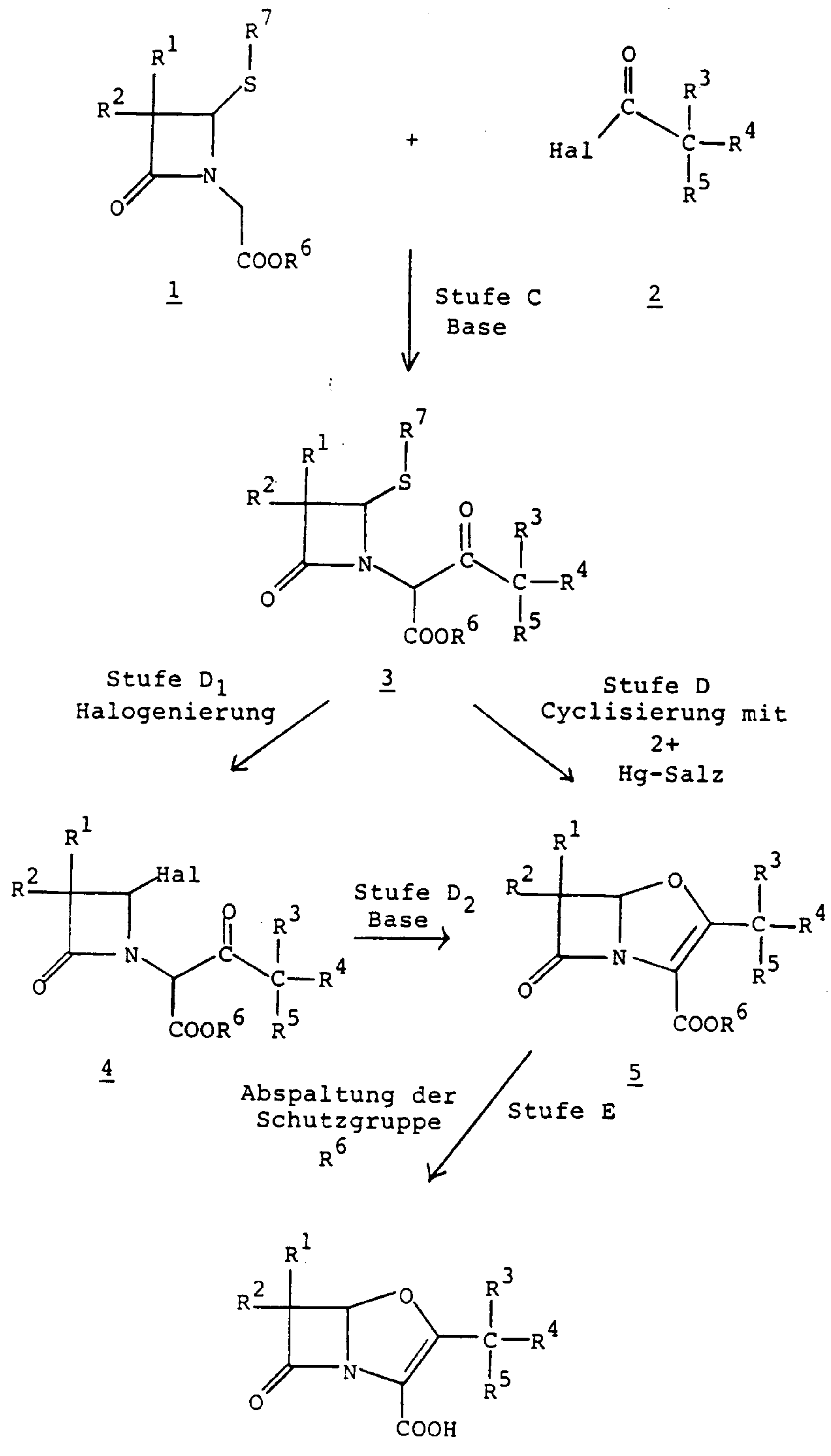

Stufe C
Base
Stufe D1
Halogenierung
Stufe D
Cyclisierung mit
2+
Hg-Salz
Stufe D2
Base
Abspaltung der
Schutzgruppe
R6
Stufe E


1 2 3 4 5 6 (I)

Verbindungen II werden zweckdienlich nach dem folgenden Reaktionsschema gewonnen:

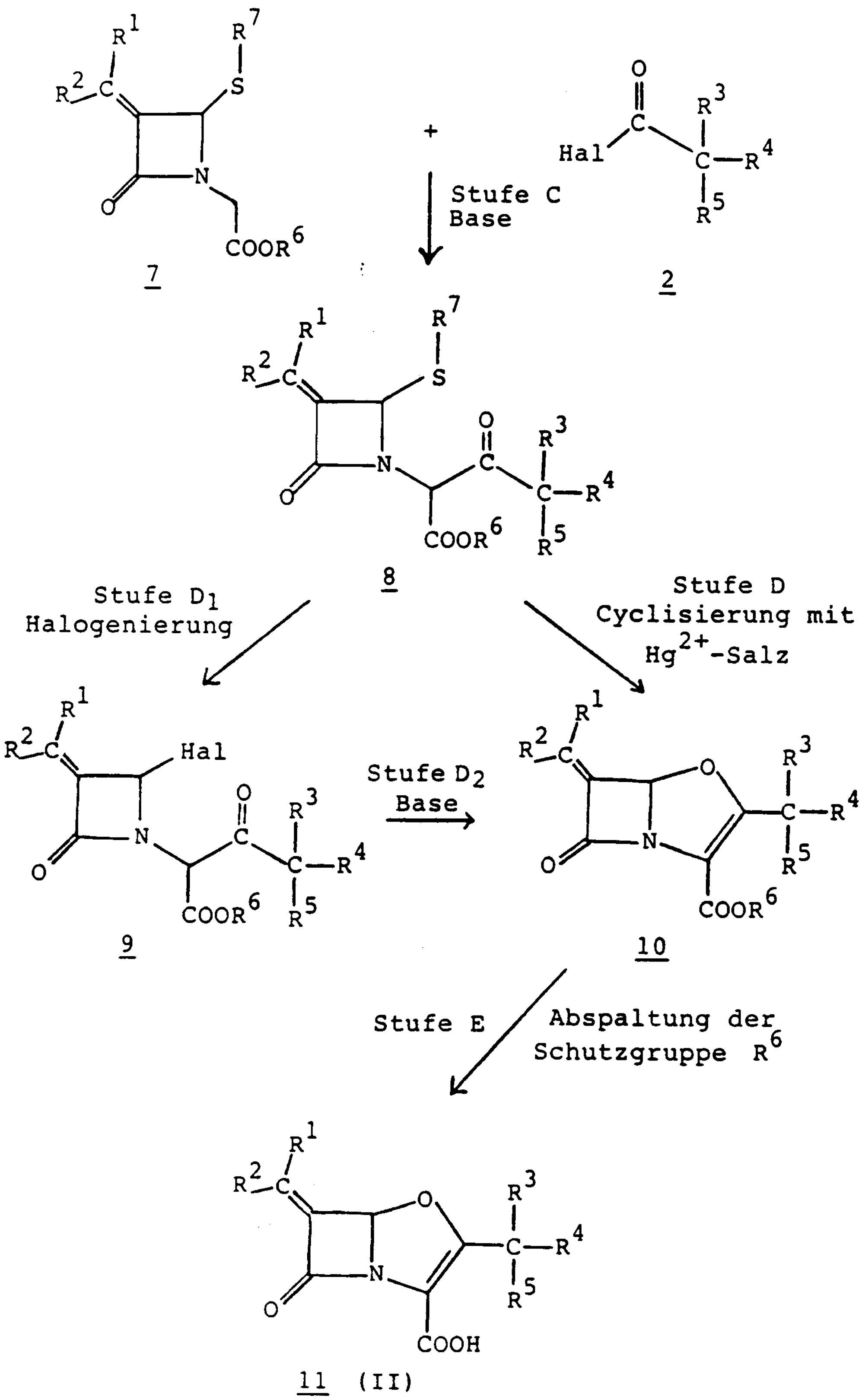


7 + 2 → 8 → 9 → 10 → 11 (II)

Worin in beiden Reaktionsschemata $R^1$, $R^2$, $R^3$, $R^4$ und $R^5$ die oben gegebenen Definitionen besitzen, $R^6$ eine leicht entfernbare Schutz- bzw. Maskierungsgruppe bedeute und wobei $R^6$ ebenfalls der Molekülteil eines pharmazeutisch annehmbaren Esters sein kann. Typischerweise ist die Schutzgruppe $R^6$ eine

Acylgruppe, wie niedrig-Alkanoyl, Aralkylcarbonyl o.ä, wie Acetyl, Brom-tert-butoxycarbonyl, Benzyloxycarbonyl, Formyl, Trifluoracetyl u.ä., oder eine Trialkylsilylgruppe wie Trimethylsilyl oder tert-Butyldimethylsilyl; und typischerweise ist die Schutzgruppe $R^6$ eine substituierte oder unsubstituierte Alkyl-, Aralkyl-, Alkenyl- oder ähnliche Gruppe, wie Benzyl, o-Nitrobenzyl, p-Nitrobenzyl, Trimethoxybenzyl, 2-Oxopropyl, 2-Oxo-2-phenylethyl, Allyl, 2-Cyanoethyl, 2-Trimethylsilyloxyethyl, 2,2,2-Trichlorethyl, Pivaloyloxymethyl, Brom-tert-butyl u.ä.

Typischerweise ist $R^7$ eine substituierte oder unsubstituierte, verzweigte oder unverzweigte Alkylgruppe, Aralkylgruppe, Arylgruppe, Heteroaryl oder Heteroaralkylgruppe, wobei die Substituenten Niederalkyl, Acyloxy, Chlor, Brom, Nitro, Niederalkyloxy, Cyano u.ä. bedeuten und die Heteroatome des Heteroaryl- oder Heteroaralkylteils ausgewählt werden aus der Gruppe Sauerstoff, Stickstoff und Schwefel. Besonders typische Reste $R^7$ sind Methyl, Ethyl, Propyl, Isopropyl, Butyl, Phenyl, Tolyl, Benzyl, Triphenylmethyl, tert-Butyl, 2-Mercaptobenzthiazolyl u.ä.

Obige Reaktionsdiagramme werden im folgenden näher in Worten erläutert. Ein geeignet substituiertes Azetidinon (1) oder (7) wird mit dem Säurehalogenid (2) mit etwa 1 bis 2 Äquivalenten einer Base wie Butyllithium, Lithiumdiisopropylamid oder Lithium-bis-(trimethylsilylamid) u.ä., bei niedriger Temperatur von etwa -70 °C bis 0 °C innerhalb etwa einer Stunde zu 3 bzw. 8 umgesetzt. Die Identität des Lösungsmittels ist nicht kritisch, vorausgesetzt nur, dass die Reaktionsteilnehmer löslich sind und es bei der Umsetzung inert oder im wesentlichen inert ist. Bei der Reaktion (1→3) bzw. (7→8) wird zweckdienlich Tetrahydrofuran, Dioxan, Glyme, Dimethylformamid oder ein Gemisch dieser Lösungsmittel mit Hexan verwendet.

Die Reaktion (3→4) bzw. (8→9) kann nach irgend einem bekannten Halogenierungsverfahren durchgeführt werden. Geeignete Halogenierungsmittel sind Chlor, Brom, Iod, Sulfurylchlorid u.ä. Bei einem bevorzugten Halogenierunsverfahren wird 3 bzw. 8 in einem inerten Lösungsmittel wie beispielsweise Tetrachlorkohlenstoff, Toluol oder Methylenchlorid mit 1 bis 2 Äquivalenten Chlor behandelt. Typischerweise wird diese Reaktion bei einer Temperatur von etwa -70 °C und 0 °C während 0.5 bis 2 Stunden durchgeführt.

Bei der Reaktion (4→5) bzw. (9→10) wird 4 bzw. 9 mit etwa 1 bis 2 Äquivalenten einer Base wie beispielsweise Natriummethoxid, Kalium-tert-butoxid, Natriumphenolat, Natriumthiophenolat, Diazabicycloundecen u.ä. in einem geeigneten inerten Lösungsmittel wie beispielsweise Toluol, Tetrahydrofuran oder Dimethylformamid zu 5 bzw. 10 umgesetzt. Die typische Reaktionszeit beträgt etwa 30 Minuten bis 2 Stunden, die typische Reaktionstemperatur etwa -70 °C bis Raumtemperatur.

Bei der direkten Cyclisierungsreaktion (3→5) bzw. (8→10) wird 3 bzw.8 mit 1-3 Äquivalenten eines Quecksilber(II)salzes wie beispielsweise Quecksilberchlorid in einem geeigneten inerten Lösungsmittel wie beispielsweise Glyme, Dioxan oder Tetrahydrofuran zu 5 bzw. 10 umgesetzt. Auch Gemische zweier oder mehrerer Quecksilber(II)salze, beispielsweise das 1 : 1-Gemisch von Quecksilber(II)oxid und Quecksilber(II)-chlorid werden typischerweise benutzt. Die typische Reaktionstemperatur beträgt 60 - 100 °C, die typische Reaktionszeit 2 bis 20 Stunden.

Die Entfernung der Schutzgruppe (5→6) bzw. (10→11) erfolgt nach an sich wohlbekanntem Verfahren, wie katalytische Hydrierung, Hydrolyse, Reduktion, nucleophile Substitution, Solvolyse u.ä. Geeignete Hydrierungskatalysatoren für die Schutzgruppenabspaltung umfassen die Platinmetalle und ihre Oxide, Raney-Nickel, Palladium auf Kohle u.ä.; geeignete Lösungsmittel für die Hydrierung sind Methanol, Ethanol, Essigsäureethylester/$H_2O$, Ethanol/$H_2O$ u.ä. in Anwesenheit von Wasserstoff bei einem Druck von 1 bis 50 at. Die Hydrierung dauert typischerweise 5 Minuten bis 2 Stunden bei einer Temperatur von 0-25 °C und wird gegebenenfalls in Gegenwart einer schwachen Base, beispielsweise Natriumhydrogencarbonat durchgeführt. Beim hydrolytischen Abbau der Schutzgruppe wird 5 bzw. 10 in einem geeigneten Lösungsmittel wie beispielsweise Tetrahydrofuran oder Tetrahydrofuran/$H_2O$ mit 1 Äquivalent Base wie z.B. verdünnter wässriger Natronlauge u.ä. versetzt. Typischerweise dauert die Reaktion 5 - 60 Minuten; die Reaktionstemperatur beträgt -30 bis 0 °C. Beim reduktiven Abbau der Schutzgruppe wird 5 bzw. 10 in einem geeigneten Lösungsmittel, beispielsweise Essigsäure/Wasser mit 1 - 3 Äquivalenten eines Reduktionsmittels, beispielsweise Zinkstaub u.ä. versetzt. Typischerweise dauert die Reaktion 30 Minuten bis 2 Stunden; die Reaktionstemperatur beträgt -30 °C bis Raumtemperatur. Beim Abbau der Schutzgruppe durch nucleophile Substitution wird 5 bzw. 10 in einem inerten Lösungsmittel, beispielsweise Tetrahydrofuran mit einem nucleophilen Agens, beispielsweise Tetrabutylammoniumfluorid u.ä. umgesetzt. Typischerweise dauert die Reaktion 30 Minuten bis 2 Stunden; die Reaktionstemperatur beträgt -30 °C bis Raumtemperatur. Beim Abbau der Schutzgruppe durch Solvolyse wird 5 bzw. 10 in einem geeigneten Lösungsmittel, beispielsweise Tetrahydrofuran mit 1 bis 2 Äquivalenten einer Lewissäure , beispielsweise Aluminiumtrichlorid versetzt und anschliessend mit einem solvolysierenden Lösungsmittel, beispielsweise Wasser versetzt. Typischerweise dauert die Reaktion 30 Min bis 2 Stunden; die Reaktionstemperatur beträgt 0 °C bis Raumtemperatur.

Die dreifach substituierten Acetylchloride (2) sind teilweise im Handel erhältlich wie z.B. Pivaloylchlorid oder 3-Chlorpivaloylchlorid oder sie sind literaturbekannt wie z.B. 2-Methyl-2-phenylpropanoylchlorid (Helv. Chim.

Acta 54, 870 (1971); J. Org. Chem. 39, 3268 (1974)) oder 3-Acetoxypivaloylchlorid (Bull. Chem. Soc. France 31, 125 (1904); J. Org. Chem. 24, 1228 (1959)) oder sie können in Analogie zu ähnlichen bekannten Stoffen hergestellt werden wie z.B. 2-Methyl-2-thienylpropanoylchlorid nach der Synthesevorschrift für das Phenylderivat.

Es wurde überraschend gefunden, dass die Verbindungen der Formeln 3 bzw. 8 und 4 bzw. 9 wegen der Trisubstitution des α-Kohlenstoffatoms durch die via Kohlenstoffatome gebundenen Gruppen $R^3$, $R^4$ und $R^5$ ausschliesslich als Ketone vorliegen, was sich durch das Fehlen von NMR-Enol-Resonanzen bei 11.6 ppm (Tetrahedron 38, (16), 2489-2504 (1982), Seite 2490) und die Anwesenheit einer Keton-Carbonylbande bei~1720 $cm^{-1}$ und einer Bande eines gesättigten Carbonsäureesters bei~1755 $cm^{-1}$ im IR-Spektrum aufgenommen in Methylenchlorid äussert. Die Ketonstruktur ist auch durch mangelnde Reaktivität bewiesen: So ergeben diese Verbindungen, in Methylenchlorid auf ein Filterpapier aufgetragen und mit wässriger Eisen(III)chlorid-Lösung besprüht, keine violette Färbung. Die Ketone der Formeln 3 bzw. 8 und 4 bzw. 9 werden auch nicht, mit Diazomethanlösung in Ether versetzt, in die Enolether umgewandelt. Alle diese Befunde stehen im Gegensatz zu früher bekanntgewordenen Zwischenprodukten ohne die erfindungsmässige Trisubstitution; diese lagen ausschliesslich oder hauptsächlich als Enole vor (z.B. in EP 0018305 Al, Seite 3; Tetrahedron 38, (16), 2490 (1982); J.C.S. Chem. Comm. 1977, 720, J.C.S. Chem. Comm. 1977, 905).

Dies zeigt, dass Verbindungen der Formeln 3 bzw. 8 und 4 bzw. 9 niemals zuvor hergestellt und niemals weiter umgesetzt worden sind. Weil die Endprodukte I bzw. II nur via ketonische Zwischenprodukte hergestellt werden können, zeigt es ebenfalls die Neuheit von I und II. Zwar wurden in früheren Patentanmeldungen (z.B. in EP 0018305 A1) Oxapenem-3-carbonsäuren mit verzweigten aliphatischen Resten in 2-Stellung erwähnt. Weil diese aber aus Enolen hergestellt wurden, kann es sich nicht um die erfindungsmässigen Verbindungen I oder II handeln.

Bei Verwendung von chiralen Azetidin-2-onen der Formeln 1 bzw. 7 mit der 4R-Konfiguration werden gegebenenfalls nach den beschriebenen Reaktionsschemata (1→6) bzw. (7→11) chirale 1-Oxapen-2-em-3-carbonsäuren (I) bzw. (II) erhalten, die ebenfalls die 4R-Konfiguration besitzen.

Eine Variante der Synthese von Verbindungen (I) und (II) ergibt sich gegebenenfalls durch eine Umwandlung der Gruppen $R^3$, $R^4$, $R^5$ auf der Stufe des Ketons 3 bzw. 8. So kann beispielsweise eine Gruppe $R^3$ = Alkyl-Cl mit Nucleophilen wie z.B. Tetraalkylammoniumazid in eine Gruppe $R^3$ = Alkyl-$N_3$ übergeführt werden. Typisches Lösungsmittel für die beispielhafte Reaktion ist DMF. Typische Reaktionstemperatur ist 0 °C bis 80 °C und die Reaktion dauert typischerweise 2-48 Stunden.

Eine vorteilhafte Variante der Synthese von Verbindungen (I) und (II) ergibt sich gegebenenfalls durch eine mehrfache Abspaltung von ausgewählten Schutzgruppen in der Stufe (5→6) bzw. (10→11). So können beispielsweise geschützte Hydroxyalkylgruppen $R^1$, $R^2$, aber auch geschützte Hydroxyalkyl- oder geschützte Aminoalkylgruppen $R^3$, $R^4$, $R^5$ bei der Entfernung der Schutzgruppe $R^6$ gleich mit freigesetzt werden.

Im folgenden wird die Synthese des Ausgangsmaterials 1 beschrieben. 1 wird nach an sich bekannten Verfahren aus 4-Acyloxyazetidin-2-onen der Formel 12 oder aus Sulfonylazetidin-2-onen der Formel 13 auf folgendem Weg hergestellt:

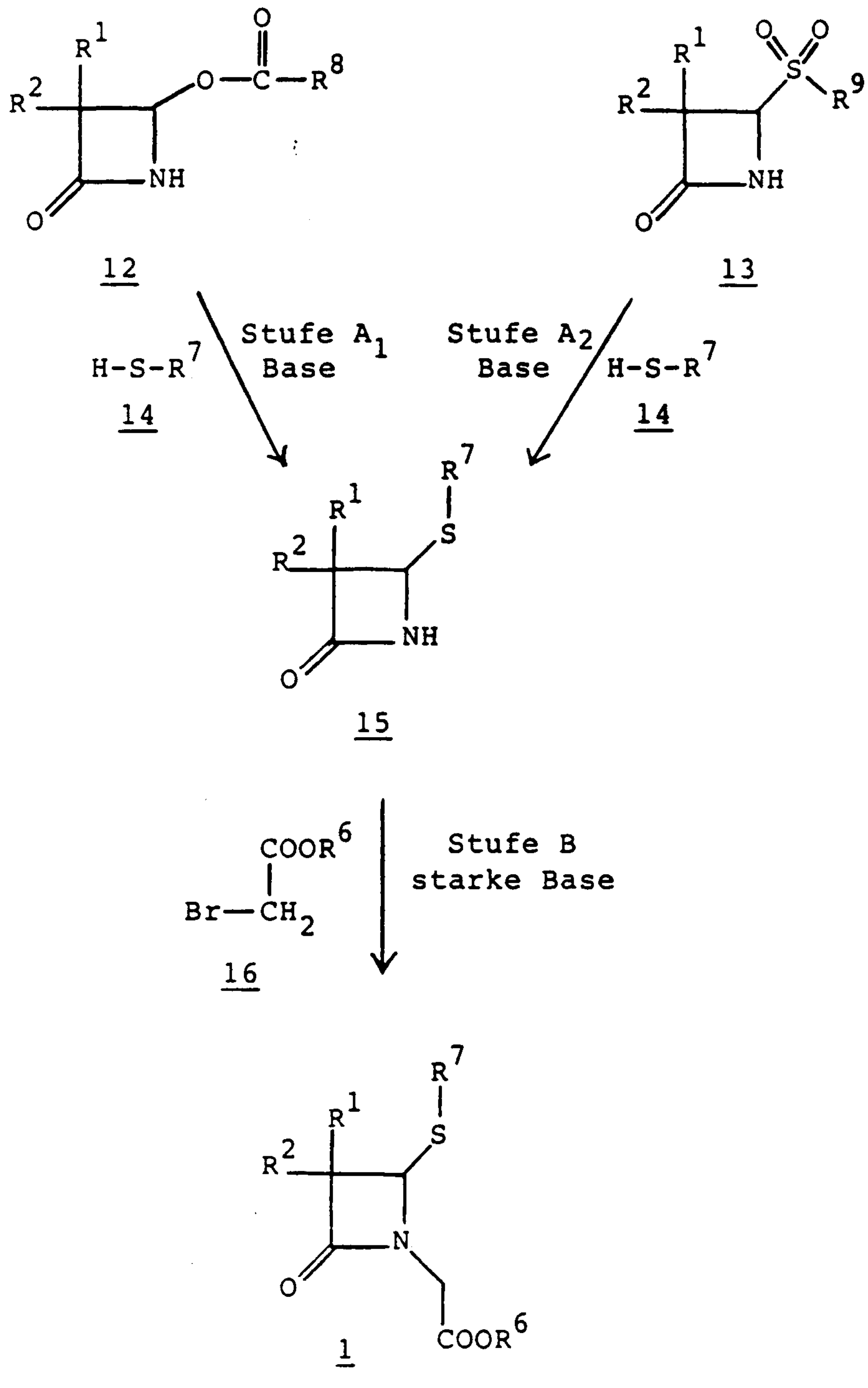


12 13 14 14 15 16 1

worin $R^1$, $R^2$, $R^7$ und $R^6$ die obengenannten Bedeutungen haben und $R^8$ eine Alkyl- oder Arylgruppe wie beispielsweise Methyl oder Phenyl bedeutet $R^9$ ist typischerweise eine Alkyl- oder Arylgruppe, wie beispielsweise Methyl oder Phenyl oder eine Hydroxyalkyl- oder Hydroxyaralkylgruppe wie beispielsweise 2-Hydroxyethyl, 2-Hydroxyisopropyl, 2-Hydroxy-1-phenylethyl oder 2-Hydroxy-tert-butyl u.ä.

Bei der Reaktion (12→15) bzw. (13→15) wird 12 bzw. 13 mit 1 - 1.5 Äquivalenten eines Mercaptans (14) in einem geeigneten Lösungsmittel wie Tetrahydrofuran, Tetrahydrofuran/$H_2O$ oder Isopropanol/$H_2O$ mit einer Base wie Diazabicycloundecen oder Natronlauge u.ä. zu 15 umgesetzt. Typischerweise beträgt die Reaktionstemperatur -30 °C bis Raumtemperatur und die Reaktionsdauer etwa 30 Minuten bis 4 Stunden.

Bei der Reaktion (15→1) wird 15 mit einem geeigneten Bromessigsäureester(16)in einem inerten Lösungsmittel wie Tetrahydrofuran, Dimethylformamid oder einem Gemisch dieser Lösungsmittel mit Hexan mit einer starken Base wie Butyllithium, Kalium-tert-butoxid, Lithiumdiisopropylamid oder Lithium-bis-

(trimethylsilylamid) u.ä. zu 1 umgesetzt. Typische Reaktionstemperaturen sind etwa -70-0 °C, typische Reaktionszeiten 30 Minuten bis 2 Stunden.

Die Verbindungen 12 sind aus Chlorsulfonylisocyanat und Vinylestern nach Ann. Chem. 1974, 539 herstellbar, aber auch Synthesen, die von Penicillin ausgehen, sind bekannt (z.B. in Recent Advances in the Chemistry of β-Lactam Antibiotics ed. by G.I. Gregory,The Royal Society of Chemistry, London, Seite 330-348 (1981)). Verbindungen 13 sind entweder aus 12 herstellbar nach Ann. Chem. 1974, 539 oder nach Journ. Amer. Chem. Soc., 102, 2039 (1980) bzw. Recent Adv. in the Chem. of β-Lactam Antibiotics, ed. by G.I. Gregory, the Royal Society of Chemistry, London, Seite 368-378 (1981)), aber auch Verfahren der herstellung von 13 aus Penicillin sind bekannt (z.B. Tetrahedron Lett. 22, 4141-4144 (1981)).

Bei Verwendung von chiralen Azetidinonen 12 bzw. 13 mit der 4R-Konfiguration entstehen Verbindungen 1 mit der gleichen 4R-Konfiguration.

Im folgenden wird die Synthese des ungesättigten Ausgangsmaterials 7 beschrieben. 7 wird zweckmässigerweise nach dem folgenden Reaktionsschema hergestellt.

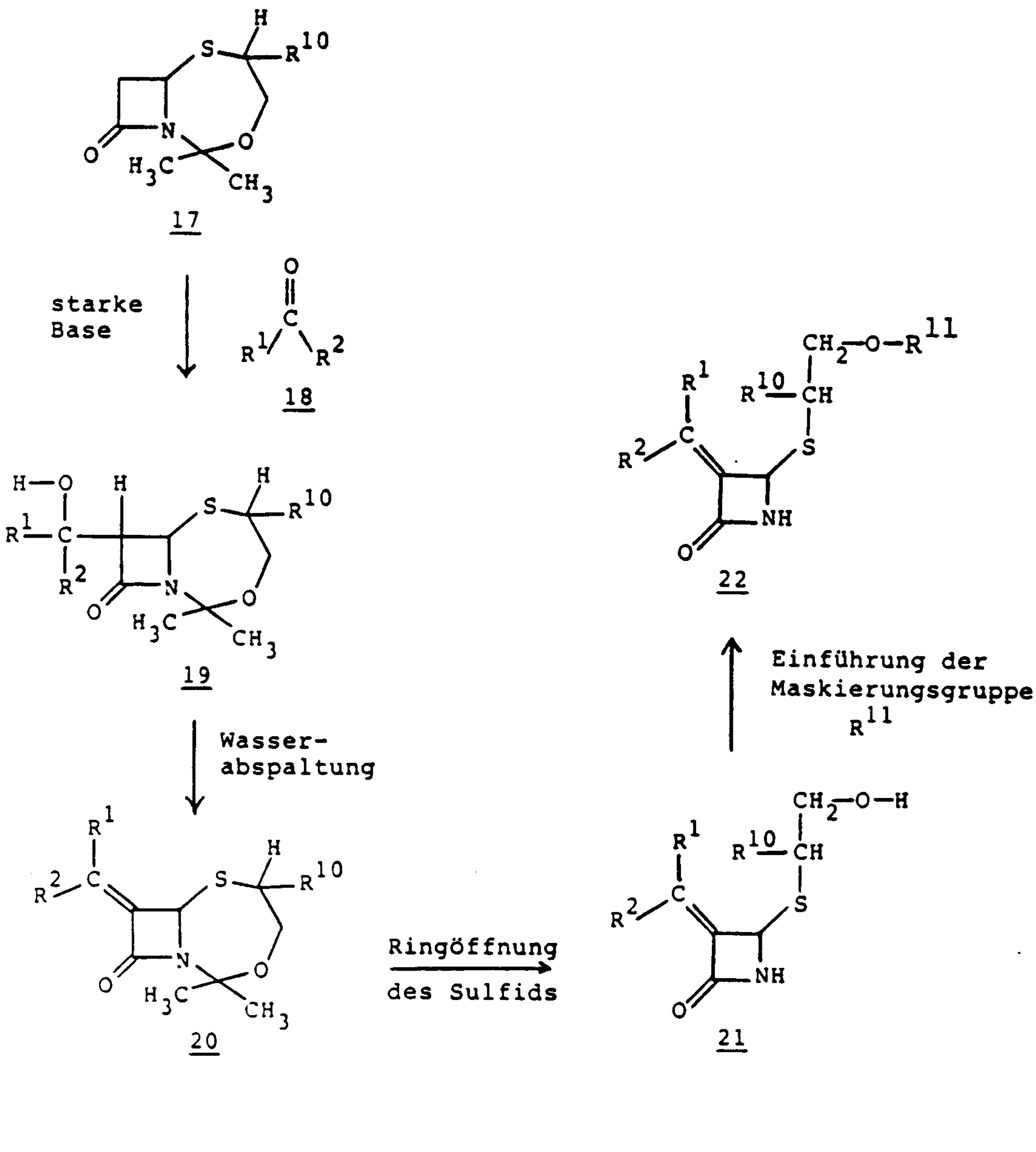


17

18

19

20

21

22

20

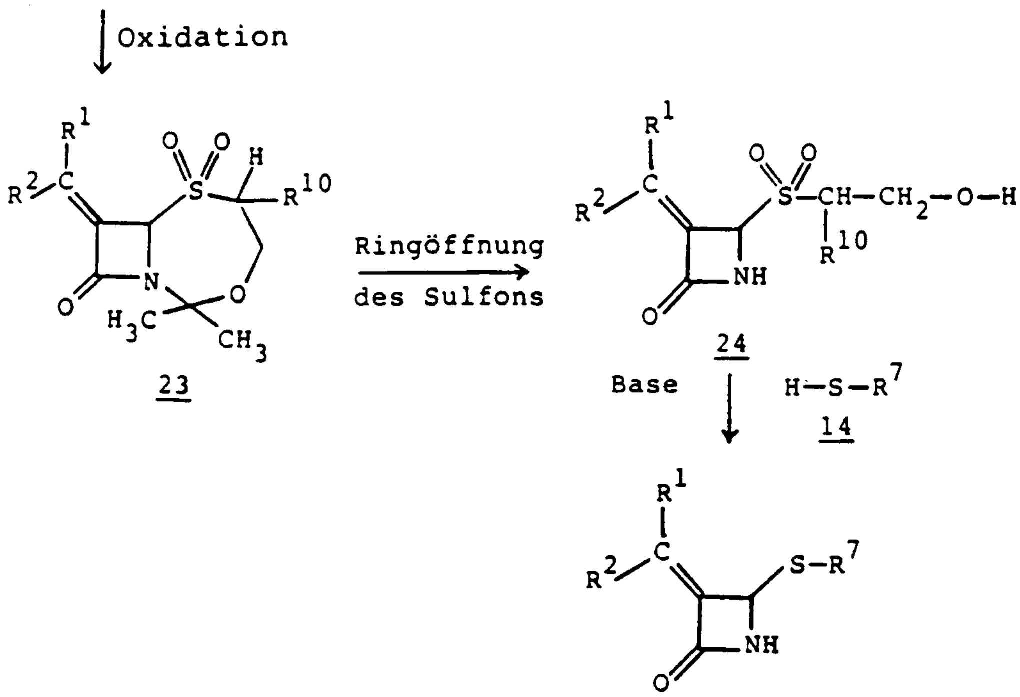


23 24 14

22

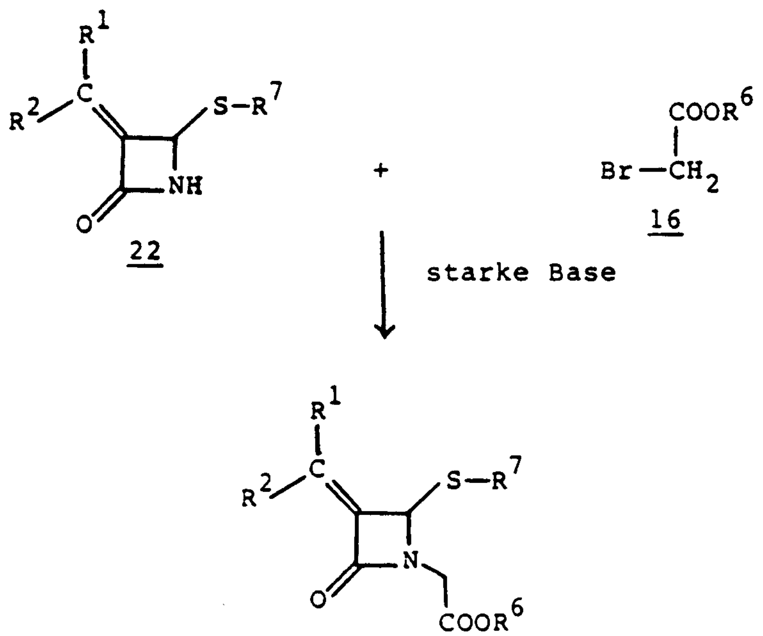


22 16

7

worin $R^1$, $R^2$, $R^6$ und $R^7$ die oben gegebenen Definitionen besitzen, $R^{10}$ Wasserstoff, eine Alkyl- oder Arylgruppe bedeutet wie beispielsweise Methyl oder Phenyl und $R^{11}$ eine leicht einführbare Maskierungsgruppe wie Alkyl, Aryl, Aralkyl, Acyl, Trialkylsilyl. Typischerweise ist $R^{11}$ Benzyl, Acetyl, Benzoyl, Trimethylsilyl- oder tert-Butyldimethylsilyl u.ä. Die Identität der Maskierungsgruppe ist jedoch nicht besonders kritisch, da sie nicht als solche abgespalten werden muss aber trotzdem auf einer späteren Reaktionsstufe, bei der Halogenierung (8→9) oder der Cyclisierung mit Quecksilber(II)salz (8→10), als Teil

eines abgespaltenen Moleküls wieder verschwindet.

Bei der Reaktion (17→19) wird 17 in einem inerten Lösungsmittel, wie beispielsweise Tetrahydrofuran oder Tetrahydrofuran/Hexan u.ä. mit 1 bis 1.2 Äquivalenten einer starken Base, wie beispielsweise Lithiumdiisopropylamid u.ä. versetzt und anschliessend mit dem Keton 18 zu 19 umgesetzt. Typischerweise beträgt die Reaktionstemperatur -70 °C bis 0 °C; die Reaktionszeit 30 Minuten bis 2 Stunden.

Bei der Wasserabspaltungs-Stufe (19→20) wird 19 in einem inerten Lösungsmittel wie beispielsweise Tetrahydrofuran mit 1 bis 1.5 Äquivalenten eines Säurechlorids wie beispielsweise Thionylchlorid, p-Toluolsulfonsäurechlorid, Methansulfonsäurechlorid oder Acetylchlorid o.ä. und 1 bis 5 Äquivalenten einer Base wie Pyridin, Triethylamin, N,N-Dimethylaminopyridin o.ä. versetzt und der intermediäre Ester anschliessend in einem inerten Lösungsmittel, wie beispielsweise Tetrahydrofuran mit einer starken Base wie Kalium-tert-butoxid oder Diazabicycloundecen o.ä. zu 20 umgesetzt. Typischerweise betragen die Reaktionstemperaturen bei beiden Reaktionsschritten, d.h. bei der Veresterung und bei der Elimination -30 °C bis +50 °C. Die Reaktionsdauer der Veresterung beträgt je nach Basenstärke der verwendeten Base 2 Stunden bis 48 Stunden. Die Reaktionsdauer der Elimination beträgt etwa 30 Minuten bis 2 Stunden. Einfacher gelingt die Wasserabspaltungs-Stufe durch Erhitzen von 19 in einem inerten Lösungsmittel wie Toluol u.ä. im Wasserabscheider mithilfe eines Katalysators, wie beispielsweise p-Toluolsulfonsäure oder p-Toluolsulfochlorid. Bei der Rückflusstemperatur des Toluols beträgt die Reaktionszeit typischerweise 2 - 10 Stunden.

Bei der Ringöffnungs-Stufe des Sulfids (20→21) wird 20 in einem sauren Lösungsmittel, wie beispielsweise Essigsäure/$H_2O$ o.ä. erhitzt. Bei der Rückflusstemperatur von ca. 110 °C dauert die Reaktion typischerweise 30 Minuten bis 2 Stunden.

Die Einführung der Maskierungsgruppe $R^{11}$ (21→22) erfolgt durch Umsatz von 21 mit 1 bis 1.3 Äquivalenten eines geeigneten leicht einführbaren Alkylierung- oder Acylierungsmittels wie beispielsweise Benzylchlorid, Benzylbromid, Acetylchlorid, Benzoylchlorid, Trimethylchlorsilan oder tert-Butyldimethylchlorsilan in Gegenwart einer Base, wie Kalium-tert-butoxid, Triethylamin, N,N-Dimethylaminopyridin, Pyridin, Imidazol o.ä. in einem inerten Lösungsmittel wie Tetrahydrofuran oder Dimethylformamid u.ä. Typischerweise beträgt die Reaktionstemperatur etwa -30 °C bis Raumtemperatur.

Die Oxidations-Stufe (20→23) erfolgt durch Umsatz von 20 mit einem an sich bekannten Oxidationsmittel, das zur Sulfoxidierung verwendet werden kann wie beispielsweise Kaliumpermanganat, Wasserstoffperoxid, m-Chlorperbenzoesäure o.ä. Typischerweise wird eine Lösung von 20 in einem inerten Lösungsmittel, wie beispielsweise Methylenchlorid, Chloroform oder Aceton mit 2 bis 2.5 Äquivalenten eines Oxidationsmittels wie m-Chlorperbenzoesäure zu 23 umgesetzt. Typischerweise beträgt die Reaktionstemperatur -30 °C bis Raumtemperatur und die Reaktionszeit 30 Minuten bis 2 Stunden.

Bei der Ringöffnungs-Stufe des Sulfons (23→24) wird 23 in einem sauren Lösungsmittel, wie beispielsweise Essigsäure/$H_2O$ o.ä. erhitzt. Bei der Reaktionstemperatur von ca. 110 °C dauert die Reaktion typischerweise 30 Minuten bis 2 Stunden.

Verbindungen der Formel 24 werden dann mit einem Mercaptan 14 in Gegenwart einer Base, wie beispielsweise Natronlauge und Diazabicycloundecen o.ä. zu 22 umgesetzt. Die Reaktionsbedingungen entsprechen denjenigen der Reaktionsstufe (12→15).

Verbindungen der Formel 22 werden mit einem Bromessigsäureester der Formel 16 mithilfe einer starken Base, wie beispielsweise Butyllithium, Lithiumdiisopropylamid oder Lithium-bis-trimethylsilyl-amid zu Verbindung 7 umgesetzt. Die Reaktionsbedingungen entsprechen denjenigen der Reaktionsstufe (15→1).

Die Verbindungen 17 sind nach Recent Adv. in the Chem. of β-Lactam Antibiotics, ed. by G.I. Gregory, the Royal Society of Chemistry, London, Seite 368-378 (1981) oder nach Tet. Lett. 22, 4141-4144 (1981) zugänglich. Bei Verwendung von chiralem Ausgangsmaterial 17 mit der 7R-Konfiguration entsteht bei der Umwandlung (17→7) chirales 7 mit der gleichen 4R-Konfiguration.

In der allgemeinen Beschreibung der vorliegenden Erfindung werden die Gruppen $R^1$ und $R^2$ bevorzugt ausgewählt aus: Wasserstoff, Alkyl, geschütztem oder ungeschütztem Hydroxyalkyl oder geschütztem oder ungeschütztem Dihydroxyalkyl mit bis zu 6 Kohlenstoffatomen. $R^3$, $R^4$ und $R^5$ werden bevorzugt ausgewählt aus substituiertem oder unsubstituiertem Alkyl, Alkenyl, Alkinyl, Cycloalkyl, Alkylcycloalkyl, Cycloalkylalkyl, Alkenylcycloalkyl, Cycloalkenylalkyl, Aryl, Aralkyl, Aralkenyl, Aralkinyl, worin die vorhergehenden Alkyl-, Alkenyl-, oder Alkinylteile 1 bis 6 Kohlenstoffatome, die Cycloalkyl- oder Cycloalkenyl-Molekülteile 3 bis 6 und die Aryl-Molekülteile 6 bis 10 Kohlenstoffatome enthalten, Heteroaryl, Heteroaralkyl, Heteroaralkenyl, Heteroaralkinyl, Alkylheteroaryl, Heterocyclyl, Heterocyclylalkyl, Heterocyclylalkenyl, Heterocyclylalkinyl, Alkylheterocyclyl, worin die vorhergehenden Alkyl-, Alkenyl-, oder Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können:

Geschütztes oder ungeschütztes Hydroxy, Hydroxyalkyloxy, Aminoalkyloxy, Amidinoalkyloxy, Alkyloxy, Acyloxy, Aryloxy, Heteroaryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, geschütztes oder ungeschütztes Amino oder Monoalkylamino, Dialkylamino, Oxo, geschütztes oder ungeschütztes Oximino oder Alkylimino, Tetraalkylammonium, Cycloalkylamino, Arylamino, Heteroarylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder geschütztes oder ungeschütztes Sulfo, Sulfoxy oder Carboxy, wobei die Substituenten ein-oder mehrfach auftreten und ihr Alkyl-Molekülteil 1 bis 6 Kohlenstoffatome, ihr Aryl-Molekülteil 6 bis 10 Kohlenstoffatome enthält, und wobei der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff.

Eine besonders bevorzugte Verbindungsklasse ist die, worin $R^1$ und $R^2$ unabhängig voneinander Wasserstoff, Alkyl, geschütztes oder ungeschütztes Hydroxyalkyl oder geschütztes oder ungeschütztes Dihydroxyalkyl bis zu 6 Kohlenstoffatomen bedeuten, $R^3$ und $R^4$ Methyl bedeuten und $R^5$ ausgewählt wird unter den Gruppen:

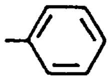
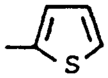
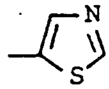

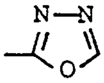
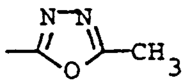
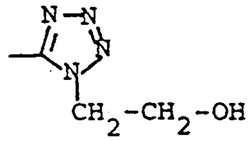

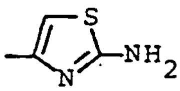
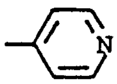
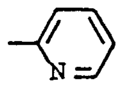

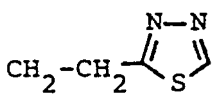
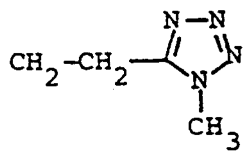

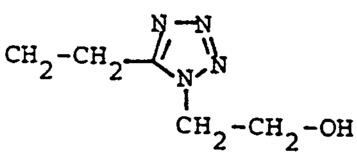
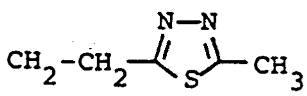

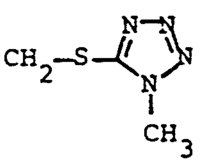
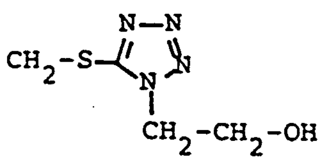
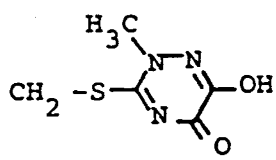

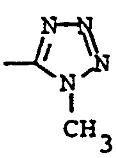
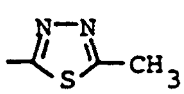
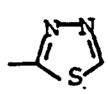

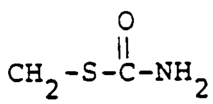 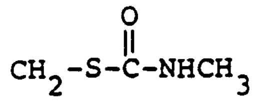 $CH_2-S-CH_3$

$CH_2-S-CH_2-CH_2-NH_2$ 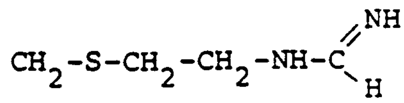

$CH_2-NH_2$ 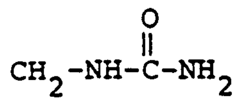 $CH_2-NH-CHO$

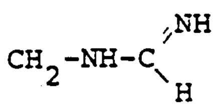 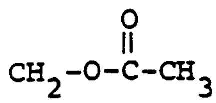 $CH_2-O-CHO$

$CH_2-Cl$ 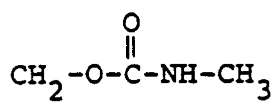 $CH_2-N_3$

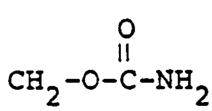 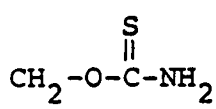 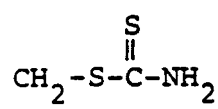

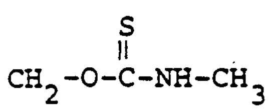 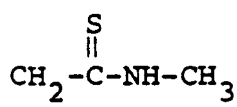 $CH_2-CH_2-NH_2$

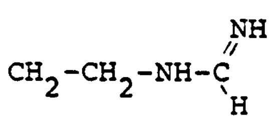 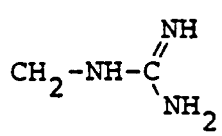 $CH_2-CH_2-CH_2-NH_2$

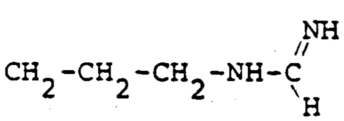 $CH_2-CH_2-COOH$ $CH_2-COOH$

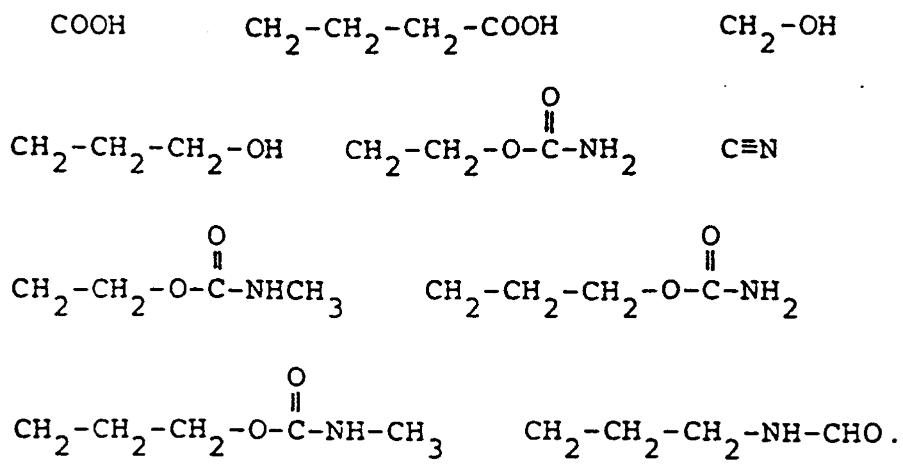

Ester, die als Schutzgruppen verwendet werden, sind solche, worin $R^6$ Benzyl, p-Nitrobenzyl, Methyl, tert-Butyl, Diphenylmethyl, Trimethylsilyl, tert-Butyldimethylsilyl, Trichlorethyl bedeutet; oder $R^6$ die pharmazeutisch annehmbaren Estermolekülteile, wie Pivaloyloxymethyl, Allyl, Methallyl, 2-Oxoethyl, 2-Oxopropyl, (2-Methylthio)-äthyl oder 3-Buten-1-yl bedeutet.

Schutzgruppen der geschützten Hydroxyalkyl und Dihydroxyalkylgruppen $R^1$ und $R^2$ sind Benzyl, p-Nitrobenzyl, Benzyloxycarbonyl, p-Nitrobenzyloxycarbonyl, Trimethylsilyl, tert-Butyldimethylsilyl, Benzyliden und Oxomethylen.

Die Schutzgruppen der geschützten Substituenten von $R^3$, $R^4$ und $R^5$ sind identisch mit den vorstehend genannten.

Die erfindungsgemässen Produkte (I) und (II) bilden eine grosse Vielzahl pharmakologisch annehmbarer Salze mit anorganischen und organischen Basen. Diese umfassen beispielsweise Metallsalze, die sich von Alkalimetall- oder Erdalkalimetallhydroxiden, -carbonaten oder -bicarbonaten ableiten, und Salze, die sich von primären, sekundären oder tertiären Aminen ableiten, wie Monoalkylamine, Dialkylamine, Trialkylamine, niedrig-Alkanolamine, Di-niedrig-alkanolamine, niedrig-Alkylendiamine, N,N-Diaralkyl-niedrig-alkylendiamine, Aralkylamine, Amino-subst.-niedrig-alkanole, N,N-Di-niedrig-alkylamino-subst.-niedrig-alkanole, Amino-, Polyamino- und Guanidino-subst.-niedrig- Alkansäuren und Stickstoff enthaltende heterocyclische Amine. Beispiele für Salze sind solche, die sich von Natriumhydroxid, Natriumcarbonat, Natriumbicarbonat, Kaliumcarbonat, Kaliumhydroxid, Calciumcarbonat, Trimethylamin, Triäthylamin, Piperidin, Morpholin, Chinin, Lysin, Protamin, Arginin, Procain, Äthanolamin, Morphin, Benzylamin, Äthylendiamin, N,N'-Dibenzyläthylendiamin, Diäthanolamin, Piperazin, Dimethyl-aminoäthanol, 2-Amino-2-methyl-1-propanol, Theophyllin, N-Methylglucamin u.ä. ableiten.

Gegenstand der Erfindung sind weiterhin Salze von Aminogruppen, die in bestimmten Species I und II an den Seitenketten von $R^3$, $R^4$ und $R^5$ enthalten sind. Solche pharmazeutisch annehmbaren Säureadditionssalze leiten sich von organischen und anorganischen Säuren, wie HCl, HBr, Citronensäure, Weinsäure u.ä., ab.

Die Salze können Monosalze, wie das Mononatriumsalz, das durch Behandlung von 1 Äquiv. Natriumhydroxid mit 1 Äquiv. der Produkte (I) und (II) erhalten wird, wie auch gemischte Disalze sein. Solche Salze können durch Behandlung von 1 Äquiv. einer Base mit einem zweiwertigen Kation, wie Calciumhydroxid, mit 1 Äquiv. der Produkte (I) und (II) erhalten werden. Die erfindungsgemässen Salze sind pharmakologisch annehmbare, nichttoxische Derivate, die als aktiver Bestandteil in geeigneten pharmazeutischen Dosiseinheitsformen verwendet werden können. Sie können ebenfalls mit anderen Arzneimitteln unter Bildung von Zubereitungen mit einem breiten Aktivitätsspektrum kombiniert werden.

Die neuen erfindungsgemässen stabilen Oxapen-2-em-carbonsäuren sind wertvolle antimikrobielle Substanzen, die gegenüber verschiedenen grampositiven und gramnegativen Pathogenen wirksam sind. Die freie Säure und insbesondere ihre Salze, wie die Amin- und Metallsalze, insbesondere die Alkalimetall- und Erdalkalimetallsalze, sind nützliche Bakterizide und können zur Entfernung empfindlicher Pathogener von zahnmedizinischen und medizinischen Vorrichtungen, für die Abtrennung von Mikroorganismen unf für die therapeutische Verwendung bei Menschen und Tieren eingesetzt werden. Zu diesem letzteren Zweck werden pharmakologisch annehmbare Salze mit anorganischen und organischen Basen, wie sie an sich bekannt sind und bei der Verabreichung von Penicillinen und Cephalosporinen verwendet werden, verwendet. Beispielsweise können Salze, wie Alkalimetall- und Erdalkalimetallsalze, und primäre, sekundäre und tertiäre Aminsalze für diesen Zweck verwendet werden. Diese Salze können mit pharmazeutisch annehmbaren flüssigen und festen Trägern unter Bildung geeigneter Dosiseinheitsformen, wie Pillen, Tabletten, Kapseln, Suppositorien, Sirupen, Elixieren u.ä., verwendet werden, die nach an sich bekannten Verfahren hergestellt werden können.

Die neuen Verbindungen sind wertvolle Antibiotika gegenüber verschiedenen grampositiven und gramnegativen Bakterien und finden dementsprechend in der Human- und Veterinärmedizin Verwendung. Die erfindungsgemässen Verbindungen können als antibakterielle Arzneimittel zur Behandlung von Infektionen verwendet werden, die durch grampositive oder gramnegative Bakterien erzeugt werden, z.B. gegen Staphylococcus aureus, Escherichia coli, Klebsiella pneumoniae, Bacillus subtilis, Salmonella typhosa, Pseudomonas und Bacterium proteus.

Die erfindungsgemässen antibakteriellen Mittel können weiterhin als Zusatzstoffe für Tierfutter, für die Konservierung von Nahrungsmitteln bzw. Futter und als Desinfektionsmittel verwendet werden. Beispielsweise können sie in wässrigen Zubereitungen in Konzentrationen im Bereich von 0.1 bis 100 Teilen Antibiotikum/Million Teile Lösung zur Zerstörung und Inhibierung des Wachstums schädlicher Bakterien auf medizinischen und zahnmedizinischen Geräten und als Bakterizide bei industriellen Anwendungen, z.B. bei Anstrichmitteln auf Wassergrundlage und in weichem Wasser der Papiermühlen, zur Inhibierung des Wachstums schädlicher Bakterien verwendet werden.

Die erfindungsgemässen Produkte können allein oder zusammen als aktiver Bestandteil in irgendeiner Vielzahl von pharmazeutischen Zubereitungen verwendet werden. Diese Antibiotika und ihre entsprechenden Salze können in Kapselform oder als Tabletten, Pulver oder flüssige Lösungen oder als Suspensionen oder Elixiere verwendet werden. Sie können oral, intravenös oder intramuskulär verabreicht werden.

Die Zubereitungen werden bevorzugt in einer für die Absorption durch den gastro-intestinalen Trakt geeigneten Form verabreicht. Tabletten und Kapseln für die orale Verabreichung können in Dosiseinheitsform vorliegen und sie können übliche Arzneimittelträgerstoffe, wie Bindemittel, z.B. Sirup, Akaziengummi, Gelatine, Sorbit, Tragant oder Polyvinylpyrrolidon; Füllstoffe, z.B. Lactose, Zucker, Maisstärke, Calciumphosphat, Sorbit oder Glycin; Schmiermittel, z.B. Magnesiumstearat, Talk, Polyäthylenglykol, Siliciumdioxid; Desintegrationsmittel, z.B. Kartoffelstärke, oder annehmbare Benetzungsmittel, wie Natriumlaurylsulfat, enthalten. Die Tabletten können nach an sich gut bekannten Verfahren beschichtet werden. Orale, flüssige Zubereitungen können in Form von wässrigen oder öligen Suspensionen, Lösungen, Emulsionen, Sirupen, Elixieren usw. vorliegen, oder sie können als Trockenprodukt, z.B. für die Rekonstitution mit Wasser oder anderen geeigneten Trägern vor der Verwendung, vorliegen. Solche flüssigen Präparationen können an sich bekannte Zusatzstoffe, wie Suspensionsmittel, z.B. Sorbitsirup, Methylcellulose, Glucose/Zuckersirup, Gelatine, Hydroxyäthylcellulose, Carboxymethylcellulose, Aluminiumstearatgel, oder hydrierte geniessbare Öle, z.B. Mandelöl, fraktioniertes Cocosnussöl, ölige Ester, Propylenglykol oder Äthylalkohol; Konservierungsmittel, z.B. Methyl- oder Propyl-p-hydroxybenzoat oder Sorbinsäure, enthalten. Suppositorien werden an sich bekannte Suppositoriengrundstoffe, z.B. Kakaobutter oder andere Glyceride, enthalten.

Die Zubereitungen für die Injektion können in Dosiseinheitsform in Ampullen oder in Behältern mit mehreren Dosen mit einem zugegebenen Konservierungsstoff vorliegen. Die Zubereitungen können in Form von Suspensionen, Lösungen oder Emulsionen in öligen oder wässrigen Trägern vorliegen, und sie können Formulationsmittel, wie Suspensions-, Stabilisations- und/oder Dispersionsmittel enthalten. Alternativ kann der aktive Bestandteil in Pulverform für die Rekonstitution mit einem geeigneten Träger, z.B. sterilem, pyrogenfreiem Wasser, vor der Verwendung vorliegen.

Die Zubereitungen können ebenfalls in einer für die Absorption durch die Schleimhautmembranen der Nase und des Halses oder der Bronchialgewebe geeigneten Form vorliegen, und sie können zweckdienlich in Form von Pulvern oder flüssigen Sprays oder Inhalationsmitteln, Lutschbonbons, als Auspinselungsmittel für den Hals, etc. vorliegen. Für die Medikation der Augen und Ohren können die Zubereitungen in Form einzelner Kapseln in flüssiger oder semi-fester Form vorliegen, oder sie können als Tropfen usw. verwendet werden. Topische Anwendungen können in hydrophoben oder hydrophilen Grundstoffen als Salben, Cremes, Lotionen, Auspinselungsmittel, Pulver usw. vorliegen bzw. formuliert werden.

Die erfindungsgemässen Zubereitungen können zusätzlich zu dem Träger einen anderen Bestandteil, wie Stabilisatoren, Bindemittel, Antioxydantien, Konservierungsmittel, Schmiermittel, Suspensionsmittel, Viskositätsmittel oder Geschmacksmittel u.ä., enthalten. Zusätzlich können in den Zubereitungen andere aktive Bestandteile enthalten sein, so daß man ein breiteres antibiotisches Aktivitätsspektrum erhält.

Für die Veterinärmedizin können die Zubereitungen z.B. als intramammare Zubereitung in entweder langwirkenden oder schnellfreisetzenden Grundstoffen formuliert werden.

Die zu verabreichende Dosis hängt in grossem Ausmass von dem Zustand des zu behandelnden Subjekts und dem Gewicht des Wirts, dem Weg und der Frequenz der Verabreichung ab. Der parenterale Weg ist für generalisierte Infektionen und der orale Weg für intestinale Infektionen bevorzugt. Im allgemeinen enthält eine tägliche orale Dosis etwa 15 bis etwa 600 mg aktiven Bestandteil/kg Körpergewicht des

Subjekts bei einer oder mehreren Anwendungen pro Tag. Eine bevorzugte tägliche Dosis für erwachsene Menschen liegt im Bereich von etwa 40 bis 120 mg aktiven Bestandteil/kg Körpergewicht.

Die erfindungsgemässen Zubereitungen können in verschiedenen Einheitsdosisformen, z.B. in festen oder flüssigen, oral aufnehmbaren Dosisformen, verabreicht werden. Die Zubereitungen pro Einheitsdosis können entweder in fester oder in flüssiger Form 0.1 bis 99 % aktives Material enthalten. Der bevorzugte Bereich beträgt etwa 10 bis 60 %. Die Zubereitungen werden im allgemeinen 15 bis etwa 1500 mg aktiven Bestandteil enthalten, im allgemeinen ist es jedoch bevorzugt, eine Dosismenge im Bereich von etwa 250 bis 1000 mg zu verwenden. Bei der parenteralen Verabreichung wird die Einheitsdosis normalerweise die reine Verbindung in einer sterilen Wasserlösung sein oder in Form eines löslichen Pulvers, das aufgelöst werden kann, vorliegen.

Die folgenden Beispiele erläutern die erfindungsgemässen Produkte, Verfahren, Zubereitungen und Behandlungsverfahren.

Beispiel 1

Herstellung von 2-tert-Butyl-1-oxapen-2-em-3-carbonsäure, dem p-Nitrobenzylester und ihrem Natriumsalz

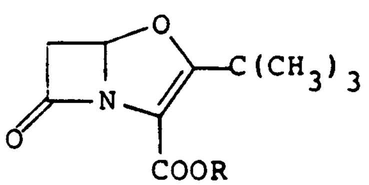

R = H, $CH_2C_6H_4NO_2$, Na

Stufe $A_1$: tert-Butylthioazetidin-2-on

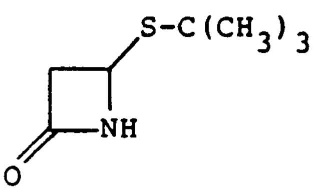

Zu einer Lösung von 9.689 g (75 mMol) 4-Acetoxyazetidin-2-on und 7.76 g (86 mMol) tert-Butylmercaptan in 75 ml trockenem THF wird bei -3 °C unter Rühren 13.13 g Diazabicycloundecen (DBU) innerhalb von 35 Minuten getropft, so dass die Reaktionstemperatur nicht über -1.5 °C steigt. Nach Aufbewahrung über Nacht bei 0 °C wird noch $1\frac{1}{2}$ Stunden bei Raumtemperatur gerührt. Verdünnen mit 500 ml Methylenchlorid, Waschen mit 100 ml gesättigter wässriger Kochsalzlösung, mit 100 ml 2N Salzsäure und weiteren 100 ml Kochsalzlösung, Trocknen der organischen Phase über Magnesiumsulfat und Eindampfen des Lösungsmittels im Vakuum ergibt einen festen Rückstand, der über 300g Kieselgel mit Toluol-Essigsäureethylester 2 : 1 chromatographiert wird. Nach Umkristallisieren des chromatographierten Produktes aus Methylenchlorid-Hexan werden 6.5 g reine Titelverbindung vom Schmp. 119 - 121 °C erhalten. IR-Spektrum in Methylenchlorid: 3410, 2955, 2905, 2865, 1770, 1460, 1410, 1370, 1340, 1160, 970, 925 $cm^{-1}$.

Alternative Herstellung von tert-Butylthioazetidin-2-on aus 4-Benzoyloxyazetidin-2-on

Zu einer Lösung von 9.3 ml (82.5 mMol) tert-Butylmercaptan in 37.5 ml Acetonitril wird bei 0 ° C 41.25 ml (82.5 mMol) 2N NaOH in Wasser zugetropft. Innerhalb von 25 Minuten wird dann eine Lösung (Erwärmen!) 14.32 g (75 mMol) 4-Benzoyloxyazetidin-2-on in 56 ml Acetonitril zugetropft, so dass die Reaktionstemperatur nicht über 0 °C steigt. Der intermediär beim Zutropfen entstandene Niederschlag löst sich vollständig auf beim weiteren Rühren bei 0 °C. Das Gemisch wird dann bei 0 °C über Nacht stehen gelassen wobei die Dünnschichtchromatographie auf Kieselgel mit Toluol-Essigsäureethylester (1 : 1) kein Ausgangsmaterial mehr anzeigt. Die gelbe Reaktionslösung wird mit 500 ml Methylenchlorid versetzt, die wässrige Phase abgetrennt und nochmals mit 100 ml Methylenchlorid extrahiert. Die vereinigten Extraktionslösungen werden nacheinander gewaschen mit je 100 ml 1 N HCl-Lösung, zweimal mit $NaHCO_3$-Lösung und einmal mit verdünnter NaCl-Lösung. Trocknen der organischen Phase über $MgSO_4$, Filtration und Eindampfen des Lösungsmittels im Vakuum ergibt 11.8 g (99 %) eines gelben kristallinen Rückstandes. Umkristallisation aus 160 ml Dibutylether bei 90 °C →0 °C ergibt 10.3 g (86 %) reine Titelverbindung vom Schmp. 119 - 120 °C.

Stufe B: (4-tert-Butylthio-2-oxo-1-azetidinyl)-essigsäure-p-nitrobenzylester

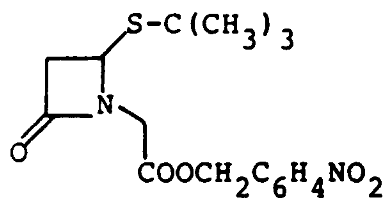

Zu einer Lösung Von 1.91g (12 mMol) tert-Butylthioazetidin-2-on in 6 ml trockenem N,N-Dimethylformamid (DMF) werden unter Rühren bei -70°C 14,4 ml 1N Lösung von Lithium-bis-trimethylsilylamid in Tetrahydrofuran (THF) getropft und anschliessend zum Gemisch eine Lösung von 4.93 g (18 mMol) Bromessigsäure-p-nitrobenzylester in 6 ml DMF zugetropft und das Gemisch noch 30 Minuten bei -30°C gerührt. Verdünnen des Reaktionsgemisches mit 100 ml Toluol, Waschen mit drei Portionen von je 50 ml Wasser, Trocknen der organischen Phase über Magnesiumsulfat und Eindampfen des Lösungsmittels im Vakuum ergibt 4.3 g festes Rohprodukt welches über 120 g Kieselgel mit Toluol-Essigsäureethylester (4 : 1) chromatographiert wird. Das gereinigte Produkt (2.6 g) wird aus 100 ml trockenem Isopropanol umkristallisiert. Ausbeute 2.09 g vom Schmp. 82.5-84°C. IR-Spektrum in Methylenchlorid: 2955, 1770, 1755, 1610, 1530, 1390, 1375, 1365, 1345, 1180, 1110, 945, 915, 855, 845 $cm^{-1}$.

Stufe C: 2-(4-tert-Butylthio-2-oxo-1-azetidinyl)-4,4-dimethyl 3-oxopentansäure-p-nitrobenzylester

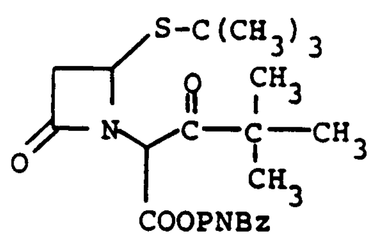

Zu einer Lösung von 1.059 mg (3 mMol) (4-tert-Butylthio-2-oxo-1-azetidinyl)-essigsäure-p-nitrobenzylester in 7 ml trockenem THF werden bei -70°C 6 ml einer frisch hergestellten 1M Lösung von Lithium-bis-trimethylsilylamid in THF zugetropft und anschliessend bei -70°C eine Lösung von 382 mg Pivaloylchlorid in 1 ml THF zugetropft und das Reaktionsgemisch noch während 30 Min bei der gleichen Temperatur gerührt. Das Gemisch mit 200 ml Toluol verdünnt und mit wenig wässriger Essigsäure versetzt. Waschen der organischen Phase mit 100 ml 2N wässriger Salzsäure und zweimaliges Waschen mit gesättigter Kochsalzlösung (100 ml), Trocknen der organischen Phase mit $MgSO_4$ und Eindampfen des Lösungsmittels im Vakuum ergibt ein dunkelrotes Öl. Die Reinigung des Rohproduktes über 40 g Kieselgel mit Toluol-Essigester (9 : 1) ergibt 795 mg eines nichtkristallinen Festkörpers. IR-Spektrum in Methylenchlorid: 2970, 1770, 1760, 1715, 1610, 1530, 1370, 1350, 1315, 1180, 995, 845 $cm^{-1}$.

Stufe $D_1$: 2-(4-Chlor-2-oxo-1-azetidinyl)-4,4-dimethyl-3-oxopentansäure-p-nitrobenzylester

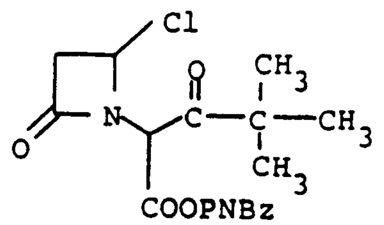

Eine Lösung von 439 mg (1.0 mMol) 2-(4-tert-Butylthio-2-oxo-1-azetidinyl)-4,4-dimethyl-3-oxopentansäure-p-nitrobenzylester in 20 ml trockenem Methylenchlorid wird auf -50 °C gekühlt und mit einer Lösung von 166 mg Chlor in 1.6 ml Tetrachlorkohlenstoff versetzt. Nach dreissigminütigem Rühren bei -50 °C wird das Lösungsmittel im Vakuum eingedampft und der Rückstand aus Methylenchlorid-Hexan umkristallisiert, wobei das Produkt (348 mg) als kristalliner Festkörper als 6 : 4 Gemisch der beiden diastereomeren Titelverbindungen erhalten wird.

Smp. 96 - 100.5 °C, Zersetzung. $^1$H-NMR ($CD_3CN$) : $\delta$ = 1.04 (s, ~ 5.4 H, t-Butyl I), 1.21 (s, ~ 3.6 H, t-Butyl II), 3.05-3.86 (m, 1 H, 3'-H), 5.29 (s, 2 H, -O-$CH_2$-Ar), 5.52 (s,~0.6 H, 2-H, I), 5.71 (s, ~ 0.4,2-H, II), 5.84 (dd, J=2 Hz, J=4 Hz~0.6H 4'-H, I), 5.98 (dd, J=2 Hz, J = 4 Hz, ~ 0.4 H, 4'-H, II), 7.51 (d, J = 9 Hz, ~ 0.8 H, Ar-H, II), 7.55 (d, J = 9 Hz, ~ 1.2 H, Ar-H, I), 8.19 (d, J = 9 Hz, 2 H, Ar-H, I und II).

Stufe $D_2$: 3-tert-Butyl-7-oxo-4-oxa-1-azabicyclo[3.2.0] hept-2-en-2-carbonsäure-p-nitrobenzylester

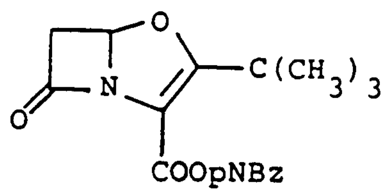

Das Gemisch der diastereomeren 2-(Chlor-2-oxo-1-azetidinyl)4,4-dimethyl-3-oxopentansäure-p-nitrobenzylester (348 mg, 0.91 mMol) wurde in trockenem THF (10 ml) gelöst und bei 0 °C mit 0.91 ml einer frisch zubereiteten 1 M Lösung von Kalium-tert-butylat in tert-Butanol versetzt und das Reaktionsgemisch bei 0 °C während 15 Minuten gerührt. Verdünnen mit 150 ml Benzol, dreimaliges Waschen mit je 50 ml 0.5 M Phosphatpufferlösung pH = 7, Trocknen der organischen Phase über $MgSO_4$ und Eindampfen des Lösungsmittels im Vakuum liefert einen fahlgelben Festkörper, der über 9 g Kieselgel mit Benzol-Essigsäureethylester 97 : 3 chromatographiert wird, wobei 237 mg Produkt erhalten werden. Umkristallisation aus Methylenchlorid-Hexan liefert 200 mg blassgelbe Kristalle vom Schmp. 142-144 °C.

$^1$H-NMR ($CD_3CN$): $\delta$ = 1.29 (s, 9 H, tert-Butyl), 3.40 (dd, J = 17 Hz, J = 1 Hz, 1 H, 6-H transständig), 3.79 (dd, J = 17 Hz, J = 2.5 Hz, 1 H, 6-H cisständig), 5.16 (d, J = 14 Hz, 1H, -O-$CH_2$-Ar), 5.42 (d, J = 14 Hz, 1 H, -O-$CH_2$-Ar), 5.85 (dd, J = 2.5 Hz, J = 1 Hz, 1 H, 5-H), 7.61 (d, J = 8.5 Hz, 2 H, Ar-H), 8.17 (d, J = 8.5 Hz, 2 H, Ar-H). IR-Spektrum in Methylenchlorid: 2955, 1804, 1715, 1610, 1585, 1525, 1350, 1315, 1200, 1165, 1145, 1120, 1080, 1040, 1025, 1015, 885, 855, 840 $cm^{-1}$. UV-Spektrum in Dioxan : $\lambda_{max}$ = 277 nm ($\epsilon$ = 15340).

Stufe E: 3-tert-Butyl-7-oxo-4-oxa-1-azabicyclo[3.2.0] hept-2-en-2-carbonsäure Na-Salz

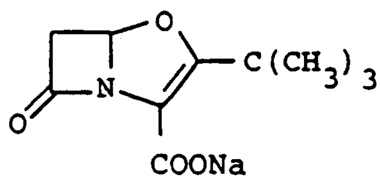

Zu einem auf 0°C gekülten Gemisch von 30 mg Palladium auf Kohle (10%), 2 ml Essigsäureethylester und einer Lösung von 4.7 mg (56 µmol) Natriumhydrogencarbonat in 1 ml Wasser in einer Wasserstoffatmoshäre wird über ein Spetum einen Lösung von 17.3 mg (50 µmol) 3-tert-Butyl-7-oxo-4-oxa-1-azabicyclo [3.2.0] hept-2-en-2-carbonsäure-p-nitrobenzylester in 1 ml Essigsäureethylester eingespritzt und hydriert. Innerhalb von 20 Minuten werden 5.4 ml Wasserstoff verbraucht, etwas mehr als die throretisch erforderliche Menge (4.6 ml). Das mehrphasige Gemisch wird filtriert unter Kühlung und das gekühlte (0°C) Filtrat zweimal mit je 3 ml Essigsäureethylester gewaschen. Die wässrige Lösung wird sofort lyophilisiert im Hochvakuum wobei 8.8 mg eines weissen Festkörpers erhalten werden. UV ($H_2O$): $\lambda_{max}$ = 269 nm ( $\epsilon$ = 5800) 360 MHz - $^1$H-NMR-Spektrum in $D_2O$: $\delta$ = 1.23 (s, 9H, tert-Butyl), 3.43 (dd, J=18 Hz, J = 1 Hz, 1 H, 6-H transständig), 3.72 (dd, J=18 Hz, J= 2.5 Hz, 1 H, 6-H cisständig), 5.82 (s, 1 H, 5-H).

Beispiel 2

Herstellung von 2-tert-Butyl-6-methyl-1-oxapen-2-em-3-carbonsäure, dem p-Nitrobenzylester und ihrem Natriumsalz

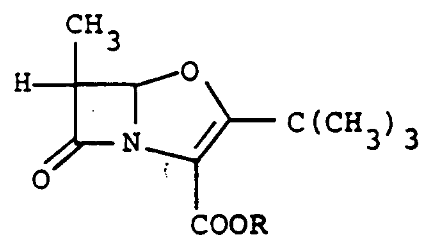

R = H, Na, p-NBz

Nach dem im Beispiel 1 beschriebenen Verfahren unter Verwendung der gleichen Reaktionsbedingungen wurde ausgehend von 4-Acetoxy-3-methylazetidin-2-on über die Stufen $A_1$, B und C 2-(4-tert-Butylthio-3-methyl-2-oxo-1-azetidinyl)-4,4-dimethyl-3-oxopentansäure-p-nitrobenzylester

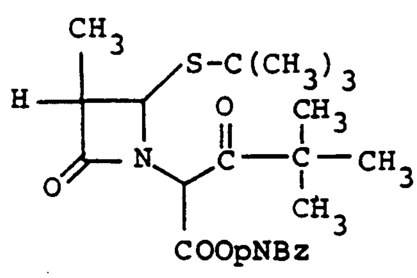

als nichtkristalliner Festkörper erhalten. IR-Spektrum in Methylenchlorid: 2955, 1765, 1760, 1720, 1610, 1525, 1460, 1380, 1365, 1350, 1315, 1205, 1180, 1120, 1050, 855, 840 $cm^{-1}$. UV-Spektrum in Ethanol: $\lambda_{max}$ = 264 nm ($\epsilon$ = 10160).

Stufe $D_1$: 2-(4-Chlor-3-methyl-2-oxo-1-azetidinyl)-4,4-dimethyl-3-oxopentansäure-p-nitrobenzylester

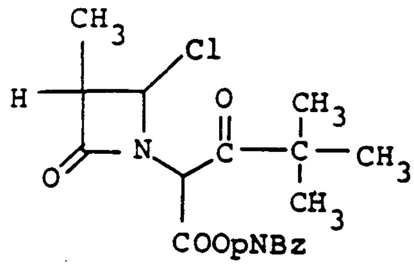

Nach dem im Beispiel 1 beschriebenen Verfahren unter Verwendung der gleichen Reaktionsbedingungen wurde ausgehend von 2-(4-tert-Butylthio-3-methyl-2-oxo-1-azetidinyl)-4,4-dimethyl-3-oxopentansäure-p-nitrobenzylester die Titelverbindung (Stufe $D_1$) als nichtkristalliner Festkörper (Gemisch von zwei Diastereomeren) erhalten. NMR-Spektrum in $CD_3CN$: $\delta$ = 1.19, 1.21 und 1.32 (3 Signale, 12 H), 3.59 - 3.98 (m, 1 H), 5.30 (s, 2 H), 5.50 (s, ~ 0.25 H), 5.70 (s, ~ 0.75 H), 5.94 d, J = 5 Hz, ~ 0.25 H), 6.09 (d, J = 5 Hz, ~ 0.75 H), 7.43 - 7.64 (m, 2 H), 8.17 (d, J = 9 Hz, 2 H).

Stufe $D_2$: 2-tert-Butyl-6-Methyl-1-oxapen-2-em-3-carbonsäure-p-nitrobenzylester (3-tert-Butyl-7-oxo-4-oxa-1-azabicyclo[3.2.0]hept-2-en-2-carbonsäure-p-nitrobenzylester)

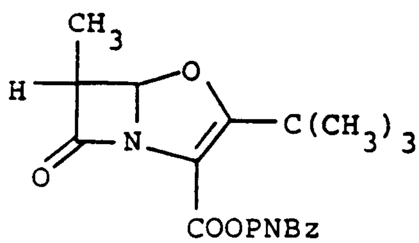

Nach dem im Beispiel 1 beschriebenen Verfahren unter Verwendung der gleichen Reaktionsbedingungen wurde ausgehend von 2-(4-Chlor-3-methyl-2-oxo-1-azetidinyl)-4,4-dimethyl-3-oxopentansäure-p-nitrobenzylester die Titelverbindung als nichtkristalliner Festkörper (Gemisch von cis-trans Isomeren) erhalten. IR-Spektrum in $CH_2Cl_2$: 2965, 1800, 1715, 1585, 1525, 1345, 1310, 1165, 1140, 1085, 1025, 1015, 935, 850 $cm^{-1}$. UV-Spektrum in Dioxan: $\lambda_{max}$ = 277 nm ($\epsilon$ = 15200).

Stufe E: 3-tert-Butyl-6-methyl-7-oxo-4-oxa-1-azabicyclo [3.2.0]-hept-2-en-2-carbonsäure, Na-Salz

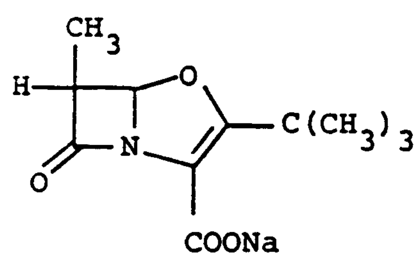

Nach dem im Beispiel 1 beschriebenen Verfahren unter Verwendung der gleichen Reaktionsbedingungen wurde ausgehend von 3-t-butyl-6-methyl-7-oxo-4-oxa-1-azabicyclo[3.2.0]hept-2-en-2-carbonsäure-p-nitrobenzylester die Titelverbindung als weisser Festkörper (Lyophilisat) erhalten. UV-Spektrum in $H_2O$: $\lambda_{max}$ = 260 nm ($\epsilon$ = 5800). $^1H$-NMR-Spektrum in $D_2O$: 1.24 und 1.27 (2s, 9 H), 1.38 (d, J = 7.5 Hz), 3.67 (q, J = 7.5 Hz, ~ 0.5 H, trans), 3.96 (dq, J = 7.5 Hz, J = 3 Hz, ~ 0.5 H, cis). 5.55 (s, ~ 0.5 H, trans), 5.80 (d, J

= 3 Hz, ~ 0.5 Hz, cis).

Beispiel 3

Herstellung von 2-tert-Butyl-6,6-dimethyl-1-oxapen-2-em-3-carbonsäure, dem p-Nitrobenzylester und ihrem Natriumsalz

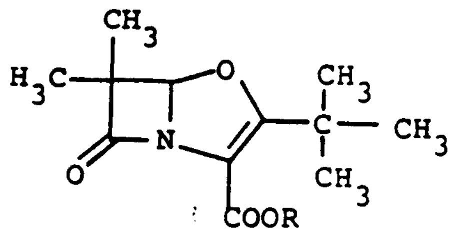

R = H, Na, pNBz

Nach dem im Beispiel 1 beschriebenen Verfahren unter Verwendung der gleichen Reaktionsbedingungen wurde ausgehend von 4-Acetoxy-3,3-dimethylazetidin-2-on über die Stufen $A_1$, B und C 2-(4-tert-Butylthio-3,3-dimethyl-2-oxo-1-azetidinyl)-4,4-dimethyl-3-oxopentansäure-p-nitrobenzylester als kri

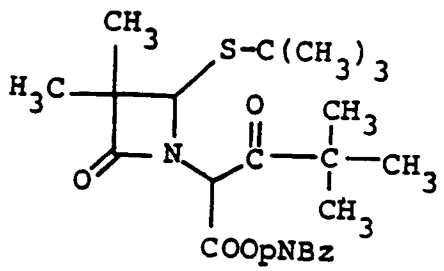

stalliner Festkörper vom Schmp. 87.5-90.5 °C aus Methylenchlorid-Hexan (Gemisch von zwei Diastereomeren) erhalten. IR-Spektrum in Methylenchlorid: 2955, 2865, 1765, 1755, 1715, 1610, 1525, 1460, 1390, 1370, 1350, 1315, 1185, 1135, 1105, 995, 850 $cm^{-1}$. UV-Spektrum in Ethanol: $\lambda_{max}$ = 264.5 nm ($\epsilon$ = 10930).

Stufe D: 3-tert-Butyl-6,6-dimethyl-7-oxo-4-oxa-1-azabicyclo[3.2.0]hept-2-en-2-carbonsäure-p-nitrobenzylester

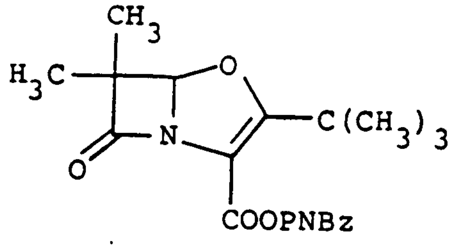

Eine Lösung von 930 mg (2.0 mMol) 2-(4-tert-Butylthio-3,3-dimethyl-2-oxo-1-azetidinyl)-4,4-dimethyl-3-oxopentansäure-p-nitrobenzylester in 460 ml trockenem Dimethoxyethan wurde zusammen mit 1046 mg (5.0 mMol) gelbem Quecksilber(II)oxid und 1358 mg (5.0 mMol) Quecksilber(II)chlorid kräftig gerührt und am Rückfluss drei Stunden gekocht. Nach dem Abkühlen wurde die gelbliche Lösung durch Cellite filtriert und auf etwa ein Zehntel des Volumens eingeengt. Nach Verdünnung mit 500 ml Benzol und Stehenlassen für zwei Tage bei 0 °C wurde von dem entstandenen farblosen Niederschlag abfiltriert und die erhaltene klare Lösung mit 250 ml gesättigter Kochsalzlösung, 250 ml 0.5 M Phosphatpufferlösung pH 7 und 250 ml gesättigter Kochsalzlösung gewaschen. Trocknung der organischen Phase mit Magnesiumsulfat, Einengen

der Lösung auf 50 ml und Stehenlassen bei 0 ° C, Abfiltrieren von wenig erneut angefallenem Niederschlag und Abziehen des Lösungsmittels im Vakuum ergaben ein gelbes, leicht getrübtes Öl. Chromatographie des Rohproduktes über 25 g Florisil mit Benzol-Essigsäureethylester (7 : 1) lieferte 560 mg reine Titelverbindung. Nach Umkristallisieren aus Methylenchlorid-Hexan beträgt der Schmp. 119 - 120.5 ° C. IR-Spektrum in Methylenchlorid: 2935, 2870, 1797, 1715, 1610, 1585, 1525, 1460, 1350, 1315, 1155, 1140, 1085, 1010, 850 $cm^{-1}$, UV-Spektrum in Dioxan: $\lambda_{max}$ = 278 nm ($\epsilon$ = 14980). Massenspektrum (20eV, 80 ° C): 374 $M^+$. Von dieser Substanz wurde eine Röntgenstrukturanalyse durchgeführt, welche die Struktur bestätigt.

Stufe E: 3-tert-Butyl-6,6-dimethyl-7-oxo-4-oxa-1-azabicyclo[3.2.0]hept-2-en-2-carbonsäure, Na-Salz

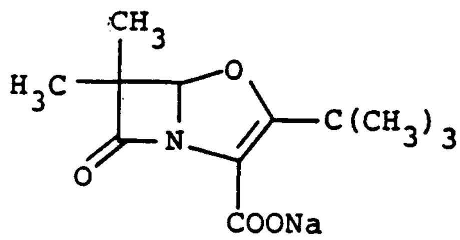

Nach dem im Beispiel 1 beschriebenen Verfahren unter Verwendung der gleichen Reaktionsbedingungen wurde ausgehend von 3-tert-Butyl-6,6-dimethyl-7-oxo-4-oxa-1-azabicyclo[3.2.0]hept-2-en-2-carbonsäure-p-nitrobenzylester die Titelverbindung als blassgelber Festkörper (Lyophilisat) erhalten. UV-Spektrum in $H_2O$: $\lambda_{max}$ = 261 nm. NMR-Spektrum in $D_2O$: 1.23 (s, 9 H), 1.26 (s, 3 H), 1.39 (s, 3 H), 5.50 (s, 1 H).

Beispiel 4

Herstellung von 2-(2-Chlor-1,1-dimethylethyl)-6,6-dimethyl-1-oxapen-2-em-2-carbonsäure, dem p-Nitrobenzylester und ihrem Na-Salz

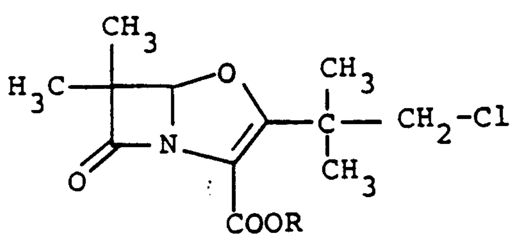

R = H, Na, pNBz

Nach dem im Beispiel 3 beschriebenen Verfahren bei Verwendung der gleichen Reaktionsbedingungen wurde ausgehend von 4-Acetoxy-3,3-dimethylazetidin-2-on unter Verwendung von Chlorpivaloylchlorid in Stufe C über die Stufen $A_1$, B, C, D die Verbindung

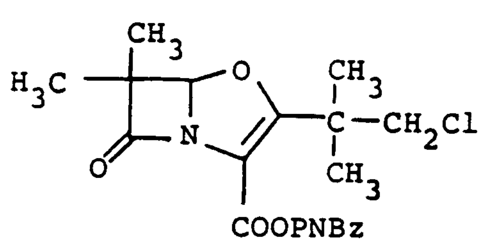

als nichtkristalliner Festkörper erhalten. IR-Spektrum in Methylenchlorid: 2930, 2875, 1803, 1710, 1590, 1525, 1460, 1370, 1350, 1315, 1255, 1160, 1130, 1115, 1090, 1010, 990, 920, 850 $cm^{-1}$

Stufe E: 3-(2-Chlor-1,1-dimethylethyl)-6,6-dimethyl-7-oxo-4-oxa-1-azabicyclo[3.2.0]hept-2-en-2-carbonsäure, Na-Salz

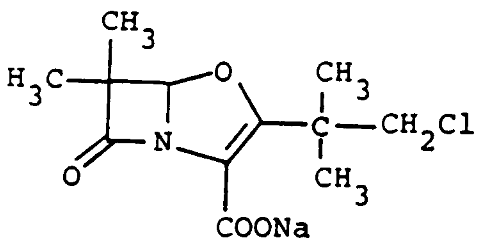

Ausgehend von dem entsprechenden p-Nitrobenzylester wurde nach dem im Beispiel 3 gegebenen Verfahren die Titelverbindung als nichtkristalliner farbloser Festkörper nach Lyophilisation in 60 % Ausbeute erhalten. NMR-Spektrum in $D_2O$: $\delta$ = 1.27, 1.30, 1.33 und 1.39 (4s, 12 H), 3.74 (d, J = 10 Hz, 1 H), 4.05 (d, J = 10 Hz, 1 H) 5.52 (s, 1 H). UV-Spektrum in $H_2O$: $\lambda_{max}$ = 265 nm ($\epsilon$ = 5800).

Beispiel 5

Herstellung von 6,6-Dimethyl-3-(1-methyl-1-phenylethyl)-7-oxo-4-oxa-1-azabicyclo[3.2.0]hept-2-en-2-carbonsäure, ihrem Na-Salz und ihrem p-Nitrobenzylester

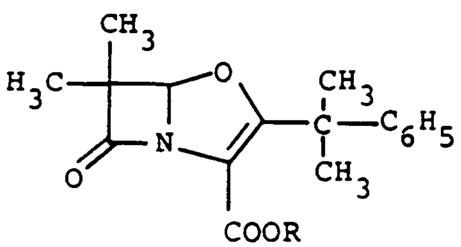

R = H, Na, pNBz

Ausgehend von 2-(4-tert-butylthio-3,3-dimethyl-2-oxoazetidinyl)-essigsäure-p-nitrobenzylester und 2-Methyl-2-phenylpropionsäurechlorid wurde nach dem im Beispiel 1 beschriebenen Verfahren über die Stufen C, $D_1$ und $D_2$ die Titelverbindung (p-Nitrobenzylester) als nichtkristalliner leicht gelblicher Festkörper erhalten. IR-Spektrum in $CH_2Cl_2$: 2930, 2875, 1800, 1720, 1600, 1525, 1350, 1320, 1145, 1085, 1075 $cm^{-1}$.

Stufe E: 6,6-Dimethyl-3-(1-methyl-1-phenylethyl)-7-oxo-4-oxa-1-azabicyclo[3.2.0]hept-2-en-2-carbonsäure, Na-Salz

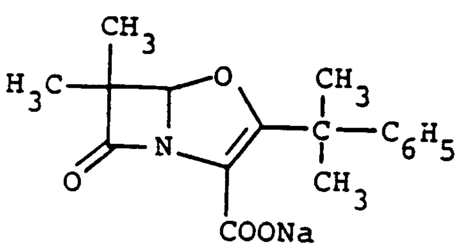

Ausgehend von dem entsprechenden p-Nitrobenzylester wurde nach dem im Beispiel 1 beschriebenen Verfahren die Titelverbindung als farbloser Festkörper nach Lyphilisation in 50 % Ausbeute erhalten. UV-Spektrum in $H_2O$: $\lambda_{max}$ = 263 nm ($\epsilon$ = 5600).

Beispiel 6

Herstellung von 6,6-Dimethyl-3-(1,1-diphenylethyl)-7-oxo-4-oxa-1-azabicyclo[3.2.0]hept-2-en-2-carbonsäure, ihrem Na-Salz und ihrem p-Nitrobenzylester

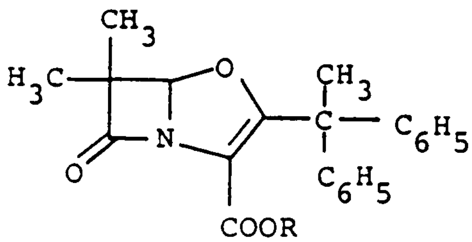

R = H, Na,
pNBz

Ausgehend von 2-(4-tert-butylthio-3,3-dimethyl-2-oxoazetidinyl)-essigsäure-p-nitrobenzylester und 2,2-Diphenylpropionsäurechlorid wurde nach dem im Beispiel 1 beschriebenen Verfahren über die Stufen C, $D_1$ und $D_2$ die Titelverbindung (p-Nitrobenzylester) als farbloser Festkörper erhalten. IR-Spektrum in $CH_2Cl_2$: 2930, 2875, 1805, 1725, 1600, 1525, 1350, 1315, 1150, 1085, 1075 $cm^{-1}$.

Stufe E: 6,6-Dimethyl-3-(1,1-diphenylethyl)-7-oxo-4-oxa 1-azabicyclo[3.2.0]hept-2-en-2-carbonsäure, Na-Salz

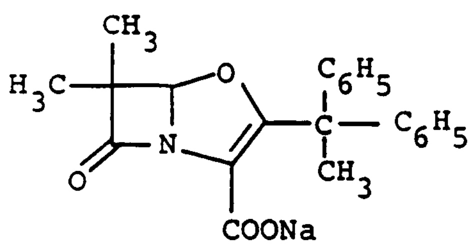

Ausgehend von dem entsprechenden p-Nitrobenzylester wurde nach dem im Beispiel 1 beschriebenen Verfahren die Titelverbindung als farbloser Festkörper nach Lyophilisation in 63 % Ausbeute erhalten. UV-Spektrum in $H_2O$: $\lambda_{max}$ = 265 nm ($\epsilon$ = 6000)

Beispiel 7

Herstellung von 6,6-Dimethyl-3-[1-methyl-1-(2-thienyl)ethyl]-7-oxo-4-oxa-1-azabicyclo[3.2.0]hept-2-en-2-carbonsäure, ihrem Na-Salz und ihrem p-Nitrobenzylester

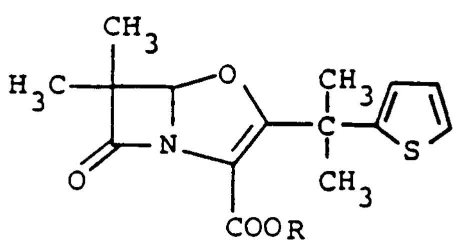

R = H, Na
pNBz

Ausgehend von 2-(4-tert-butylthio-3,3-dimethyl-2-oxoazetidinyl)-essigsäure-p-nitrobenzylester und 2-Methyl-2-thienylpropionsäurechlorid wurde nach dem im Beispiel 1 beschriebenen Verfahren über die Stufen C, $D_1$ und $D_2$ die Titelverbindung (p-Nitrobenzylester) als leicht gelblicher Festkörper erhalten. IR-Spektrum in $CH_2Cl_2$: 2930, 1795, 1715, 1590, 1520, 1350, 1310, 1140, 1080 $cm^{-1}$.

Stufe E: 6,6-Dimethyl-3-[1-Methyl-1-(2-thienyl)ethyl]-7-oxo-4-oxa-1-azabicyclo[3.2.0]hept-2-en-2-carbonsäure, Na-Salz

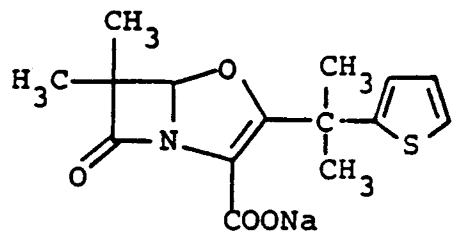

Ausgehend von dem entsprechenden p-Nitrobenzylester wurde nach dem im Beispiel 1 beschriebenen Verfahren die Titelverbindung als farbloser nichtkristalliner Festkörper (Lyophilisat) in 70 % Ausbeute erhalten. UV-Spektrum in $H_2O$: $\lambda_{max}$ = 270 nm ($\epsilon$ = 6500).

Beispiel 8

Herstellung von 3-(2-Amino-1,1-dimethylethyl)-7-oxo-4-oxa-1-azabicyclo[3.2.0]hept-2-en-2-carbonsäure

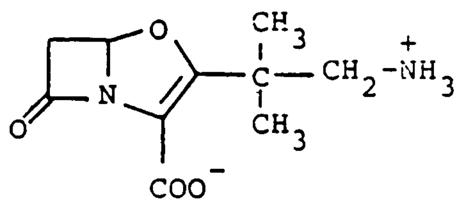

Stufe C: 2-(4-tert-Butylthio-2-oxo-1-azetidinyl)-5-chlor-4,4-dimethyl-3-oxopentansäure-p-nitrobenzylester

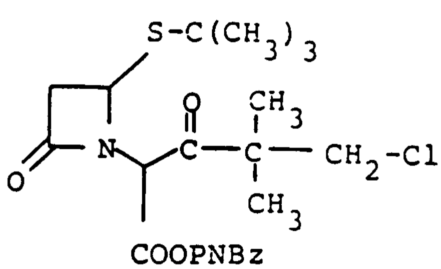

Zu einem Gemisch von 1.06 g (3 mMol) 2-(4-tert-Butylthio-2-oxo-1-azetidinyl)-essigsäure-p-nitrobenzylester und 410 µl (3.17 mMol) Chlorpivaloylchlorid in 38 ml absolutem Tetrahydrofuran wird unter Rühren bei -70 °C 6 ml einer 1 M Lösung von Lithium-bis(trimethylsilylamid) in THF langsam zugetropft und das Gemisch noch 30 Minuten bei -70 °C gerührt. Das Reaktionsgemisch wird verdünnt mit 250 ml Toluol, mit 10 ml 2 N wässriger HCl und 100 ml gesättigter NaCl-Lösung versetzt. Nach Abtrennen wird die organische Phase nochmals mit 100 ml gesättigter NaCl Lösung gewaschen, dann über $MgSO_4$ getrocknet, filtriert und das Lösungsmittel am Vakuum-Rotationsverdampfer entfernt. Der nichtkristalline Rückstand wird über 46 g Kieselgel mit Toluol-Essigsäureethylester (19 : 1) chromatographiert, wobei 980 mg nichtkristalline Titelverbindung erhalten werden. IR in $CH_2Cl_2$: 2930 1770, 1760, 1725, 1615, 1530, 1465, 1370, 1250, 1215, 1190, 1110, 1040, 1000, 847 $cm^{-1}$.

Umwandlung einer Gruppe $R^3$; 5-Azido-2-(4-tert-butylthio-2-oxo-1-azetidinyl)-4,4-dimethyl-3-oxopentansäure-p-nitrobenzylester

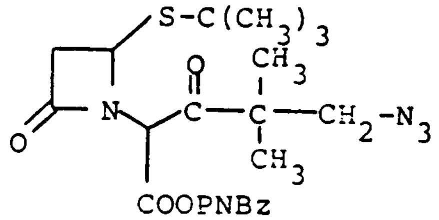

Ein Gemisch von 236 mg (0.5 mMol) 2-(4-tert-Butylthio-2-oxo-1-azetidinyl)-5-chlor-4,4-dimethyl-3-oxopentansäure-p-nitrobenzylester und 200 mg (1.04 mMol) Triton B-azid in 0.3 ml DMF wird während 20 Stunden bei Raumtemperatur gerührt, dann mit Toluol verdünnt und zweimal mit Wasser gewaschen. Die wässrige Phase wird mit wenig Toluol extrahiert und die vereinigten organischen Phasen über $MgSO_4$ getrocknet. Filtration und Eindampfen des Lösungsmittels im Vakuum liefert 290 mg eines farblosen Rückstands IR-Spektrum in $CH_2Cl_2$: 2930, 2860, 2110, 1775, 1755, 1720, 1610, 1530, 1350, 1180, 1120, 850 $cm^{-1}$.

Stufe $D_1$: 5-Azido-2-(4-chlor-2-oxo-1-azetidinyl)-4,4-dimethyl-3-oxopentansäure-p-nitrobenzylester

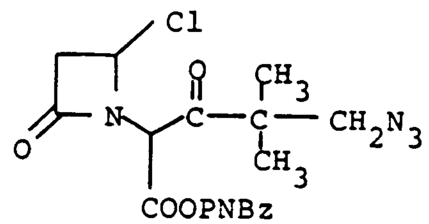

Zu einer Lösung von 110 mg 5-Azido-2-(4-tert-Butylthio-2-oxo-1-azetidinyl)-4,4-dimethyl-3-oxopentansäure-p-nitrobenzylester in 9 ml Methylenchlorid wird bei -60 °C 286 µl einer 1.14 g/10 ml enthaltenden Lösung von Chlor in Tetrachlorkohlenstoff zugegeben und das Gemisch anschliessend während zwei Stunden bei -60 °C gerührt. Die Reaktionslösung wird eingedampft im Rotationsverdampfer wobei 119 mg gelbes Öl erhalten werden. IR-Spektrum in $CH_2Cl_2$: 2930, 2860, 2105, 1780, 1755, 1720, 1610, 1530, 1350, 1190 $cm^{-1}$.

Stufe $D_2$: 3-(2-Azido-1,1-dimethylethyl)-7-oxo-4-oxa-1-azabicyclo[3.2.0]hept-2-en-2-carbonsäure-p-nitrobenzylester

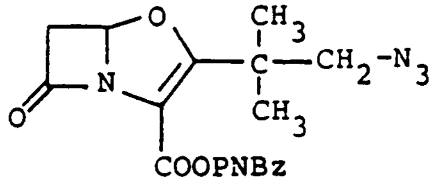

Eine Lösung von 107 mg (0.23 mMol) 5-Azido-2-(4-chlor-2-oxo-1-azetidinyl)-4,4-dimethyl-3-oxopentansäure-p-nitrobenzylester in 4.5 ml trockenem Tetrahydrofuran wird bei -30 °C mit 313 µl einer 0.75 M Lösung von Kaliumtert-butylat in tert-Butanol versetzt und die Lösung während 30 Minuten bei -30 °C gerührt. Das Reaktionsgemisch wird dann verdünnt mit 20 ml Essigsäureethylester, und mit 10 ml

Wasser und 10 ml NaCl-Lösung gewaschen. Die wässrigen Phasen werden mit 10 ml Essigsäureethylester extrahiert und die vereinigten organischen Phasen über $MgSO_4$ getrocknet, filtriert und dann im Vakuum eingedampft. Dabei werden 105 mg eines gelben Rückstandes erhalten, der aus Methylenchlorid-Diisopropylether kristallisiert wird. Ausbeute 61 mg vom Schmelzpunkt 81-82 °C. IR-Spektrum in $CH_2Cl_2$: 2950, 2860, 2110, 1810, 1720, 1590, 1530, 1370, 1320, 1085, 1020 $cm^{-1}$.

Stufe E: 3-(2-Amino-1,1-dimethylethyl)-7-oxo-4-oxa-1-azabicyclo[3.2.0]hept-2-en-2-carbonsäure

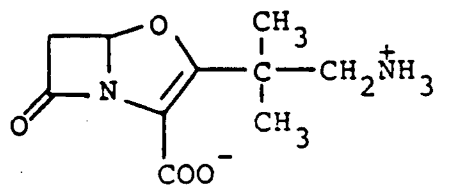

Zu einem vorhydrierten Gemisch von 60 mg Palladium auf Kohle (10 %) in 1 ml Essigsäureethylester und 0.7 ml Wasser wird bei 0 °C durch ein Septum eine Lösung von 21 mg (0.054 mMol) 3-(2-Azido-1,1-dimethylethyl)-7-oxo-4-oxa-1-azabicyclo[3.2.0]hept-2-en-2-carbonsäure-p-nitrobenzylester in 1 ml Essigsäureethylester mit einer Spritze zugegeben. Nach 20 Minuten Reaktionszeit werden 5.7 ml Wasserstoff aufgenommen (theoretische Menge: 4.9 ml). Das Reaktionsgemisch wird bei 0 °C filtriert und die wässrige Phase zweimal mit 2 ml vorgekühltem Essigsäureethylester gewaschen. Die wässrige Phase enthält 8.96 mg der Titelverbindung. UV-Spektrum in $H_2O$: $\lambda_{max}$ = 271 nm ($\epsilon$ = 5000)

Beispiel 9

Herstellung von 3-[1,1-Dimethyl-2-((1-methyl-1,2,3,4-tetrazol-5-yl)-thio)-ethyl]-7-oxo-4-oxa-1-azabicyclo-[3.2.0]hept-2-en-carbonsäure, ihrem Na-Salz und ihrem p-Nitrobenzylester

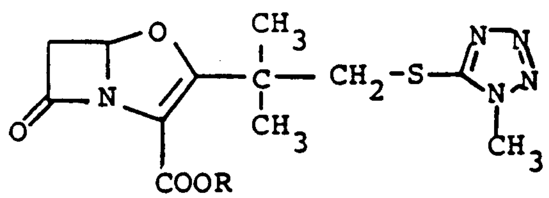

R = H, Na, pNBz

Umwandlung einer Gruppe $R^3$; 2-(4-tert-Butylthio-2-oxo-1-azetidinyl)-4,4-dimethyl-5-[(1-methyl-1,2,3,4-tetrazol-5-yl)-thio-3-oxopentansäure-p-nitrobenzylester

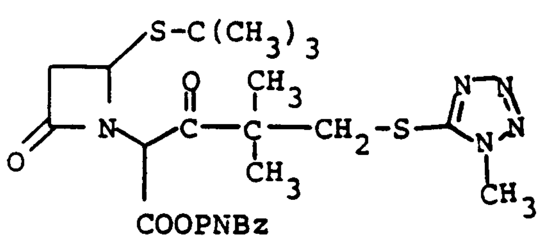

Ein Gemisch von 118 mg (0.25 mMol) 2-(4-tert-Butylthio-2-oxo-1-azetidinyl)-5-chlor-4,4-dimethyl-3-oxopentansäure-p-nitrobenzylester und 82 mg (0.59 mMol) 1-Methyl-5-mercapto-1,2,3,4-tetrazol Na-Salz werden in 0.2 ml Dimethylformamid während 20 Stunden bei Raumtemperatur gerührt. Das Reaktionsgemisch wird direkt auf eine Chromatographie-Säule mit 6 g Kieselgel mit Toluol-Essigsäureethylester (19 : 1) chromatographiert, wobei 60 mg reine Titelverbindung erhalten werden als nichtkristalliner Festkörper.

Stufe $D_1$ und $D_2$: 3-[1,1-Dimethyl-2-((1-methyl-1,2,3,4-tetrazol-5-yl) thio)-ethyl]-7-oxo-4-oxa-1-azabicyclo-[3.2.0]hept-2-en-2-carbonsäure-p-nitrobenzylester

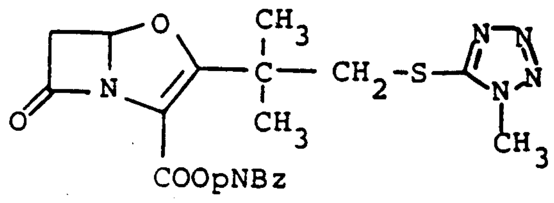

Ausgehend von 2-(4-tert-Butylthio-2-oxo-1-azetidinyl)-4,4-dimethyl-5-[(1-methyl-1,2,3,4-tetrazol-5-yl)thio]-3-oxo-pentansäure-p-nitrobenzylester wurde nach dem im Beispiel 1 gegebenen Verfahren über die Stufen $D_1$ und $D_2$ die Titelverbindung erhalten nach Chromatographie über Kieselgel mit Toluol-Essigsäureethylester (3 : 1) IR-Spektrum in $CH_2Cl_2$: 2930, 2860, 1810, 1720, 1590, 1525, 1350, 1320, 1175, 1120, 1030, 1015 $cm^{-1}$.

Stufe E: 3-[1,1-Dimethyl-2-((1-methyl-1,2,3,4-tetrazol-5-yl)-thio)-ethyl]-7-oxo-4-oxa-1-azabicyclo[3.2.0] hept-2-en-2-carbonsäure, Na-Salz

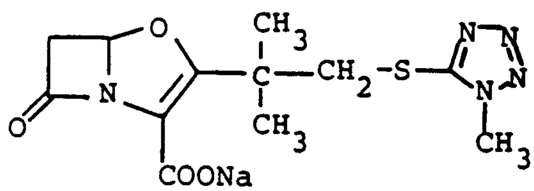

Ausgehend von dem entsprechenden p-Nitrobenzylester wurde nach dem im Beispiel 1 beschriebenen Verfahren über die Stufe E die Titelverbindung als weisser Festkörper (nach Lyophilisation) erhalten. UV-Spektrum in $H_2O$: Starke Endabsorption, Schulter bei 270 nm ($\epsilon$ = 4000).

Beispiel 10

Herstellung von 3-[1-Methyl-1-(2-thienyl)-ethyl]-7-oxo-4-oxa-1-azabicyclo[3.2.0]hept-2-en-2-carbonsäure, ihrem Na-Salz und ihrem p-Nitrobenzylester

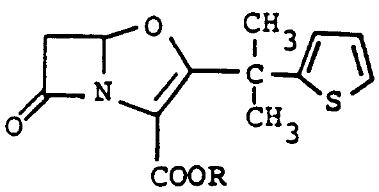

R = H, Na, pNBz

Ausgehend von 2-(4-tert-butylthio-3,3-dimethyl-2-oxo-azetidinyl)-essigsäure-p-nitrobenzylester und 2-Methyl-2-thienylpropionsäurechlorid wurde nach dem im Beispiel 1 beschriebenen Verfahren über die Stufen C, $D_1$ und $D_2$ die Titelverbindung (p-Nitrobenzylester) erhalten als farbloser nichtkristalliner Festkörper. IR-Spektrum in $CH_2Cl_2$: 2930, 1800, 1720, 1605, 1530, 1350, 1080 $cm^{-1}$.

Stufe E: 3-[1-Methyl-1-(2-thienyl)-ethyl]-7-oxo-4-oxa-1-azabicyclo[3.2.0]hept-2-en-2-carbonsäure, Na-Salz

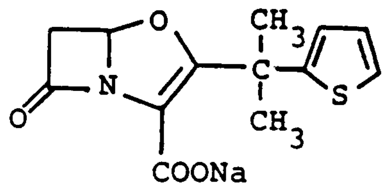

Ausgehend vom entsprechenden p-Nitrobenzylester wurde nach dem im Beispiel 1 beschriebenen Verfahren die reine Titelverbindung als weisser Festkörper erhaltern. UV-Spektrum in $H_2O$: $\lambda_{max} = 270$ nm ($\epsilon$ = 6500)

Beispiel 11

Herstellung von 2-(2-Acetoxy-1,1-dimethylethyl)-7-oxo-4-oxa-1-azabicyclo[3.2.0]hept-2-en-2-carbonsäure, ihrem Na-Salz und ihrem p-Nitrobenzylester

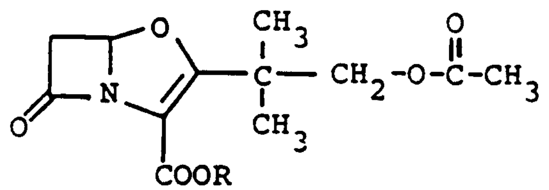

R = H, Na
pNBz

Ausgehend von 2-(4-tert-butylthio-2-oxoazetidinyl)-essigsäure-p- nitrobenzylester und $\beta$-Acetoxypivalinsäurechlorid wurde nach dem im Beispiel 1 beschriebenen Verfahren die Titelverbindung (p-Nitrobenzylester) über die Stufen C, $D_1$, und $D_2$ als leicht gelblicher nichtkristalliner Festkörper erhalten. IR-Spektrum in $CH_2Cl_2$: 2950, 2850, 1810, 1740, 1720, 1590, 1550, 1340, 1080 $cm^{-1}$.

Stufe E: 2-(2-Acetoxy-1,1-dimethylethyl)-7-oxo-4-oxa-1-azabicyclo[3.2.0]hept-2-en-2-carbonsäure, Na-Salz

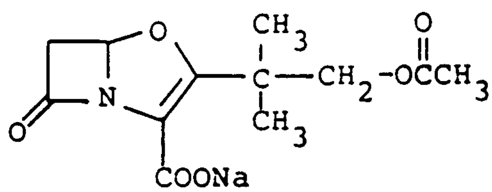

Ausgehend von dem entsprechenden p-Nitrobenzylester wurde nach dem im Beispiel 1 beschriebenen Verfahren über die Stufe E die Titelverbindung in 70 % Ausbeute als nichtkristalliner weisser Festkörper (Lyophilisat) erhalten. UV-Spektrum in $H_2O$: $\lambda_{max}$ = 270 nm ($\epsilon$ = 6300). NMR-Spektrum in $D_2O$: $\delta$ = 1.25 (s, 3 H), 1.26 (s, 3 H) 2.06 (s, 3 H), 3.40 (dd, J = 1 Hz, J = 17 Hz, 2 H), 3.72 (dd, J = 3 Hz, J = 17 Hz, 2 H), 4.23 (AB, J = 17 Hz, 2 H), 5.80 (dd, J = 1 Hz, J = 3 Hz, 1 H).

Beispiel 12

Herstellung von 3-tert-Butyl-6-(1-hydroxyethyl)-7-oxo-4-oxa-1-azabicyclo[3.2.0]hept-2-en-2-carbonsäure, ihrem Na-Salz und ihrem p-Nitrobenzylester

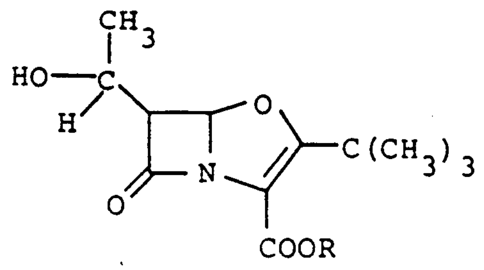

R = H, Na
pNBz

Stufe $A_2$: 4-Methylthio-3-[1-(p-nitrobenzyloxycarbonyloxy)ethyl]-azetidin-2-on

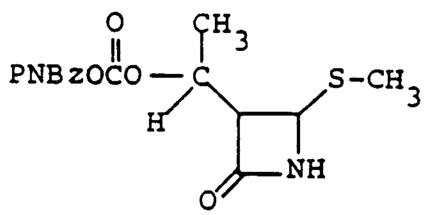

Zu einer Lösung von 4.48 g (11.1 mMol) 4-(2-hydroxyethylsulfonyl)-3-[1-(p-nitrobenzyloxycarbonyloxy)-ethyl]azetidin-2-on in 11 ml Acetonitril und 11 ml $H_2O$ werden bei 0 °C 1.17g (16. 7mMol) Methylmercaptan-Na-Salz gegeben und das Gemisch während 20 Minuten bei 0 °C gerührt. Die Reaktionsmischung wird verdünnt mit 100 ml Methylenchlorid und 25 ml $H_2O$ und nach Abtrennen der organischen Phase die wässrige Phase dreimal mit je 25 ml Methylenchlorid extrahiert. Die vereinigten organischen Phasen werden über $MgSO_4$ getrocknet, filtriert und das Lösungsmittel im Vakuum entfernt, wobei 3.80 g Titelverbindung als leicht gelbliche nichtkristalline Verbindung erhalten werden. NMR-Spektrum in $CDCl_3$: $\delta$ = 1.45 (d, J = 7 Hz, 3 H), 2.12 (s, 3 H), 3.3 (dd, J = 7 Hz, J = 2 Hz), 4.70 (d, J = 2 Hz, 1 H), 5.12 (m, 1 H), 5.20 (s, 2 H), 6.45 (breites s, 1 H) 7.50 (d, J = 8.5 Hz, 2 H), 8.20 (d, J = 8.5 Hz, 2 H).

Stufe B: 2-[4-Methylthio-3-(1-(p-nitrobenzyloxycarbonyloxy)-ethyl)-2-oxoazetidinyl]-essigsäure-p-nitrobenzylester

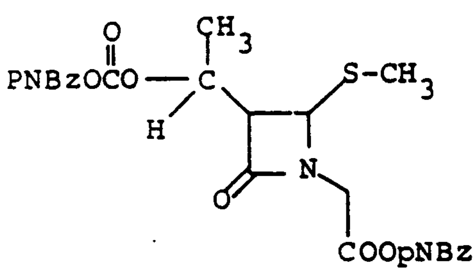

Zu einem Gemisch von 680 mg (2 mMol) 4-Methylthio-3-[1-(p-nitrobenzyloxycarbonyloxy)-ethyl]-azetidin-2-on und 602 mg (2.2 mMol) Bromessigsäure-p-nitrobenzylester in 2 ml trockenem Tetrahydrofuran und 2 ml trockenem DMF werden unter Rühren bei -70 °C 2.2 ml einer 1 M Lösung von Lithium-bis-(trimethylsilyl)amidinnerhalb von 5 Minuten zugegeben. Das Reaktionsgemisch wird während 30 Minuten bei -70 °C gerührt, mit 30 ml Essigsäureethylester und 70 ml Toluol verdünnt und zweimal mit verdünnter NaCl-Lösung gewaschen. Die organische Phase wird über $MgSO_4$ getrocknet, filtriert und das Lösungsmittel in Vakuum abgedampft. Der Rückstand wird über 65 g Kieselgel mit Toluol-Essigester 4 : 1 chromatographiert wobei 520 mg eines gelben Öls erhalten werden. IR-Spektrum in $CH_2Cl_2$: 2920, 2850 1765, 1750, 1605, 1520, 1355, 1345 $cm^{-1}$.

Stufe C: 4,4-Dimethyl-2-[4-Methylthio-3-(1-(p-nitrobenzyloxy-carbonyloxy)-ethyl)-2-oxoazetidinyl]-3-oxopentansäure-p-nitrobenzylester

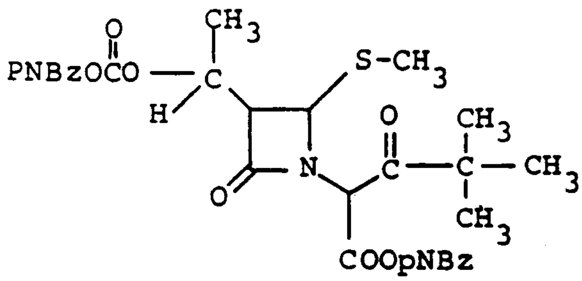

Zu einem Gemisch 275 mg (0.515 mMol) 2-[4-Methylthio-3-(1-(p-nitrobenzyloxycarbonyloxy)-ethyl)-2-oxoazetidinyl]-essigsäure-p-nitrobenzylester und 67 µl (0.55 mMol) Pivalinsäurechlorid in 6.7 ml trockenem Tetrahydrofuran wird bei -70 °C unter Rühren 1.1 ml 1 M Lithium-bis(trimethylsilyl)amid innerhalb von 5 Minuten zugegeben und das Reaktionsgemisch noch während 30 Minuten bei -70 °C gerührt und dann mit 40 ml Toluol verdünnt und mit 30 ml 2 N HCl, dann zweimal mit je 40 ml NaCl-Lösung gewaschen. Die organische Phase wird über $MgSO_4$ getrocknet, filtriert und das Lösungsmittel im Vakuum entfernt. Der Rückstand wird über 10 g Kieselgel mit Toluol-Essigsäureethylester (9 : 1) chromatographiert, wobei 268 mg eines weissen nichtkristallinen Festkörpers erhalten werden. IR-Spektrum in $CH_2Cl_2$: 2930, 2850, 1775, 1760, 1720, 1610, 1535, 1350 $cm^{-1}$.

Stufe $D_1$: 2-[4-Chlor-3-(1-(p-nitrobenzyloxycarbonyloxy)ethyl)-2-oxoazetidinyl]-4,4-dimethyl-3-oxopentansäure-p-nitrobenzylester

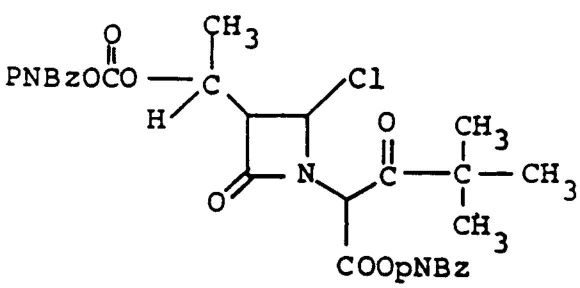

Eine Lösung von 244 mg (0.395 mMol) 4,4-Dimethyl-2-[4-Methylthio-3-(1-(p-nitrobenzyloxy-carbonyloxy)-ethyl)-2-oxoazetidinyl]-3-oxopentansäure-p-nitrobenzylester in 16 ml Methylenchlorid werden mit 660 µl einer 850 mg in 10 ml enthaltenden Lösung von Chlor in Tetrachlorkohlenstoff versetzt bei -60 °C. Die schwach gelbe Lösung wird während 2 Stunden bei -60 °C gerührt und das Lösungsmittel im Vakuum entfernt, wobei 236 mg eines farblosen nichtkristallinen Festkörpers erhalten werden. IR-Spektrum in $CH_2Cl_2$: 2930, 2850, 1795, 1765, 1725, 1620, 1530, 1355 $cm^{-1}$.

Stufe $D_2$: 3-tert-Butyl-6-[1-(p-nitrobenzyloxycarbonyloxy)ethyl]-7-oxo-4-oxa-1-azabicyclo[3.2.0]hept-2-en-carbonsäure-p-nitrobenzylester

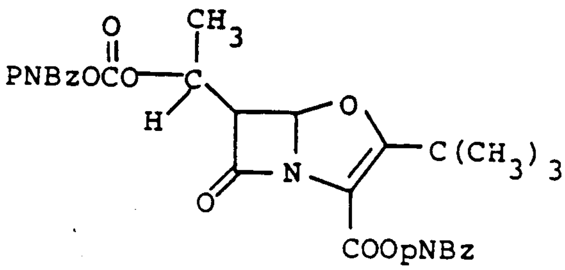

Eine Lösung von 214 mg 2-[4-Chlor-3-(1-(p-nitrobenzyloxycarbonyloxy)-ethyl)-2-oxoazetidinyl]-4,4-dimethyl-3-oxopentansäure-p-nitrobenzylester in 7 ml trockenem Tetrahydrofuran wird bei -30 °C unter Rühren mit 476 µl 0.75 M Kalium-tert-butylat in tert-Butanol versetzt und das Reaktionsgemisch anschliessend während 30 Minuten bei -30 °C gerührt. Die Reaktionslösung wird mit 40 ml Essigsäureethylester verdünnt und anschliessend mit 40 ml verdünnter NaCl- und mit 40 ml gesättigter NaCl-Lösung gewaschen. Trocknen der organischen Phase über $MgSO_4$, Filtration und Eindampfen des Lösungsmittels im Vakuum liefert 198 mg Rückstand, der über 6 g Kieselgel mit Toluol-Essigsäureethylester chromatographiert wird, wobei 183 mg eines farblosen nichtkristallinen Festkörpers (Titelverbindung) erhalten werden. IR-Spektrum in $CH_2Cl_2$: 3030, 2950, 1805, 1755, 1720, 1610, 1580, 1530, 1350, 1320, 1090 $cm^{-1}$.

Stufe E: Gleichzeitige Entfernung von zwei Schutzgruppen, 3-tert-Butyl-6-(1-hydroxyethyl)-7-oxo-4-oxa-1-azabicyclo[3.2.0]hept-2-en-2-carbonsäure, Na-Salz

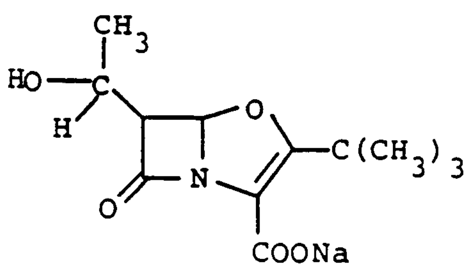

Zu einem vorhydrierten Gemisch von 84 mg Pd/C (10 %), 4.6 mg $NaHCO_3$ in 1 ml Essigsäureethylester und 0.7 ml $H_2O$ wird eine Lösung von 28 mg (0.05 mMol) 3-tert-Butyl-6-[1-(p-nitrobenzylcarbonyloxy)-ethyl]-7-oxo-4-oxa-1-azabicyclo[3.2.0]hept-2-en-2-carbonsäure-p-nitrobenzylester in 1 ml Essigsäureethylester durch ein Septum mit einer Spritze bei 0 °C zugegeben und das Gemisch bei 0 °C während 40 Minuten hydriert, wobei 8.0 ml $H_2$ aufgenommen werden (theor. Wert ca. 8.8 ml $H_2$). Das Gemisch wird bei 0 °C filtriert und die wässrige Phase noch zweimal mit je 2 ml vorgekühltem Essigsäureethylester gewaschen und dann im Hochvakuum lyophilisiert, wobei 11 mg eines weissen nichtkristallinen Feststoffes (Titelverbindung) erhalten werden. UV-Spektrum in $H_2O$: $\lambda_{max}$ = 278 nm ($\epsilon$ = 5500).

Beispiel 13

Herstellung von 3-tert-Butyl-6-hydroxymethyl-7-oxo-4-oxa-1-azabicyclo[3.2.0]hept-2-en-2-carbonsäure und ihrem Na-Salz

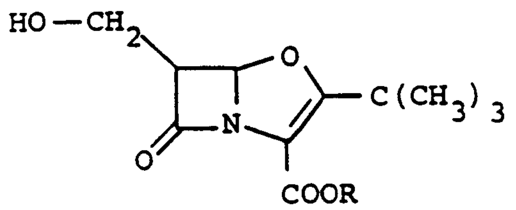

R = H, Na, p-NBz

Ausgehend von 4-(2-hydroxyethylsulfonyl)-3-(p-nitrobenzyloxy-carbonyloxymethyl)-azetidin-2-on wurde nach dem im Beispiel 12 beschriebenen Verfahren über die Stufen $A_2$, B, C, $D_1$, $D_2$ und E die Titelverbindung (Na-Salz) als weisser nichtkristalliner Festkörper (lyophilisiert) erhalten. UV-Spektrum in $H_2O$: $\lambda_{max}$ = 275 nm ($\epsilon$ = 5500).

Auf analoge Weise wurde das Kaliumsalz der 3-tert-Butyl-6-hydroxymethyl-7-oxo-4-oxa-1-azabicyclo-[3.2.0]hept-2-en-2-carbonsäure hergestellt.

Beispiel 14

Herstellung von 2-tert-Butyl-6-ethyliden-1-oxapen-2-em-3-carbonsäure, ihrem Na-Salz und ihrem p-Nitrobenzylester

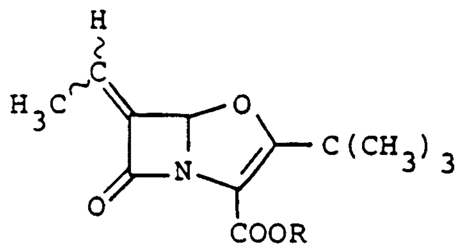

R = H, Na p-NBz

Ausgehend von 4-tert-Butylthio-3-ethylidenazetidin-2-on wird über die Stufen B, C, D , und $D_2$ unter Anwendung der im Beispiel 12 angegebenen Reagenzien und Reaktionsbedingungen die Titelverbindung (p-Nitrobenzylester) erhalten (3-tert-Butyl-6-ethyliden-4-oxa-7-oxo-1-azabicyclo[3.2.0]hept-2-en-2-carbonsäure-p-nitrobenzylester). IR-Spektrum in $CH_2Cl_2$: 1800, 1720, 1585, 1525, 1345, 1310, 1165 $cm^{-1}$.

Stufe E: 3-tert-Butyl-6-ethyliden-4-oxa-7-oxo-1-azabicyclo[3.2.0]hept-2-en-2-carbonsäure, Na-Salz

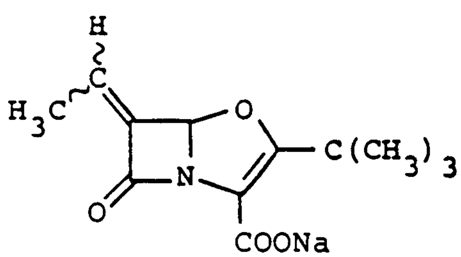

Ausgehend vom entsprechenden p-Nitrobenzylester wird unter Anwendung der im Beispiel 12 angegebenen Reaktionsbedingungen unter Verwendung von Pd auf $PbCO_3$ anstatt Pd auf C als Katalysator nach 4 Stunden Reaktionszeit die Titelverbindung erhalten (weisses Lyophilisat). UV-Spektrum in $H_2O$: $\lambda_{max}$ = 272 ($\epsilon$ = 5100).

Beispiel 15

Herstellung pharmazeutischer Zubereitungen

Eine Einheitsdosisform wird hergestellt, indem man 120 mg 3-tert-Butyl-6-(1-hydroxyethyl)-7-oxo-4-oxa-1-azabicyclo[3.2.0]hept-2-en-2-carbonsäure, Na-Salz mit 20 mg Lactose und 5 mg Magnesiumstearat vermischt und das 145 mg Gemisch in eine Nr. 3 Gelatinekapsel gibt. Ähnlich kann man, wenn man mehr aktiven Bestandteil und weniger Lactose verwendet, andere Dosisformen herstellen und in Nr. 3 Gelatinekapseln füllen; und sollte es erforderlich sein, mehr als 145 mg Bestandteile zusammen zu vermischen, können größere Kapseln, wie auch komprimierte Tabletten und Pillen, ebenfalls hergestellt werden. Die folgenden Beispiele erläutern die Herstellung pharmazeutischer Zubereitungen.

| Tablette | Tablette, mg |
|---|---|
| 3-tert-Butyl-6-(1-hydroxyethyl)-7-oxo-4-oxa-1-azabicyclo[3.2.0]hept-2-en-2-carbonsäure, Na-Salz | 125 |
| Maisstärke, U.S.P. | 6 |
| Dicalciumphosphat | 192 |
| Lactose, U.S.P. | 190 |

Der aktive Bestandteil wird mit dem Dicalciumphosphat, Lactose und etwa der Hälfte der Maisstärke vermischt, Das Gemisch wird dann mit 6 mg 15 %iger Maisstärkepaste granuliert und grob gesiebt. Es wird bei 45 °C getrocknet und erneut durch Siebe mit lichten Maschenweiten von 1.00 mm (Nr. 16 screens) gesiebt. Der Rest der Maisstärke und das Magnesiumstearat werden zugegeben und das Gemisch wird zu Tabletten, die je 800 mg wiegen und einen Durchmesser von etwa 1.27 cm (0.5 in.) besitzen, verpreßt.

| Parenterale Lösung | |
|---|---|
| Ampulle | |
| 3-tert-Butyl-6-(1-hydroxyethyl)-7-oxo-4-oxa-1-azabicyclo[3.2.0]hept-2-en--2-carbonsäure, Na-Salz<br>steriles Wasser | 500 mg<br>2 ml |
| Ophthalmische Lösung | |
| 3-tert-Butyl-6-(1-hydroxyethyl)-7-oxo-4-oxa-1-azabicyclo[3.2.0]hept-2-en--2-carbonsäure, Na-Salz<br>Hydroxypropylmethylcellulose<br>steriles Wasser bis zu | 100 mg<br>5 mg<br>1 ml |
| Otische Lösung | |
| 3-tert-Butyl-6-(1-hydroxyethyl)-7-oxo-4-oxa-1-azabicyclo[3.2.0]hept-2-en--2-carbonsäure, Na-Sal<br>Benzalkoniumchlorid<br>steriles Wasser bis zu | 100 mg<br>0.1 mg<br>1 ml |
| Topische Creme bzw. Salbe | |
| 3-tert-Butyl-6-(1-hydroxyethyl)-7-oxo-4-oxa-1-azabicyclo[3.2.0]hept-2-en--2-carbonsäure, Na-Salz<br>Polyäthylenglykol 4000 U.S.P.<br>Polyäthylenglykol 400 U.S.P. | 100 mg<br>400 mg<br>1.0 g |

Der aktive Bestandteil in den obigen Zubereitungen kann allein oder zusammen mit anderen biologisch aktiven Bestandteilen, z.B. mit anderen antibakteriellen Mitteln, wie Lincomycin, einem Penicillin, Streptomycin, Novobiocin, Gentamicin, Neomycin, Colistin und Kanamycin, oder mit anderen therapeutischen Mitteln, wie Probenecid, vermischt werden.

## Patentansprüche

**Patentansprüche für folgende Vertragsstaaten : AT, BE, CH, DE, FR, GB, IT, LI, NL, SE**

**1.** Verbindungen der Strukturformeln

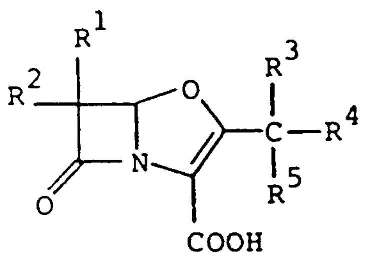

bzw.

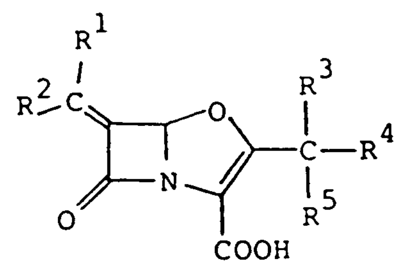

dadurch gekennzeichnet, daß $R^1$ und $R^2$ unabhängig voneinander Wasserstoff oder über Kohlenstoff-Kohlenstoff-Einfachbindungen mit dem übrigen Molekülteil gebundene pharmazeutisch annehmbare Gruppen bedeuten, die ausgewählt werden aus substituiertem oder unsubstituiertem Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl-, Alkenyl- oder Alkinyl-Molelülteile 1 bis 6 Kohlenstoffatome, die Cycloalkyloder die Cycloalkenyl-Molekülteile 3 bis 6 und die Aryl-Molekülteile 6 bis 10 Kohlenstoffatome enthalten, Heteroaralkyl, Heteroaralkenyl, Heteroaralkinyl, Alkylheteroaryl, Heterocyclyl, Heterocyclylalkyl, Heterocyclylalkenyl, Heterocyclalkinyl, Alkylheterocyclyl, worin die vorhergehenden Alkyl-, Alkenyl- oder die Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können:
Hydroxy, Hydroxyalkyloxy, Aminoalkyloxy, Amidinoalkyloxy, Alkyloxy, Acyloxy, Aryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, Amino oder Monoalkylamino, Dialkylamino, Oxo, Oximino oder Alkylimino, Tetraalkyl ammonium, Cycloalkylamino, Arylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und dadurch gekennzeichnet, dass $R^3$, $R^4$ und $R^5$ unabhängig voneinander ausgewählt werden aus den vorhergehend genannten, über Kohlenstoff-Kohlenstoff-Einfachbindungen an den übrigen Molekülteil gebundenen pharmazeutisch annehmbaren Gruppen.

**2.** Verbindungen nach Anspruch 1, dadurch gekennzeichnet, daß $R^3$, $R^4$ und $R^5$ die oben angegebenen Bedeutungen haben und $R^1$ und $R^2$ unabhängig voneinander Wasserstoff, Alkyl, Hydroxyalkyl oder Dihydroxyalkyl mit jeweils 1 bis 6 Kohlenstoffatomen bedeuten.

**3.** Verbindungen nach Anspruch 2, dadurch gekennzeichnet, daß $R^1$ und $R^2$ die darin angegebenen Bedeutungen haben, $R^3$ und $R^4$ Methyl bedeuten und $R^5$ ausgewählt wird unter den Gruppen:

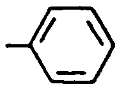
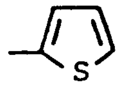
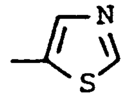

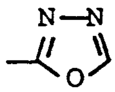
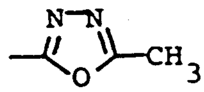
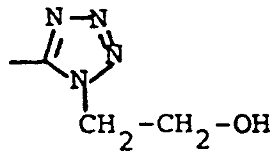

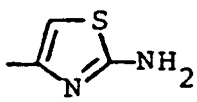
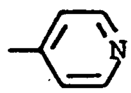
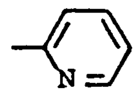

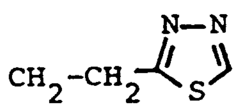
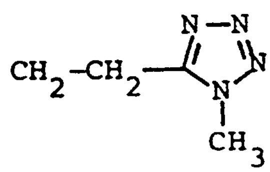

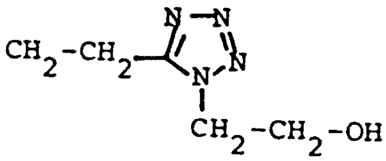 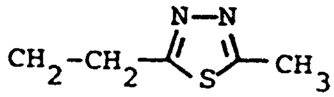

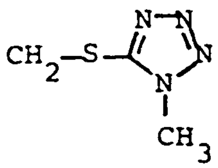 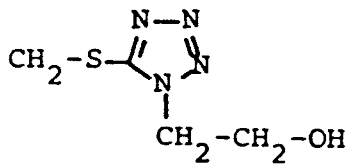 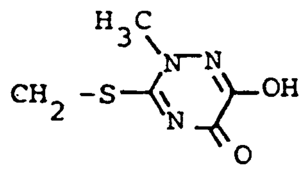

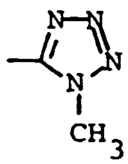 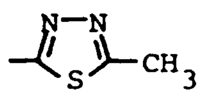 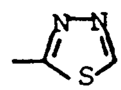

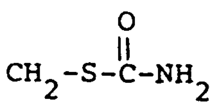 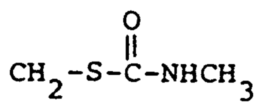 $CH_2-S-CH_3$

$CH_2-S-CH_2-CH_2-NH_2$ 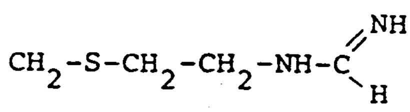

$CH_2-NH_2$ 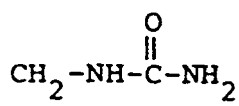 $CH_2-NH-CHO$

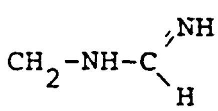 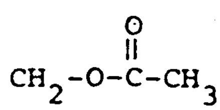 $CH_2-O-CHO$

$CH_2-Cl$ 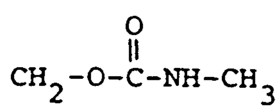 $CH_2-N_3$

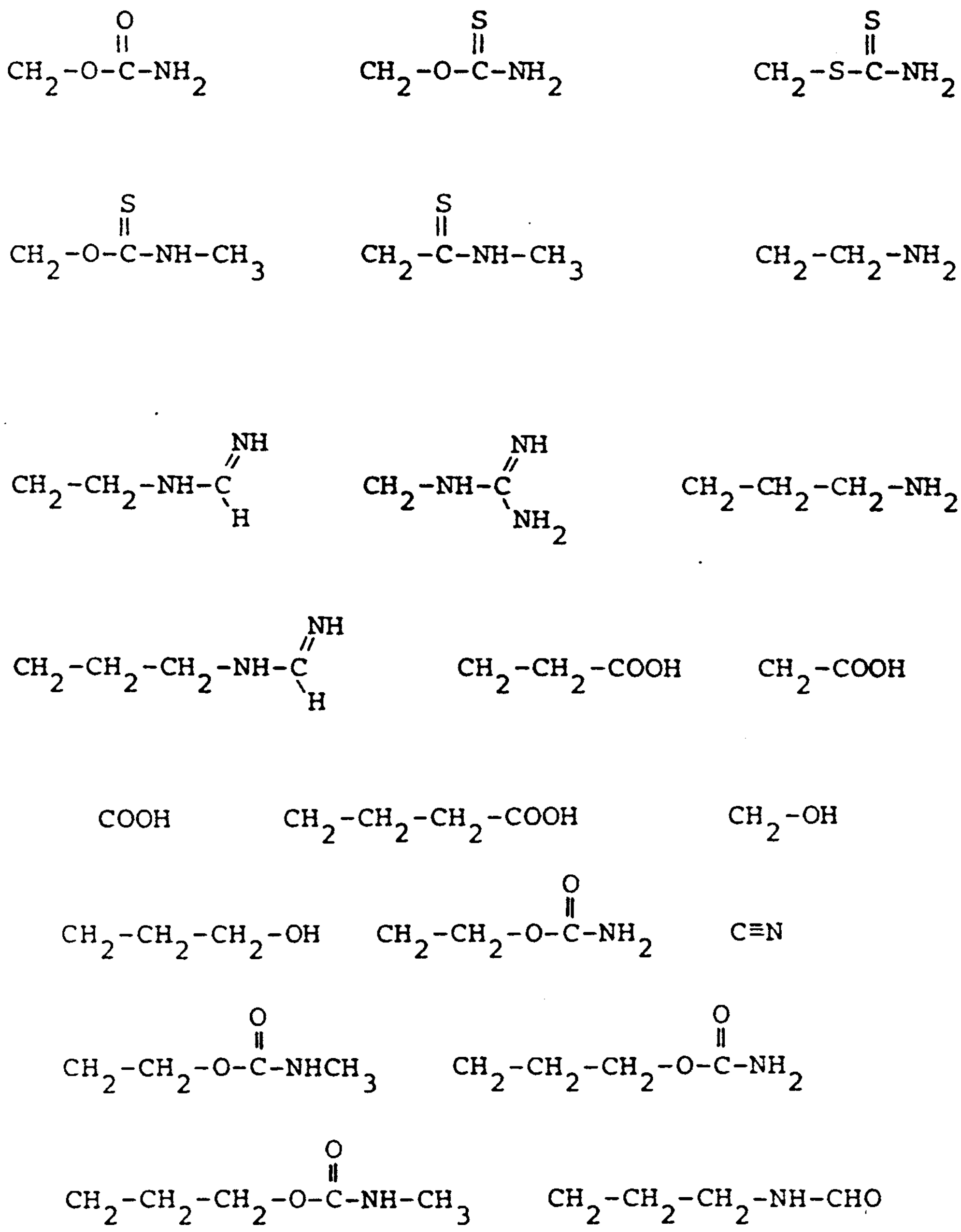

**4.** Verfahren zur Herstellung einer Verbindung nach Anspruch 1 dadurch gekennzeichnet, dass man eine Verbindung der Formel

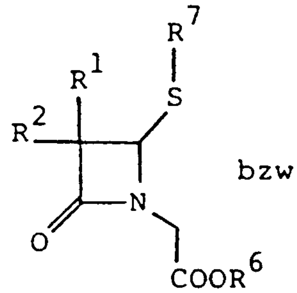

bzw.

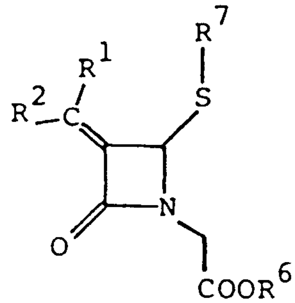

worin $R^7$ eine substituierte oder unsubstituierte verzweigte oder unverzweigte Alkylgruppe, Arylgruppe,

Aralkylgruppe, Heteroaryl oder Heteroaralkylgruppe mit bis zu 10 Kohlenstoffatomen bedeutet, wobei die Substituenten ausgewählt werden aus der Gruppe: Niederalkyl, Acyloxy, Chlor, Brom, Nitro, Niederalkyloxy und Cyano und die Heteroatome des Heteroaryl- oder Heteroaralkylteils ausgewählt werden aus der Gruppe: Sauerstoff, Stickstoff und Schwefel und der Estermolekülteil $R^6$ Benzyl, p-Nitrobenzyl, Methyl, tert.-Butyl-dimethylsilyl, Trichlorethyl oder Pivaloyloxymethyl, Allyl, Methallyl, 2-Oxoethyl, 2-Oxopropyl, 2-Methylthio-ethyl oder Buten-1-yl bedeutet mit einer starken Base und einem Säurehalogenid der allgemeinen Formel

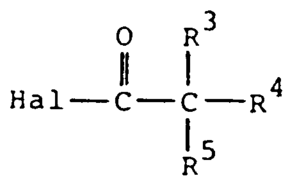

umsetzt und damit zu einem Keton der Formel

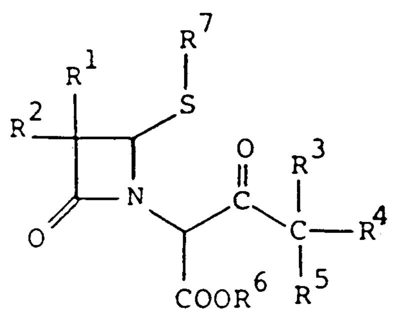

bzw.

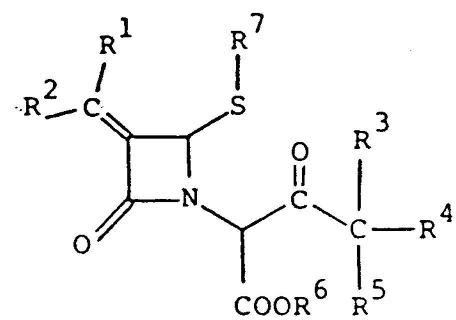

gelangt.

**5.** Verfahren zur Herstellung einer Verbindung nach Anspruch 1 dadurch gekennzeichnet, dass man ein Keton der Formel

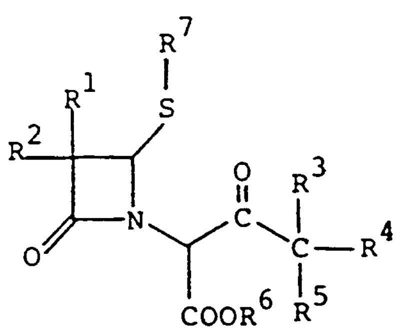

bzw.

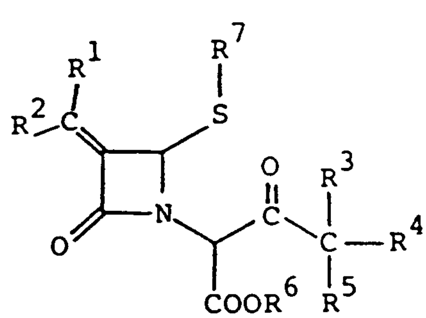

worin $R^7$ eine substituierte oder unsubstituierte verzweigte oder unverzweigte Alkylgruppe, Arylgruppe, Aralkylgruppe, Heteroaryl oder Heteroaralkylgruppe mit bis zu 10 Kohlenstoffatomen bedeutet, wobei die Substituenten ausgewählt werden aus der Gruppe: Niederalkyl, Acyloxy, Chlor, Brom, Nitro, Niederalkyloxy und Cyano und die Heteroatome des Heteroaryl- oder Heteroaralkylteils ausgewählt werden aus der Gruppe: Sauerstoff, Stickstoff und Schwefel und der Estermolekülteil $R^6$ die in Anspruch 4 angegebene Bedeutung hat, mit einem Halogenierungsmittel zu einem Keton der Formel

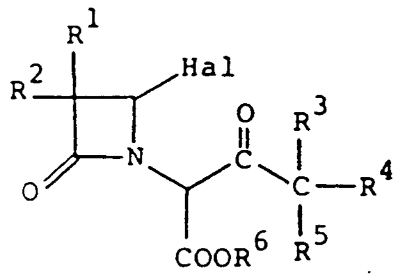

bzw.

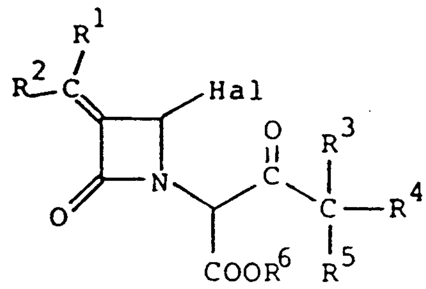

umsetzt, worin Hal Chlor, Brom oder Iod bedeutet.

**6.** Verfahren zur Herstellung einer Verbindung nach Anspruch 1 dadurch gekennzeichnet, dass man ein Keton der Formel

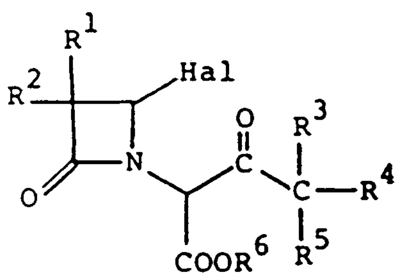

bzw.

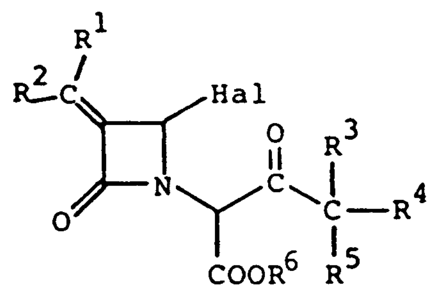

worin der Estermolekülteil $R^6$ die in Anspruch 4 angegebene Bedeutung hat, mit einer Base zu einer Verbindung der Formel

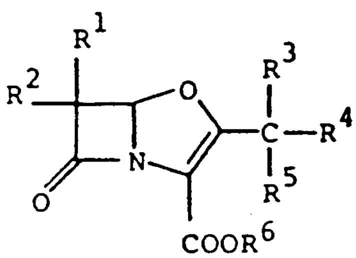

bzw.

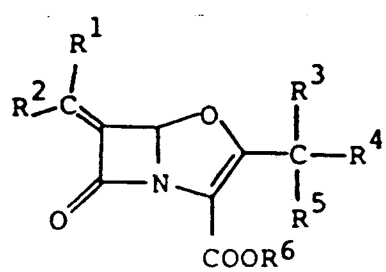

umsetzt.

**7.** Verfahren zur Herstellung einer Verbindung nach Anspruch 1 dadurch gekennzeichnet, dass man ein Keton der Formel

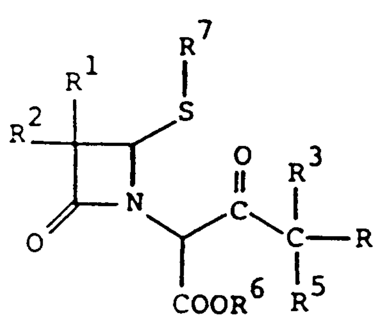

bzw.

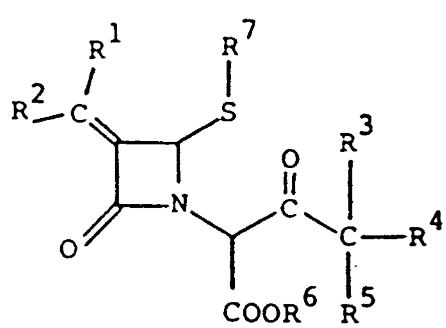

worin $R^7$ eine substituierte oder unsubstituierte verzweigte oder unverzweigte Alkylgruppe, Arylgruppe, Aralkylgruppe, Heteroaryl oder Heteroaralkylgruppe mit bis zu 10 Kohlenstoffatomen bedeutet, wobei die Substituenten ausgewählt werden aus der Gruppe: Niederalkyl, Acyloxy, Chlor, Brom, Nitro, Niederalkyloxy und Cyano und die Heteroatome des Heteroaryl- oder Heteroaralkylteils ausgewählt werden aus der Gruppe: Sauerstoff, Stickstoff und Schwefel und der Estermolekülteil $R^6$ die in Anspruch 4 angegebene Bedeutung hat, mit einem Quecksilber(II)salz zu einer Verbindung der Formel

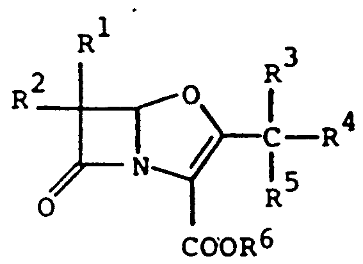

bzw.

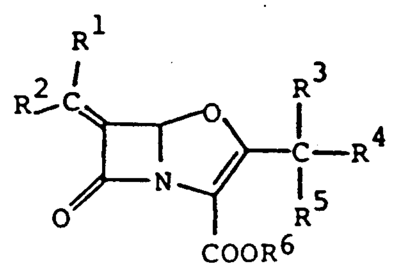

umsetzt.

8. Verfahren zur Herstellung einer Verbindung nach Anspruch 1 dadurch gekennzeichnet, dass man eine Verbindung der Formel

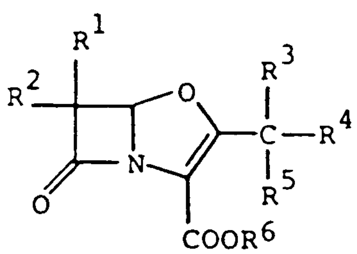

bzw.

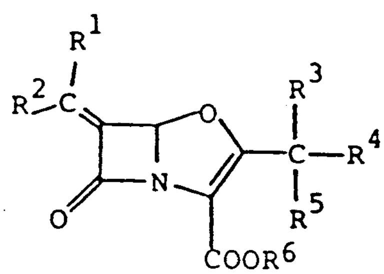

worin der Estermolekülteil $R^6$ die in Anspruch 4 angegebene Bedeutung hat, durch Abspaltung der Schutzgruppe $R^6$ zu einer Verbindung der Formel

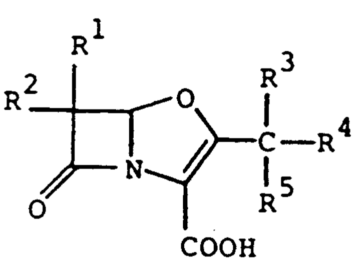

I

bzw.

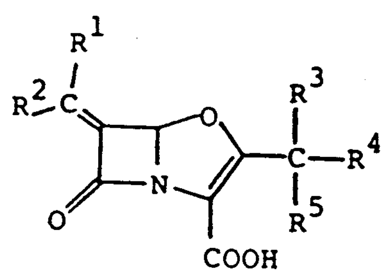

II

oder ihren pharmazeutisch annehmbaren Salzen umsetzt.

9. Pharmazeutische Zubereitung, dadurch gekennzeichnet, daß sie eine Verbindung nach mindestens einem der Ansprüche 1 bis 3 und einen pharmazeutischen Träger dafür enthält.

10. Pharmazeutische Zubereitung, dadurch gekennzeichnet, daß sie in Dosiseinheitsform vorliegt und einetherapeutisch wirksame Menge einer Verbindung nach mindestens einem der Ansprüche 1 bis 3 und einen pharmazeutischen Träger dafür enthält.

**11.** Verbindung der Strukturformel

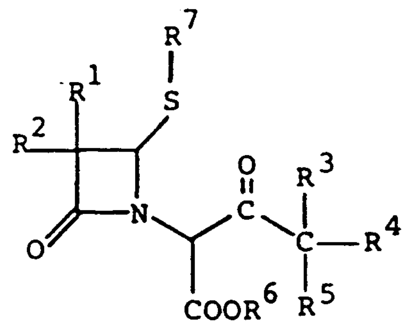

bzw.

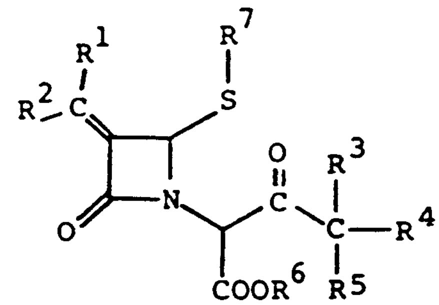

worin $R^1$ bis $R^5$ die in Anspruch 1 gegebenen Bedeutungen besitzen und worin $R^7$ eine substituierte oder unsubstituierte verzweigte oder unverzweigte Alkylgruppe, Arylgruppe, Aralkylgruppe, Heteroaryl oder Heteroaralkylgruppe mit bis zu 10 Kohlenstoffatomen bedeutet, wobei die Substituenten ausgewählt werden aus der Gruppe: Niederalkyl, Acyloxy, Chlor, Brom, Nitro, Niederalkyloxy und Cyano und die Heteroatome des Heteroaryl- oder Heteroaralkylteils ausgewählt werden aus der Gruppe: Sauerstoff, Stickstoff und Schwefel und die Estergruppe $R^6$ die in Anspruch 4 angegebene Bedeutung hat.

**12.** Verbindung der Strukturformel

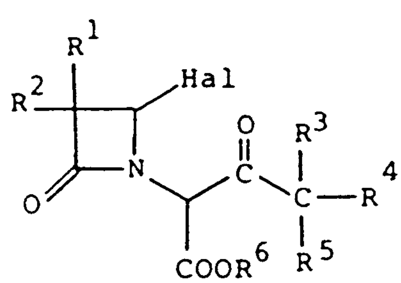

bzw.

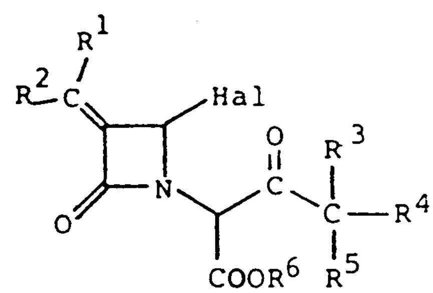

worin $R^1$ bis $R^5$ die in Anspruch 1 gegebenen Bedeutungen besitzen und worin der Estermolekülteil die in Anspruch 4 angegebene Bedeutung hat.

**13.** Verbindung der Strukturformel

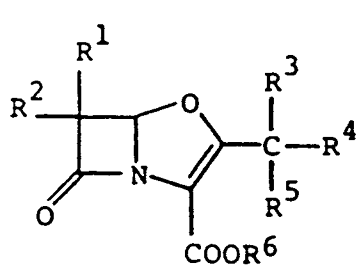

bzw.

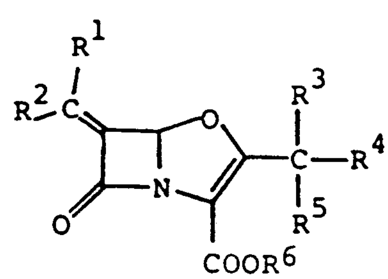

worin $R^1$ bis $R^5$ die Anspruch 1 gegebenen Bedeutungen besitzen und der Estermolekülteil $R^6$ die in Anspruch 4 angegebene Bedeutung hat.

**Patentansprüche für folgende Vertragsstaaten : ES, GR**

1. Verfahren zur Herstellung von Verbindungen der Strukturformeln (I)

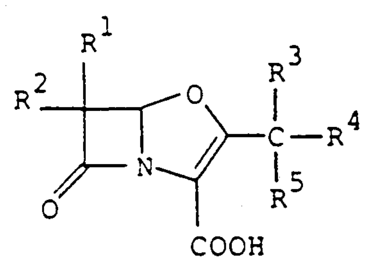

bzw.

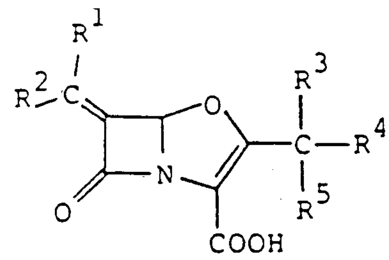

in welchen $R^1$ und $R^2$ unabhängig voneinander Wasserstoff oder über Kohlenstoff-Kohlenstoff-Einfachbindungen mit dem übrigen Molekülteil gebundene pharmazeutisch annehmbare Gruppen bedeuten, die ausgewählt werden aus substituiertem oder unsubstituiertem Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl-, Alkenyl- oder Alkinyl-Molelülteile 1 bis 6 Kohlenstoffatome, die Cycloalkyl-oder die Cycloalkenyl-Molekülteile 3 bis 6 und die Aryl-Molekülteile 6 bis 10 Kohlenstoffatome enthalten, Heteroaralkyl, Heteroaralkenyl, Heteroaralkinyl, Alkylheteroaryl, Heterocyclyl, Heterocyclylalkyl, Heterocyclylalkenyl, Heterocyclalkinyl, Alkylheterocyclyl, worin die vorhergehenden Alkyl-, Alkenyl- oder die Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können:
Hydroxy, Hydroxyalkyloxy, Aminoalkyloxy, Amidinoalkyloxy, Alkyloxy, Acyloxy, Aryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, Amino oder Monoalkylamino, Dialkylamino, Oxo, Oximino oder Alkylimino, Tetraalkylammonium, Cycloalkylamino, Arylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und in welchen $R^3$, $R^4$ und $R^5$ unabhängig voneinander ausgewählt werden aus den vorhergehend genannten, über Kohlenstoff-Kohlenstoff-Einfachbindungen an den übrigen Molekülteil gebundenen pharmazeutisch annehmbaren Gruppen,
dadurch gekennzeichnet, daß man eine Verbindung der Formel

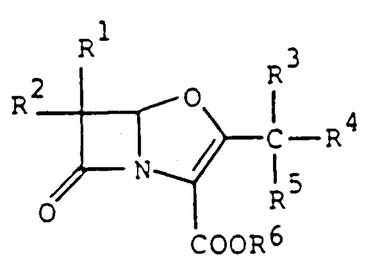

bzw.

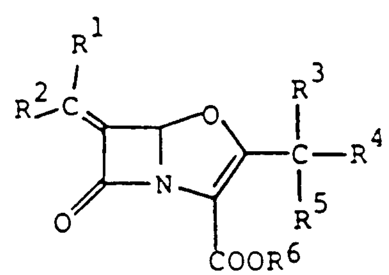

worin $R^1$ - $R^5$ die in Anspruch 1 angegebene Bedeutung haben und
$R^6$ Benzyl, p-Nitrobenzyl, Methyl, tert.-Butyl-dimethylsilyl, Trichlorethyl oder Pivaloyloxymethyl, Allyl, Methallyl, 2-Oxoethyl, 2-Oxopropyl, 2-Methylthio-ethyl oder Buten-1-yl bedeutet,

durch Abspaltung der Schutzgruppe $R^6$ zu einer Verbindung der Formel I oder ihren pharmazeutisch anwendbaren Salzen umsetzt.

2. Verfahren zur Herstellung von Verbindungen der Strukturformel (I) nach Anspruch 1, dadurch gekennzeichnet, daß man ein Keton der Formel

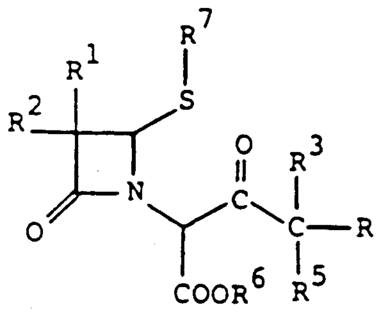

bzw.

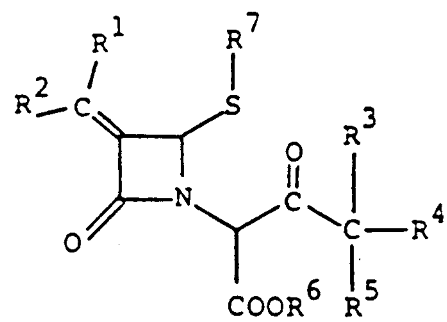

worin $R^7$ eine substituierte oder unsubstituierte verzweigte oder unverzweigte Alkylgruppe, Arylgruppe, Aralkylgruppe, Heteroaryl oder Heteroaralkylgruppe mit bis zu 10 Kohlenstoffatomen bedeutet, wobei die Substituenten ausgewählt werden aus der Gruppe: Niederalkyl, Acyloxy, Chlor, Brom, Nitro, Niederalkyloxy und Cyano und die Heteroatome des Heteroaryl- oder Heteroaralkylteils ausgewählt werden aus der Gruppe: Sauerstoff, Stickstoff und Schwefel und der Estermolekülteil $R^6$ die in Anspruch 1 angegebene Bedeutung hat, mit einem Quecksilber(II)salz zu einer Verbindung der Formel

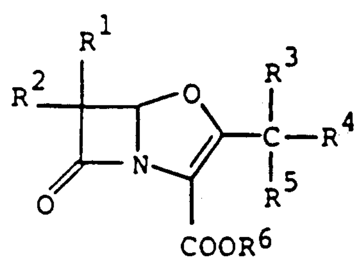

bzw.

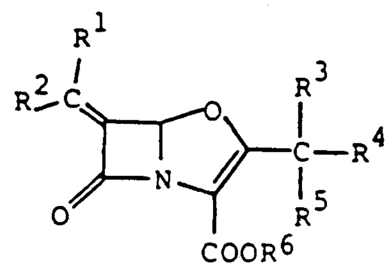

umsetzt.

3. Verfahren zur Herstellung von Verbindungen der Strukturformel (I) nach Anspruch 1, dadurch gekennzeichnet,
daß man eine Verbindung der Formel

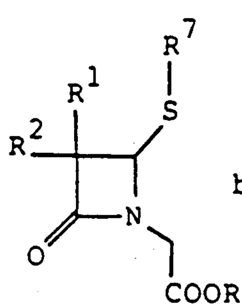

bzw.

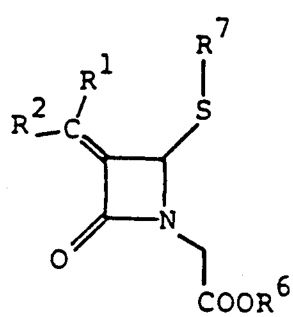

worin $R^1$ - $R^5$ die in Anspruch 1 angegebene Bedeutung haben und
$R^7$ eine substituierte oder unsubstituierte verzweigte oder unverzweigte Alkylgruppe, Arylgruppe, Aralkylgruppe, Heteroaryl- oder Heteroaralkylgruppe mit bis zu 10 Kohlenstoffatomen bedeutet, wobei die Substituenten ausgewählt werden aus der Gruppe: Niederalkyl, Acyloxy, Chlor, Brom, Nitro, Niederalkyloxy und Cyano und die Heteroatome des Heteroaryl- oder Heteroaralkylteils ausgewählt

werden aus der Gruppe: Sauerstoff, Stickstoff und Schwefel und der Estermolekülteil $R^6$ Benzyl, p-Nitrobenzyl, Methyl, tert.-Butyl-dimethylsilyl, Trichlorethyl oder Pivaloyloxymethyl, Allyl, Methallyl, 2-Oxoethyl, 2-Oxopropyl, 2-Methylthio-ethyl oder Buten-1-yl bedeutet, mit einer starken Base und einem Säurehalogenid der allgemeinen Formel

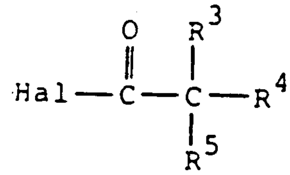 ,

in welcher Hal für Chlor, Brom oder Jod steht,
umsetzt und damit zu einem Keton der Formel

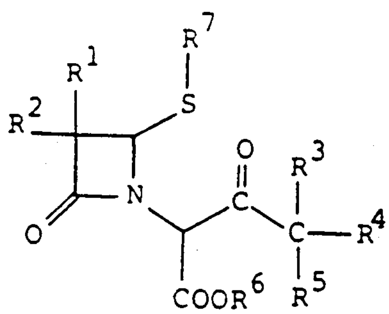 bzw. 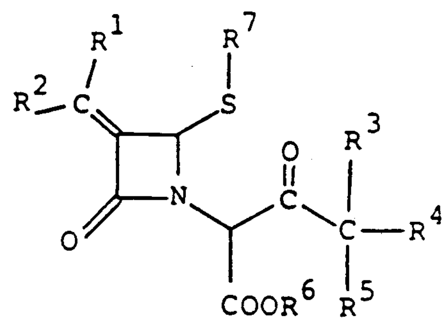

gelangt, dieses Keton anschließend mit einem Halogenierungsmittel zu einem Keton der Formel

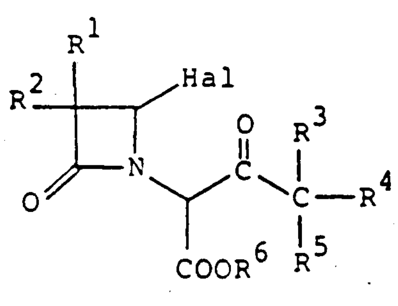 bzw. 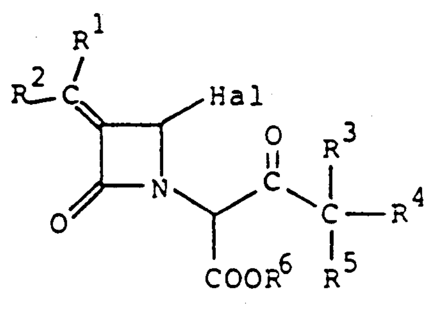

umsetzt,
worin
Hal Chlor, Brom oder Iod bedeutet und diese Verbindung anschließend mit einer Base oder einem Quecksilber(II)salz zu einer Verbindung der Formel

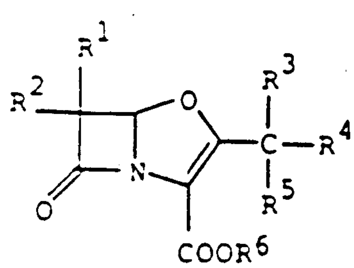 bzw. 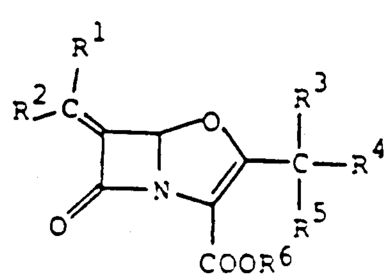

umsetzt, die Schutzgruppe $R^6$, welche die oben angegebene Bedeutung hat, abspaltet und eine Verbindung der Formel (I) oder ihre pharmazeutisch annehmbare Salze erhält.

**Claims**

**Claims for the following Contracting States : AT, BE, CH, DE, FR, GB, IT, LI, NL, SE**

1. Compounds of the structural formulae

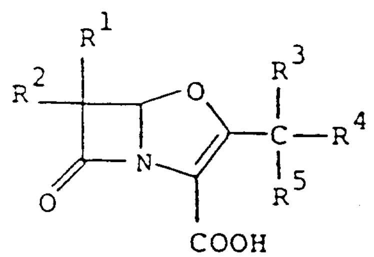

and

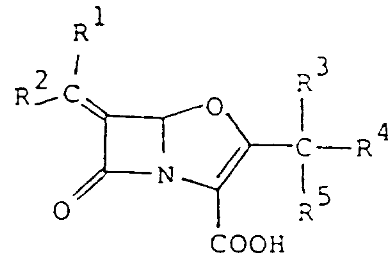

characterized in that $R^1$ and $R^2$, independently of one another, denote hydrogen or pharmaceutically acceptable groups which are bonded to the remaining part of the molecule via carbon-carbon single bonds and are selected from the series comprising substituted or unsubstituted alkyl, alkenyl, alkinyl, cycloalkyl, alkylcycloalkyl, alkylcycloalkenyl, cycloalkylalkyl, alkenylcycloalkyl, cycloalkenylalkyl, aryl, aralkyl, aralkenyl, aralkinyl, carboxyl or cyano, where the foregoing alkyl, alkenyl or alkinyl molecule parts contain 1 to 6 carbon atoms, and the cycloalkyl or cycloalkenyl molecule parts contain 3 to 6 carbon atoms and the aryl molecule parts contain 6 to 10 carbon atoms, heteroaralkyl, heteroaralkenyl, heteroaralkinyl, alkylheteroaryl, heterocyclyl, heterocyclylalkyl, heterocyclylalkenyl, heterocyclalkinyl or alkylheterocyclyl, where the foregoing alkyl, alkenyl or alkinyl molecule parts contain 1 to 6 carbon atoms and the heteroaromatic or heterocyclic moiety is monocyclic or bicyclic and contains 3 to 10 ring atoms, of which one or more are selected from the series comprising: oxygen, sulphur and nitrogen, and where the substituents of the abovementioned groups may be: hydroxyl, hydroxyalkoxy, aminoalkoxy, amidinoalkoxy, alkoxy, acyloxy, aryloxy, heterocyclyloxy, carbamoyl, carbamoyloxy, thiocarbamoyl, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylheteroarylthio, hydroxyalkylheteroarylthio, heterocyclylthio, carbamoylthio, alkylcarbamoylthio, thiocarbamoylthio or alkylthiocarbamoylthio, amino or monoalkylamino, dialkylamino, oxo, oximino or alkylimino, tetraalkylammonium, cycloalkylamino, arylamino, heterocyclylamino, acylamino, amidino, alkylamidino, guanidino, alkylguanidino, carbamoylamino, alkylcarbamoylamino, thiocarbamoylamino, alkylthiocarbamoylamino, nitro, chlorine, bromine, fluorine, iodine, azido, cyano, alkylsulphinyl, alkylsulphonyl, sulphonamido, sulphamoyloxy, alkylsulphonyloxy or sulpho, sulphoxy or carboxyl, where the substitutents, independently of one another, occur once or several times and their alkyl moiety contains 1 to 6 carbon atoms and their aryl moiety contains 6 to 10 carbon atoms, and where the heterocyclic moiety is monocyclic or bicyclic and contains 3 to 10 ring atoms, of which one or more are selected from the series comprising: oxygen, sulphur and nitrogen, and characterised in that $R^3$, $R^4$ and $R^5$, independently of one another, are selected from the abovementioned, pharmaceutically acceptable groups which are bonded to the remaining part of the molecule via carbon-carbon single bonds.

2. Compounds according to Claim 1, characterised in that $R^3$, $R^4$ and $R^5$ have the abovementioned meanings and $R^1$ and $R^2$, independently of one another, denote hydrogen, alkyl, hydroxyalkyl or dihydroxyalkyl, each having 1 to 6 carbon atoms.

3. Compounds according to Claim 2, characterised in that $R^1$ and $R^2$ have the meanings given therein, $R^3$ and $R^4$ denote methyl, and $R^5$ is selected from the series comprising:

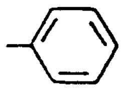
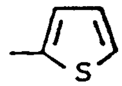
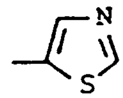

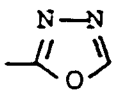
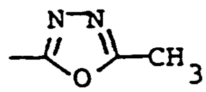
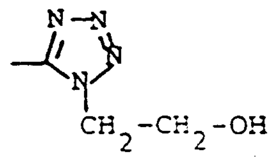

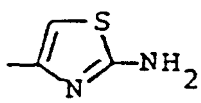
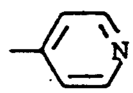
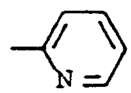

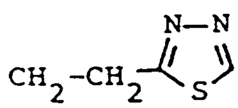
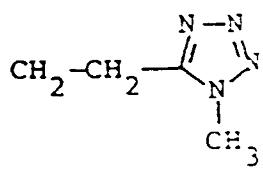

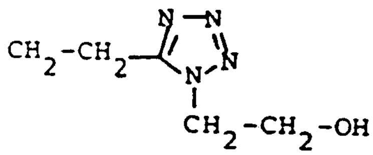

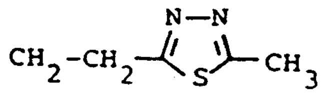

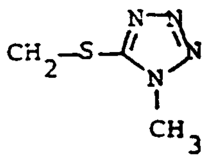

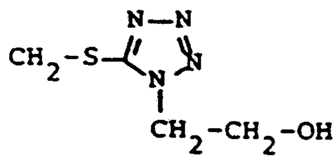

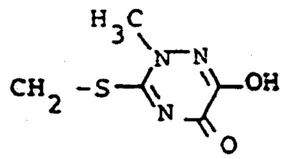

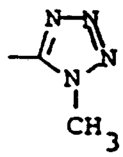

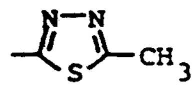

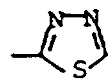

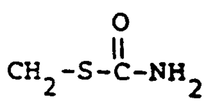

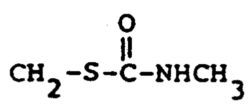

$CH_2-S-CH_3$

$CH_2-S-CH_2-CH_2-NH_2$

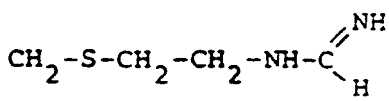

$CH_2-NH_2$

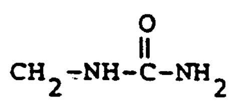

$CH_2-NH-CHO$

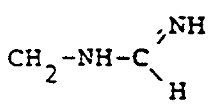

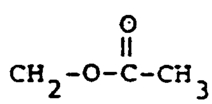

$CH_2-O-CHO$

$CH_2-Cl$

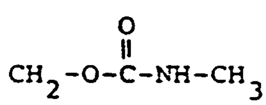

$CH_2-N_3$

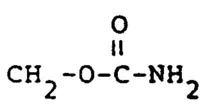

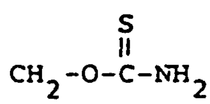

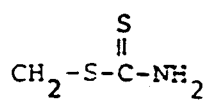

$CH_2-O-\overset{S}{\overset{\|}{C}}-NH-CH_3$ $CH_2-\overset{S}{\overset{\|}{C}}-NH-CH_3$ $CH_2-CH_2-NH_2$

$CH_2-CH_2-NH-C\begin{smallmatrix}{/\!/NH}\\{\backslash H}\end{smallmatrix}$ $CH_2-NH-C\begin{smallmatrix}{/\!/NH}\\{\backslash NH_2}\end{smallmatrix}$ $CH_2-CH_2-CH_2-NH_2$

$CH_2-CH_2-CH_2-NH-C\begin{smallmatrix}{/\!/NH}\\{\backslash H}\end{smallmatrix}$ $CH_2-CH_2-COOH$ $CH_2-COOH$

$COOH$ $CH_2-CH_2-CH_2-COOH$ $CH_2-OH$

$CH_2-CH_2-CH_2-OH$ $CH_2-CH_2-O-\overset{O}{\overset{\|}{C}}-NH_2$ $C\equiv N$

$CH_2-CH_2-O-\overset{O}{\overset{\|}{C}}-NHCH_3$ $CH_2-CH_2-CH_2-O-\overset{O}{\overset{\|}{C}}-NH_2$

$CH_2-CH_2-CH_2-O-\overset{O}{\overset{\|}{C}}-NH-CH_3$ $CH_2-CH_2-CH_2-NH-CHO$

**4.** Process for the preparation of a compound according to Claim 1, characterised in that a compound of the formula

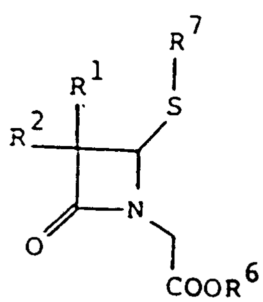

or

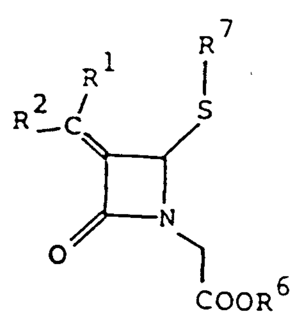

in which $R^7$ denotes a substituted or unsubstituted, branched or unbranched alkyl group, aryl group, aralkyl group, heteroaryl or heteroaralkyl group having up to 10 carbon atoms, where the substituents are selected from the series comprising: lower alkyl, acyloxy, chlorine, bromine, nitro, lower alkoxy and cyano, and the heteroatoms of the heteroaryl or heteroaralkyl part are selected from the series comprising: oxygen, nitrogen and sulphur, and the ester moiety $R^6$ denotes benzyl, p-nitrobenzyl, methyl, tert.-butyl-dimethylsilyl, trichloroethyl or pivaloyloxymethyl, allyl, methallyl, 2-oxoethyl, 2-oxopropyl, 2-methylthio-ethyl or buten-1-yl, is reacted with a strong base and an acyl halide of the general formula

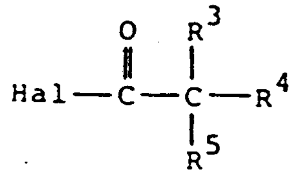

to give a ketone of the formula:

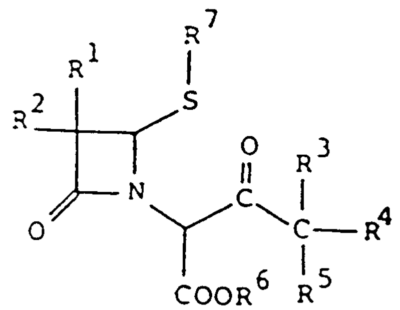

or

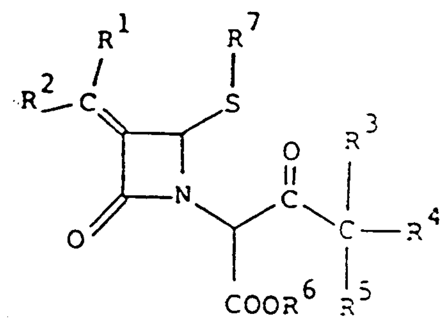

**5.** Process for the preparation of a compound according to Claim 1, characterised in that a ketone of the formula

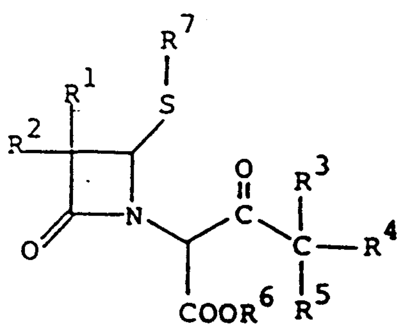

or

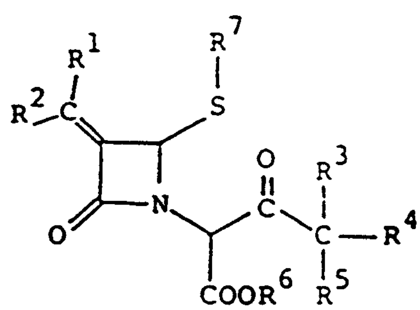

in which $R^7$ denotes a substituted or unsubstituted, branched or unbranched alkyl group, aryl group, aralkyl group, heteroaryl or heteroaralkyl group having up to 10 carbon atoms, where the substituents are selected from the series comprising: lower alkyl, acyloxy, chlorine, bromine, nitro, lower alkyloxy and cyano, and the heteroatoms of the heteroaryl or heteroaralkyl part are selected from the series comprising: oxygen, nitrogen and sulphur, and the ester moiety $R^6$ has the meaning given in claim 4, is reacted with a halogenating agent to give a ketone of the formula

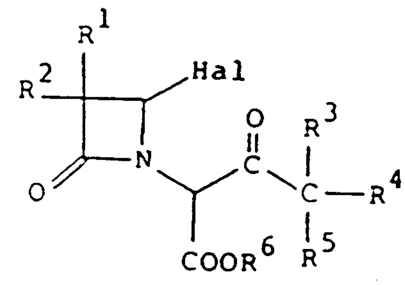 or 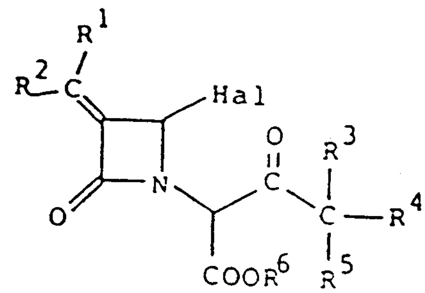

in which Hal denotes chlorine, bromine or iodine.

6. Process for the preparation of a compound according to Claim 1, characterised in that a ketone of the formula

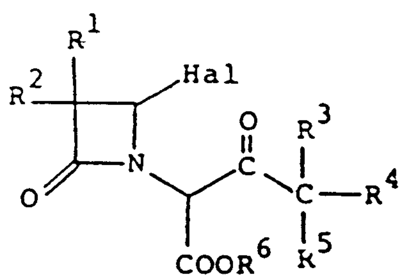 or 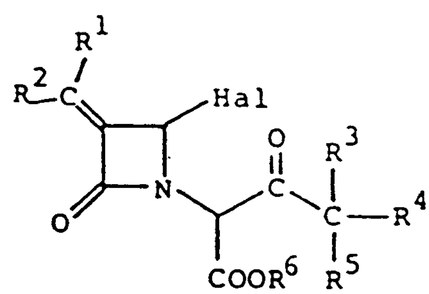

in which the ester molecule part $R^6$ has the meaning given in Claim 4, is reacted with a base to give a compound of the formula

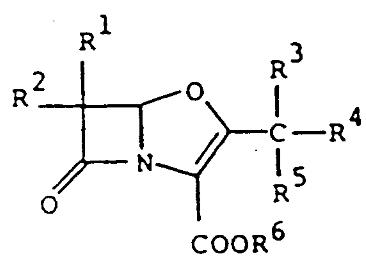 or 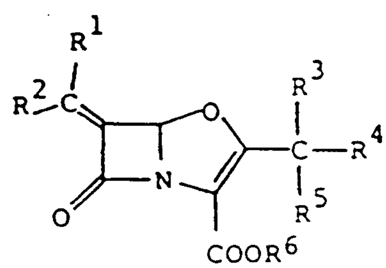

7. Process for the preparation of a compound according to Claim 1, characterised in that a ketone of the formula

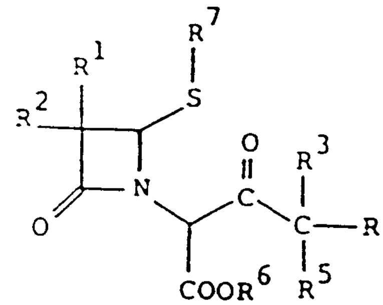 or 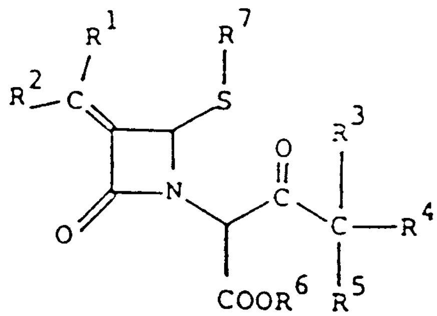

in which $R^7$ denotes a substituted or unsubstituted, branched or unbranched alkyl group, aryl group, aralkyl group, heteroaryl or heteroaralkyl group having up to 10 carbon atoms, where the substituents are selected from the series comprising: lower alkyl, acyloxy, chlorine, bromine, nitro; lower alkoxy and cyano, and the heteroatoms of the heteroaryl or heteroaralkyl part are selected from the series comprising: oxygen, nitrogen and sulphur, and the ester moiety $R^6$ has the meaning given in Claim 4, is reacted with a mercury(II) salt to give a compound of the formula

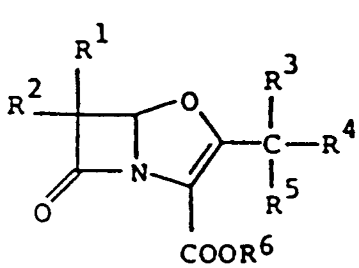 or 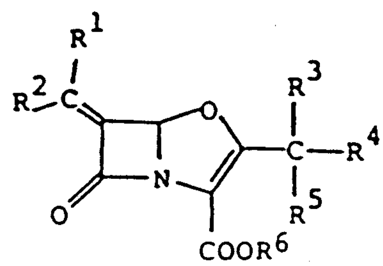

**8.** Process for the preparation of a compound according to Claim 1, characterised in that a compound of the formula

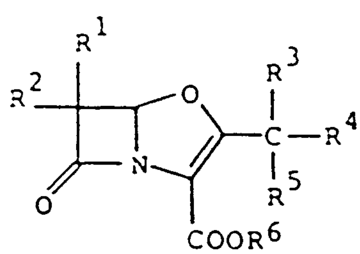 or 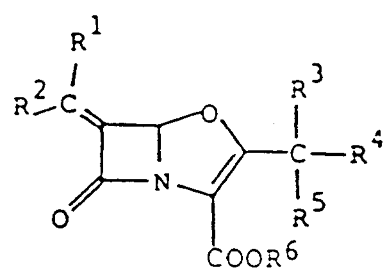

in which the ester moiety $R^6$ has the meaning given in Claim 4, is converted into a compound of the formula

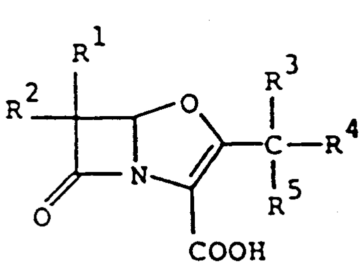

I

or

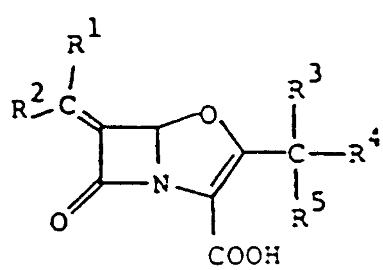

II

or its pharmaceutically acceptable salts by removing the protecting group $R^6$.

**9.** Pharmaceutical preparation, characterised in that it contains a compound according to at least one of Claims 1 to 3 and a pharmaceutical excipient for this compound.

**10.** Pharmaceutical preparation, characterised in that it is in dose unit form and contains a therapeutically effective amount of a compound according to at least one of Claims 1 to 3 and a pharmaceutical excipient for this compound.

**11.** Compound of the structural formula

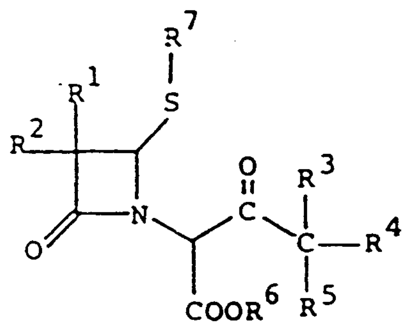

or

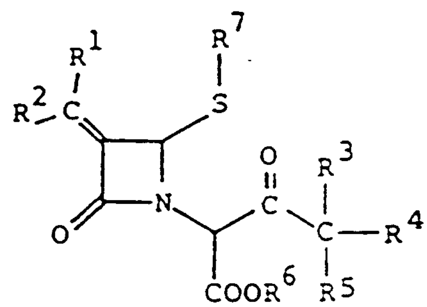

in which $R^1$ and $R^5$ have the meanings given in Claim 1 and in which $R^7$ denotes a substituted or unsubstituted, branched or unbranched alkyl group, aryl group, aralkyl group, heteroaryl or heteroaralkyl group having up to 10 carbon atoms, where the substituents are selected from the series comprising: lower alkyl, acyloxy, chlorine, bromine, nitro, lower alkoxy and cyano, and the heteroatoms of the heteroaryl or heteroralkyl part are selected from the series comprising: oxygen, nitrogen and sulphur, and the ester group $R^6$ has the meaning given in Claim 4.

**12.** Compound of the structural formula

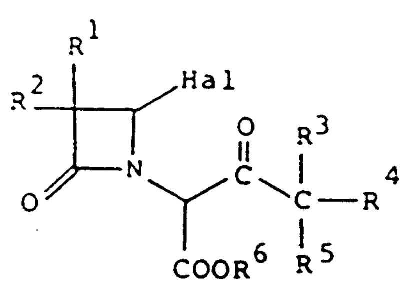

or

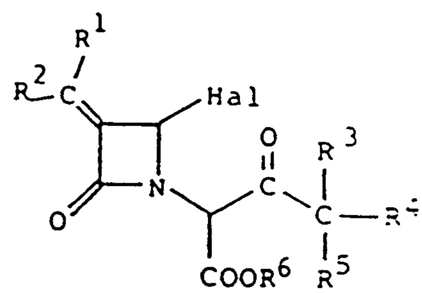

in which $R^1$ to $R^5$ have the meanings given in Claim 1 and in which the ester moiety has the meaning given in Claim 4.

**13.** Compound of the structural formula

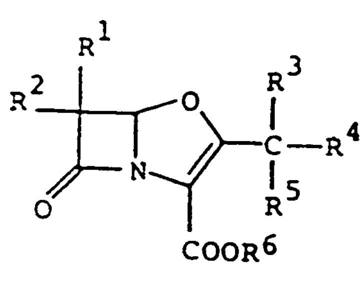

or

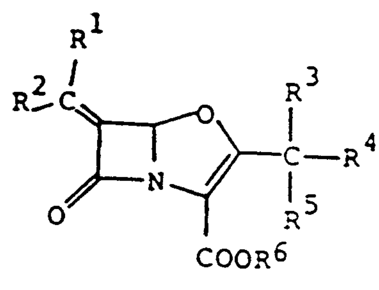

in which $R^1$ to $R^5$ have the meanings given in Claim 1 and the ester moiety $R^6$ has the meaning given in Claim 4.

**Claims for the following Contracting States : ES, GR**

1. Process for the preparation of compounds of the structural formulae (I)

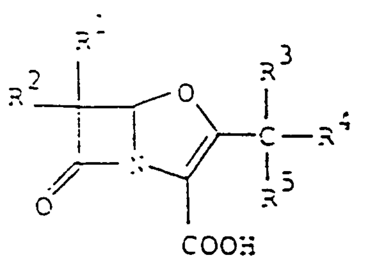

and

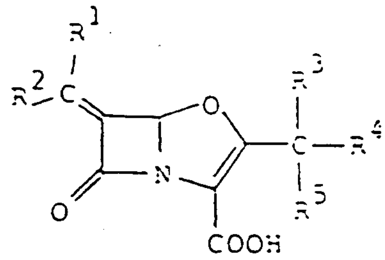

in which $R^1$ and $R^2$, independently of one another, denote hydrogen or pharmaceutically acceptable groups which are bonded to the remaining part of the molecule via carbon-carbon single bonds and are selected from the series comprising substituted or unsubstituted alkyl, alkenyl, alkinyl, cycloalkyl, alkylcycloalkyl, alkylcycloalkenyl, cycloalkylalkyl, alkenylcycloalkyl, cycloalkenylalkyl, aryl, aralkyl, aralkenyl, aralkinyl, carboxyl or cyano, where the foregoing alkyl, alkenyl or alkinyl molecule parts contain 1 to 6 carbon atoms, and the cycloalkyl or cycloalkenyl moieties contain 3 to 6 carbon atoms and the aryl moieties contain 6 to 10 carbon atoms, heteroaralkyl, heteroaralkenyl, heteroaralkinyl, alkylheteroaryl, heterocyclyl, heterocyclylalkyl, heterocyclylalkenyl, heterocyclalkinyl or alkylheterocyclyl, where the foregoing alkyl, alkenyl or alkinyl moieties contain 1 to 6 carbon atoms and the heteroaromatic or heterocyclic molecule part is monocyclic or bicyclic and contains 3 to 10 ring atoms, of which one or more are selected from the series comprising: oxygen, sulphur and nitrogen, and where the substituents of the abovementioned groups may be: hydroxyl, hydroxyalkoxy, aminoalkoxy, amidinoalkoxy, alkoxy, acyloxy, aryloxy, heterocyclyloxy, carbamoyl, carbamoyloxy, thiocarbamoyl, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylheteroarylthio, hydroxyalkylheteroarylthio, heterocyclylthio, carbamoylthio, alkylcarbamoylthio, thiocarbamoylthio or alkylthiocarbamoylthio, amino or monoalkylamino, dialkylamino, oxo, oximino or alkylimino, tetraalkylammonium, cycloalkylamino, arylamino, heterocyclylamino, acylamino, amidino, alkylamidino, guanidino, alkylguanidino, carbamoylamino, alkylcarbamoylamino, thiocarbamoylamino, alkylthiocarbamoylamino, nitro, chlorine, bromine, fluorine, iodine, azido, cyano, alkylsulphinyl, alkylsulphonyl, sulphonamido, sulphamoyloxy, alkylsulphonyloxy or sulpho, sulphoxy or carboxyl, where the substituents, independently of one another, occur once or several times and their alkyl moiety contains 1 to 6 carbon atoms and their aryl moiety contains 6 to 10 carbon atoms, and where the heterocyclic moiety is monocyclic or bicyclic and contains 3 to 10 ring atoms, of which one or more are selected from the series comprising: oxygen, sulphur and nitrogen, and in which $R^3$, $R^4$ and $R^5$, independently of one another, are selected from the abovementioned, pharmaceutically acceptable groups which are bonded to the remaining part of the molecule via carbon-carbon single bonds, characterised in that a compound of the formula

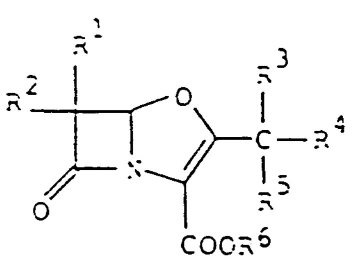

or

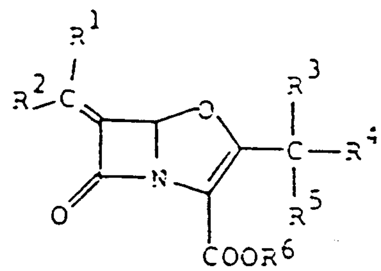

in which $R^1$-$R^5$ have the meaning given in Claim 1 and $R^6$ denotes benzyl, p-nitrobenzyl, methyl, tert.-

butyl-dimethylsilyl, trichloroethyl or pivaloyloxymethyl, allyl, methallyl, 2-oxoethyl, 2-oxopropyl, 2-methylthio-ethyl or buten-1-yl, is converted into a compound of the formula I or its pharmaceutically usable salts by removing the protecting group $R^6$.

**2.** Process for the preparation of compounds of the structural formula (I) according to Claim 1, characterised in that a ketone of the formula

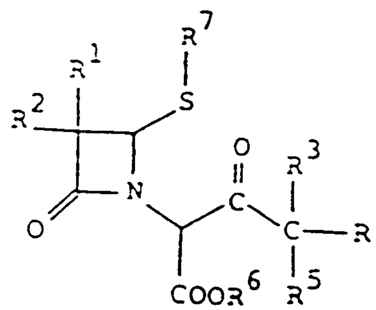

or

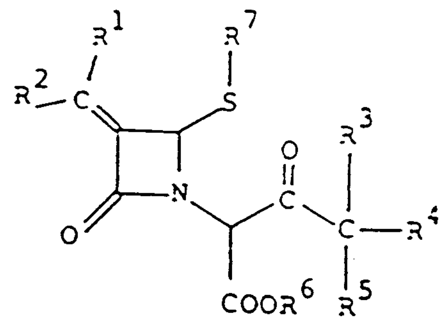

in which $R^7$ denotes a substituted or unsubstituted, branched or unbranched alkyl group, aryl group, aralkyl group, heteroaryl or heteroaralkyl group having up to 10 carbon atoms, where the substituents are selected from the series comprising: lower alkyl, acyloxy, chlorine, bromine, nitro, lower alkyloxy and cyano, and the heteroatoms of the heteroaryl or heteroaralkyl part are selected from the series comprising: oxygen, nitrogen and sulphur, and the ester moiety $R^6$ has the meaning given in Claim 1, is reacted with a mercury(II) salt to give a compound of the formula

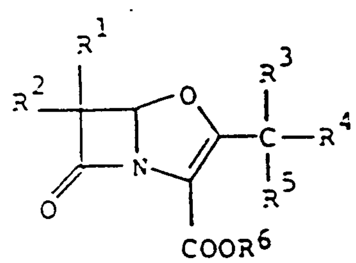

or

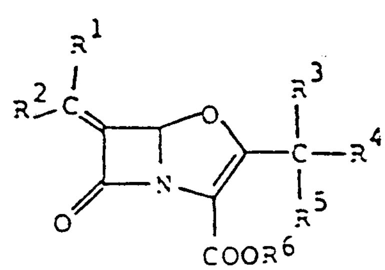

**3.** Process for the preparation of compounds of the structural formula (I) according to Claim 1, characterised in that a compound of the formula

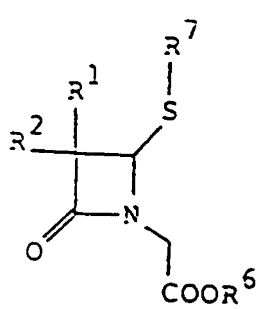

or

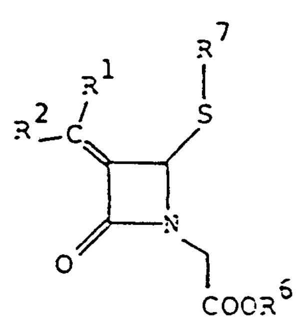

in which $R^1$-$R^5$ have the meaning given in Claim 1 and $R^7$ denotes a substituted or unsubstituted, branched or unbranched alkyl group, aryl group, aralkyl group, heteroaryl or heteroaralkyl group having up to 10 carbon atoms, where the substituents are selected from the series comprising: lower alkyl, acyloxy, chlorine, bromine, nitro, lower alkoxy and cyano, and the heteroatoms of the heteroaryl or

heteroaralkyl part are selected from the series comprising: oxygen, nitrogen and sulphur, and the ester moiety $R^6$ denotes benzyl, p-nitrobenzyl, methyl, tert.-butyl-dimethylsilyl, trichloroethyl or pivaloyloxymethyl, allyl, methallyl, 2-oxoethyl, 2-oxopropyl, 2-methylthio-ethyl or buten-1-yl, is reacted with a strong base and an acyl halide of the general formula

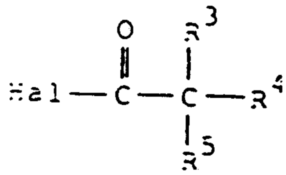

, or

in which Hal represents chlorine, bromine or iodine, and hence to give a ketone of the formula

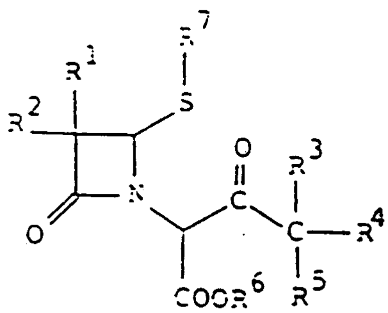

or

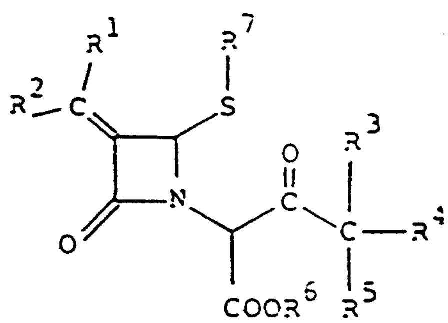

this ketone is subsequently reacted with a halogenating agent to give a ketone of the formula

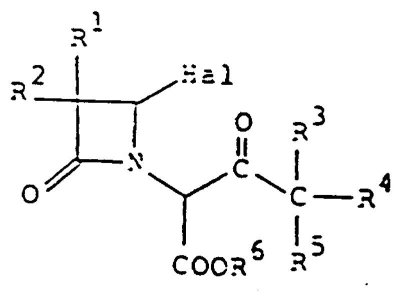

or

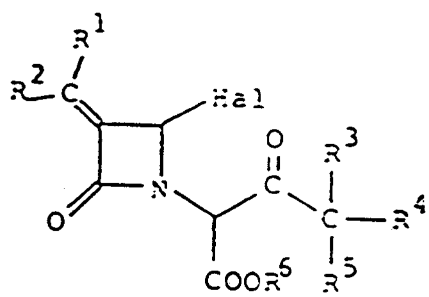

in which Hal denotes chlorine, bromine or iodine and this compound is subsequently reacted with a base or a mercury (II) salt to give a compound of the formula

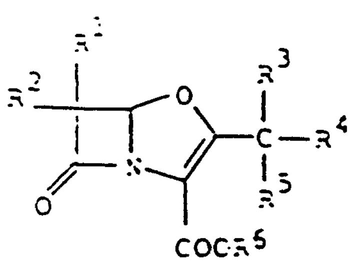

or

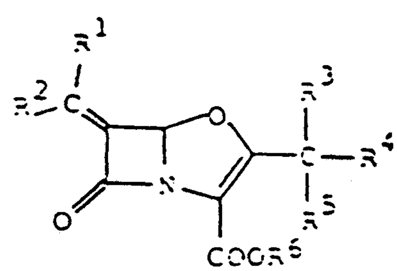

the protecting group $R^6$, which has the meaning given above, is removed and a compound of the formula (I) or its pharmaceutically acceptable salts is obtained.

## Revendications

## Revendications pour les Etats contractants suivants : AT, BE, CH, DE, FR, GB, IT, LI, NL, SE

**1.** Composés de formules structurales

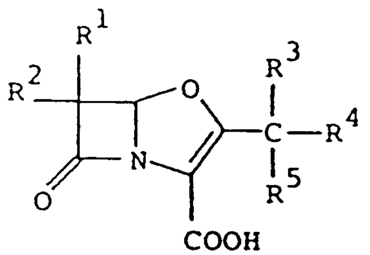

et

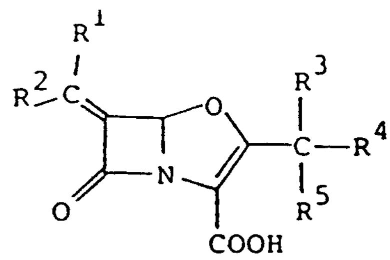

caractérisés en ce que $R^1$ et $R^2$ désignent indépendamment l'un de l'autre l'hydrogène ou des groupes acceptables du point de vue pharmaceutique attachés par des liaisons simples carbone-à-carbone à la partie restante de la molécule, qui sont choisis parmi des groupes, substitués ou non substitués, alkyle, alcényle, alcynyle, cycloalkyle, alkylcycloalkyle, alkylcycloalcényle, cycloalkylalkyle, alcénylcycloalkyle, cycloalcénylalkyle, aryle, aralkyle, aralcényle, aralcynyle, carboxy ou cyano, les parties alkyliques, alcényliques ou alcynyliques de molécules ci-dessus contenant 1 à 6 atomes de carbone, les parties cycloalkyliques ou les parties cycloalcényliques de molécules ayant 3 à 6 atomes de carbone et les parties aryliques de molécules contenant 6 à 10 atomes de carbone, des groupes hétéroaralkyle, hétéroaralcényle, hétéroaralcynyle, alkylhétéroaryle, hétérocyclyle, hétérocyclylalkyle, hétérocyclylalcényle, hétérocyclylalcynyle, alkylhétérocyclyle, les parties alkyliques, alcényliques ou alcynyliques de molécules ci-dessus contenant 1 à 6 atomes de carbone et la partie hétéro-aromatique ou hétérocyclique de molécule étant monocyclique ou bicyclique et contenant 3 à 10 atomes de carbone, dont l'un ou plusieurs sont choisis dans le groupe des atomes d'oxygène, de soufre et d'azote, et les substituants des groupes énumérés ci-dessus pouvant être des substituants :

hydroxy, hydroxyalkyloxy, aminoalkyloxy, amidinoalkyloxy, alkyloxy, acyloxy, aryloxy, hétérocyclyloxy, carbamoyle, carbamoyloxy, thiocarbamoyle, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylhétéroarylthio, hydroxyalkylhétéroarylthio, hétérocyclylthio, carbamoylthio, alkylcarbamoylthio, thiocarbamoylthio, alkylthiocarbamoylthio, amino ou monoalkylamino, dialkylamino, oxo, oximino ou alkylimino, tétra-alkylammonium, cycloalkylamino, arylamino, hétérocyclylamino, acylamino, amidino, alkylamidino, guanidino, alkylguanidino, carbamoylamino, alkylcarbamoylamino, thiocarbamoylamino, alkylthiocarbamoylamino, nitro, chloro, bromo, fluoro, iodo, azido, cyano, alkylsulfinyle, alkylsulfonyle, sulfonamido, sulfamoyloxy, alkylsulfonyloxy ou sulfo, sulfoxy ou carboxy, les substituants apparaissant une ou plusieurs fois indépendamment les uns des autres et leur partie alkylique de molécule contenant 1 à 6 atomes de carbone, leur partie arylique de molécule contenant 6 à 10 atomes de carbone et la partie hétérocyclique de molécule étant monocyclique ou bicyclique et contenant 3 à 10 atomes de carbone dont l'un ou plusieurs sont choisis dans le groupe des atomes d'oxygène, de soufre et d'azote, et caractérisés en ce que $R^3$, $R^4$ et $R^5$ sont choisis indépendamment les uns des autres parmi les groupes acceptables du point de vue pharmaceutique mentionnés ci-dessus, attachés par des liaisons simples carbone-à-carbone à la partie restante de la molécule.

**2.** Composés suivant la revendication 1, caractérisés en ce que $R^3$, $R^4$ et $R^5$ ont les définitions indiquées ci-dessus et $R^1$ et $R^2$ représentent, indépendamment l'un de l'autre, de l'hydrogène, un groupe alkyle, hydroxyalkyle ou dihydroxyalkyle ayant chacun 1 à 6 atomes de carbone.

**3.** Composés suivant la revendication 2, caractérisés en ce que $R^1$ et $R^2$ ont les définitions qui y sont indiquées, $R^3$ et $R^4$ désignent des groupes méthyle et $R^5$ est choisi parmi les groupes :

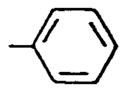
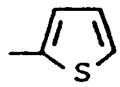
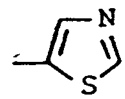

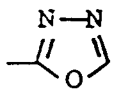
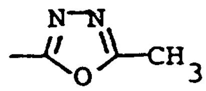
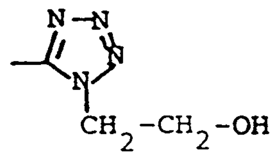

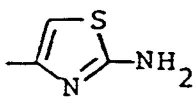
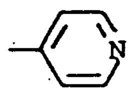
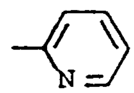

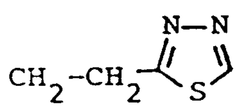
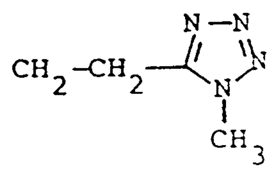

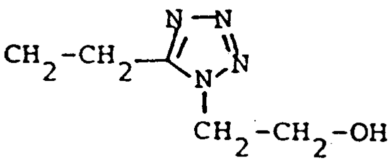

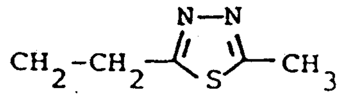

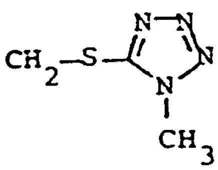

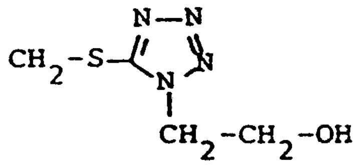

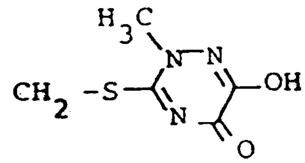

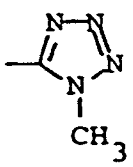

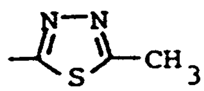

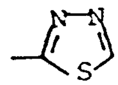

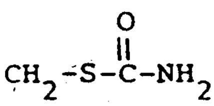

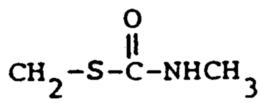

$CH_2-S-CH_3$

$CH_2-S-CH_2-CH_2-NH_2$

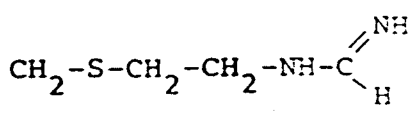

$CH_2-NH_2$

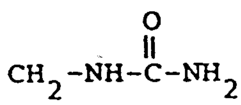

$CH_2-NH-CHO$

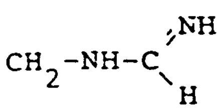

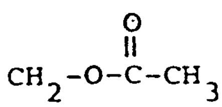

$CH_2-O-CHO$

$CH_2-Cl$

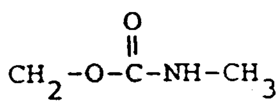

$CH_2-N_3$

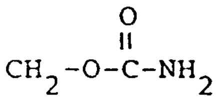 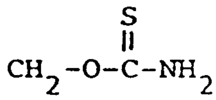 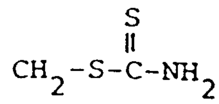

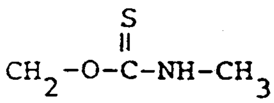 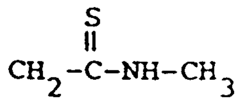 $CH_2-CH_2-NH_2$

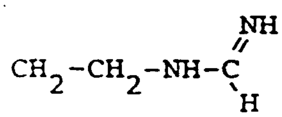 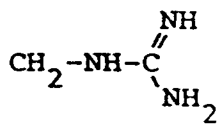 $CH_2-CH_2-CH_2-NH_2$

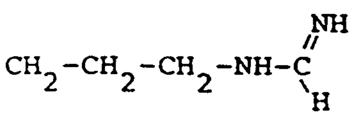 $CH_2-CH_2-COOH$ $CH_2-COOH$

COOH $CH_2-CH_2-CH_2-COOH$ $CH_2-OH$

$CH_2-CH_2-CH_2-OH$ 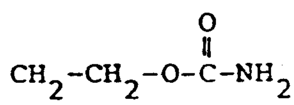 $C\equiv N$

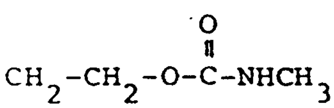 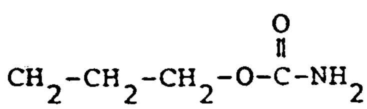

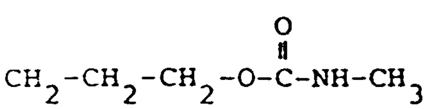 $CH_2-CH_2-CH_2-NH-CHO$

**4.** Procédé de production d'un composé suivant la revendication 1, caractérisé en ce qu'on fait réagir un composé de formule

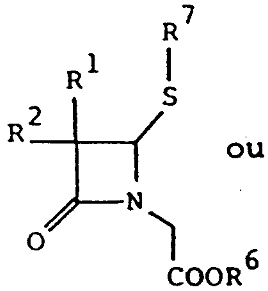 ou 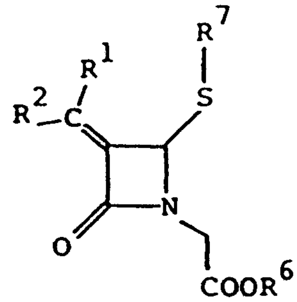

dans laquelle $R^7$ est un groupe, substitué ou non substitué, alkyle ramifié ou non ramifié, aryle, aralkyle, hétéroaryle ou hétéroaralkyle ayant jusqu'à 10 atomes de carbone, les substituants étant choisis dans le groupe des substituants alkyle inférieur, acyloxy, chloro, bromo, nitro, alkyloxy inférieur et cyano et les hétéro-atomes de la partie hétéroaryle ou hétéroaralkyle étant choisis dans le groupe des atomes d'oxygène, d'azote et de soufre et la partie $R^6$ de la molécule d'ester désignant un groupe benzyle, p-nitrobenzyle, méthyle, tertiobutyldiméthylsilyle, trichloréthyle ou pivaloyloxyméthyle, allyle, méthallyle, 2-oxo-éthyle, 2-oxopropyle, 2-méthylthioéthyle ou butène-1-yle, avec une base forte et un halogénure d'acide de formule générale

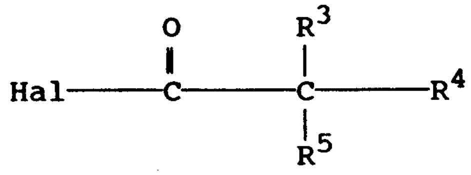

et on arrive ainsi à une cétone de formule

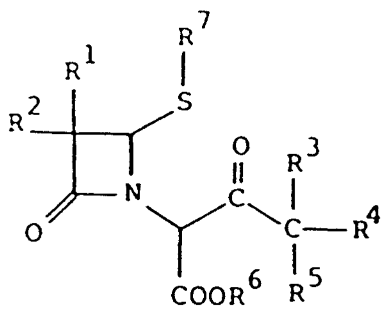

ou

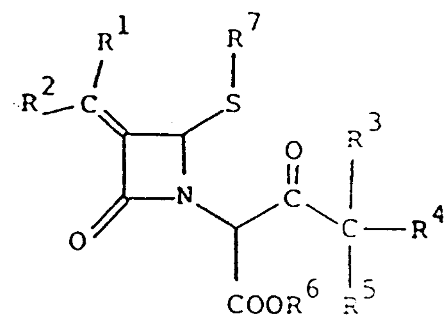

5. Procédé de production d'un composé suivant la revendication 1, caractérisé en ce qu'une cétone de formule

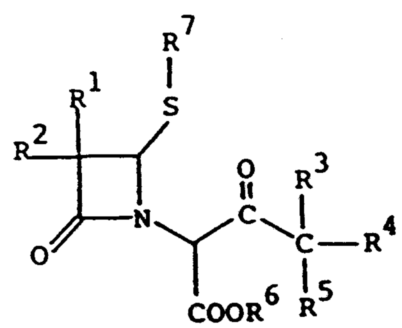

ou

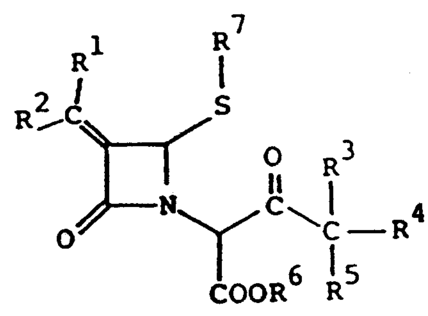

dans laquelle $R^7$ est un groupe, substitué ou non substitué, alkyle ramifié ou non ramifié, aryle, aralkyle, hétéroaryle ou hétéroaralkyle ayant jusqu'à 10 atomes de carbone, les substituants étant choisis dans le groupe des substituants alkyle inférieur, acyloxy, chloro, bromo, nitro, alkyloxy inférieur et cyano et les hétéro-atomes de la partie hétéroaryle ou hétéroaralkyle étant choisis dans le groupe des atomes d'oxygène, d'azote et de soufre et la partie $R^6$ de la molécule d'ester ayant la définition indiquée dans la revendication 4, est amenée à réagir avec un agent d'halogénation pour former une cétone de formule

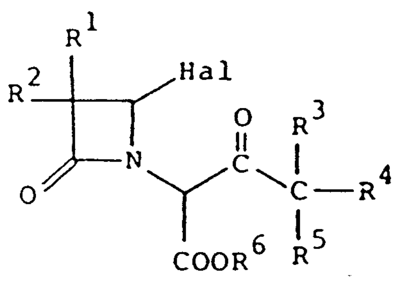

ou

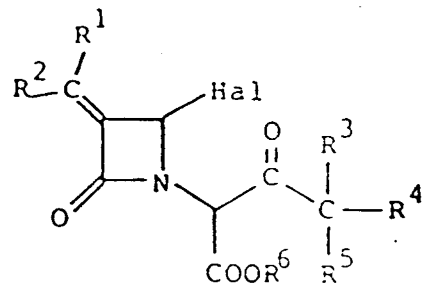

dans laquelle Hal est le chlore, le brome ou l'iode.

6. Procédé de production d'un composé suivant la revendication 1, caractérisé en ce qu'on fait réagir une cétone de formule

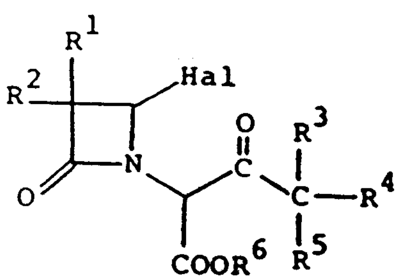

ou

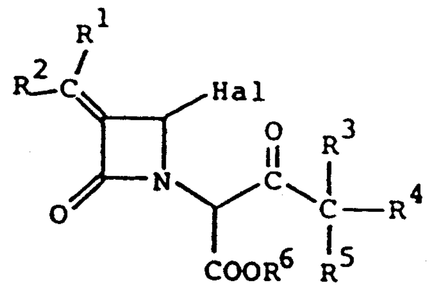

dans laquelle la partie $R^6$ de la molécule d'ester a la définition indiquée dans la revendication 4, avec une base pour former un composé de formule

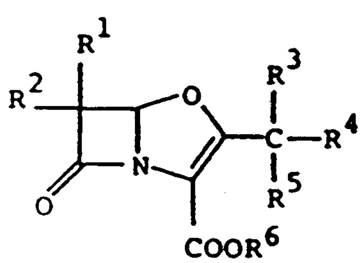

ou

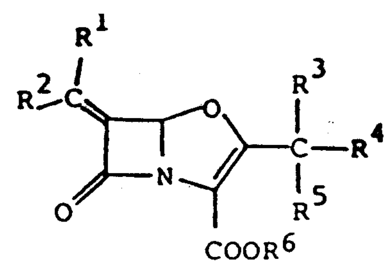

7. Procédé de production d'un composé suivant la revendication 1, caractérisé en ce qu'on fait réagir une cétone de formule

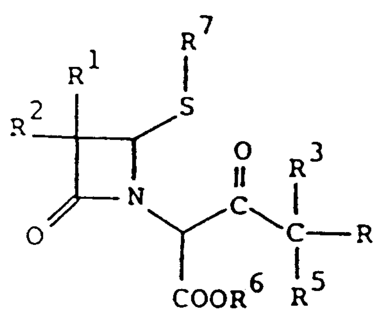

ou

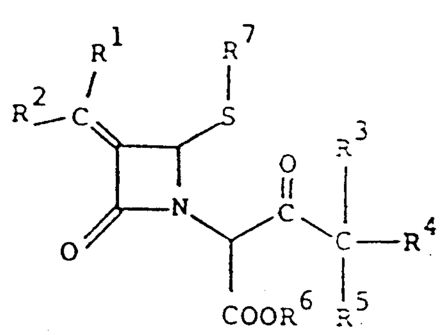

dans laquelle $R^7$ est un groupe, substitué ou non substitué, alkyle ramifié ou non ramifié, aryle, aralkyle, hétéroaryle ou hétéroarylalkyle ayant jusqu'à 10 atomes de carbone, les substituants étant choisis dans le groupe des substituants alkyle inférieur, acyloxy, chloro, bromo, nitro, alkyloxy inférieur

et cyano et les hétéro-atomes de la partie hétéroaryle ou hétéroaralkyle étant choisis dans le groupe des atomes d'oxygène, d'azote et de soufre et la partie $R^6$ de la molécule d'ester ayant la définition indiquée dans la revendication 4,
avec un sel de mercure (II) pour former un composé de formule

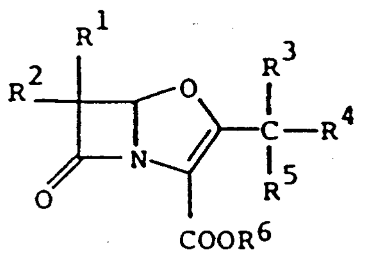

ou

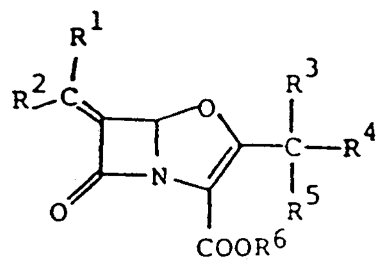

**8.** Procédé de production d'un composé suivant la revendication 1, caractérisé en ce qu'on fait réagir un composé de formule

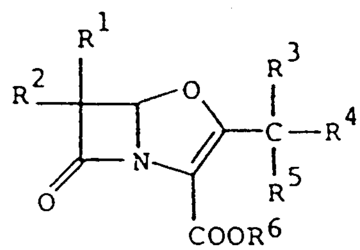

ou

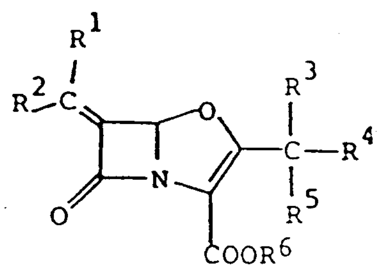

dans laquelle la partie $R^6$ de la molécule d'ester a la définition indiquée dans la revendication 4, par élimination du groupe protecteur $R^6$ pour former un composé de formule

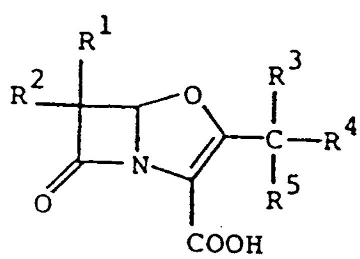

I

ou

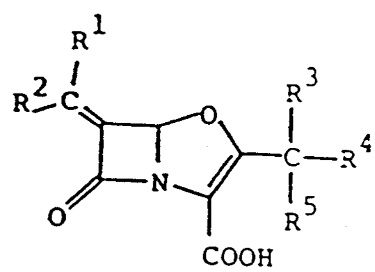

II

ou ses sels acceptables du point de vue pharmaceutique.

**9.** Préparation pharmaceutique, caractérisée en ce qu'elle contient un composé suivant l'une au moins des revendications 1 à 3 et un support pharmaceutique approprié.

**10.** Préparation pharmaceutique, caractérisée en ce qu'elle se présente sous une forme unitaire dosée et contient une quantité efficace du point de vue thérapeutique d'un composé suivant l'une au moins des revendications 1 à 3 et un support pharmaceutique approprié.

**11.** Composé de formule structurale

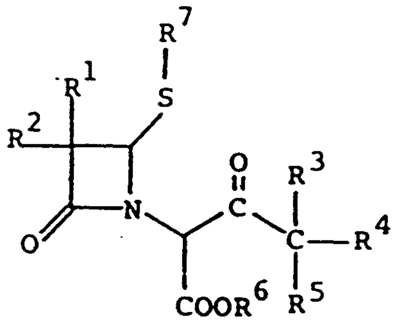

ou

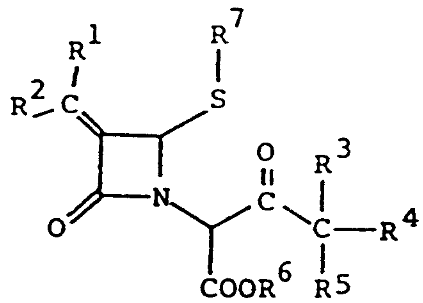

dans laquelle $R^1$ à $R^5$ ont les définitions indiquées dans la revendication 1 et dans laquelle $R^7$ est un groupe, substitué ou non substitué, alkyle ramifié ou non ramifié, aryle, aralkyle, hétéroaryle ou hétéroaralkyle ayant jusqu'à 10 atomes de carbone, les substituants étant choisis dans le groupe des substituants alkyle inférieur, acyloxy, chloro, bromo, nitro, alkyloxy inférieur et cyano et les hétéroatomes de la partie hétéroaryle ou hétéroaralkyle étant choisis dans le groupe des atomes d'oxygène, d'azote et de soufre et le groupe ester $R^6$ ayant la définition indiquée dans la revendication 4.

**12.** Composé de formule structurale

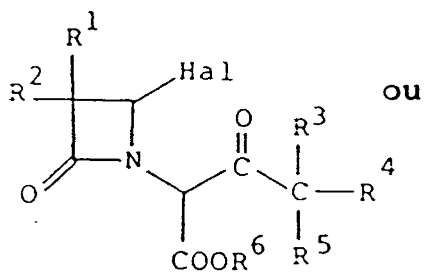

ou

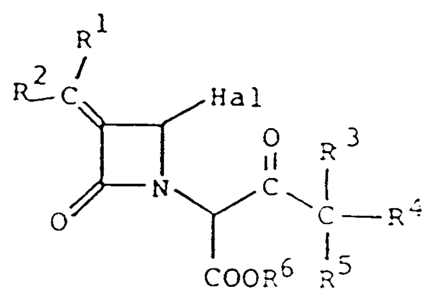

dans laquelle $R^1$ à $R^5$ ont les définitions indiquées dans la revendication 1 et la partie ester de la molécule a la définition indiquée dans la revendication 4.

**13.** Composé de formule structurale

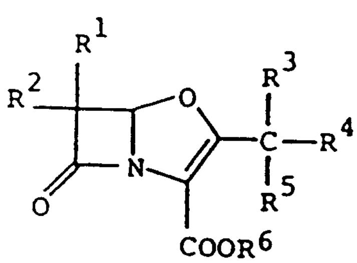

ou

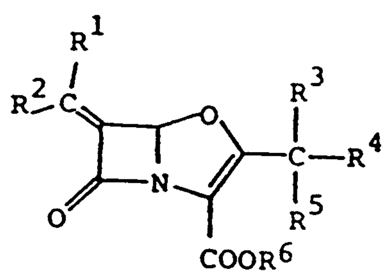

dans laquelle $R^1$ à $R^5$ ont les définitions indiquées dans la revendication 1 et la partie $R^6$ de la molécule d'ester a la définition indiquée dans la revendication 4.

**Revendications pour les Etats contractants suivants : ES, GR**

1. Procédé de production de composés de formules structurales (I)

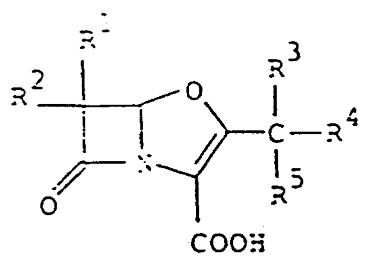

et

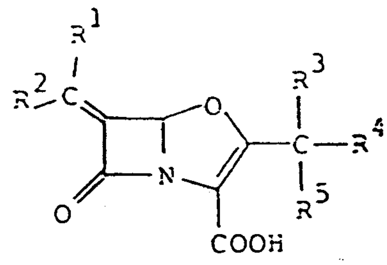

dans lesquelles $R^1$ et $R^2$ désignent indépendamment l'un de l'autre l'hydrogène ou des groupes acceptables du point de vue pharmaceutique attachés par des liaisons simples carbone-à-carbone à la partie restante de la molécule, qui sont choisis parmi des groupes, substitués ou non substitués, alkyle, alcényle, alcynyle, cycloalkyle, alkylcycloalkyle, alkylcycloalcényle, cycloalkylalkyle, alcénylcycloalkyle, cycloalcénylalkyle, aryle, aralkyle, aralcényle, aralcynyle, carboxy ou cyano, les parties alkyliques, alcényliques ou alcynyliques de molécules ci-dessus contenant 1 à 6 atomes de carbone, les parties cycloalkyliques ou les parties cycloalcényliques de molécules ayant 3 à 6 atomes de carbone et les parties aryliques de molécules contenant 6 à 10 atomes de carbone, des groupes hétéroaralkyle, hétéroaralcényle, hétéroaralcynyle, alkylhétéroaryle, hétérocyclyle, hétérocyclylalkyle, hétérocyclylalcényle, hétérocyclylalcynyle, alkylhétérocyclyle, les parties alkyliques, alcényliques ou alcynyliques de molécules ci-dessus contenant 1 à 6 atomes de carbone et la partie hétéro-aromatique ou hétérocyclique de molécule étant monocyclique ou bicyclique et contenant 3 à 10 atomes de carbone, dont l'un ou plusieurs sont choisis dans le groupe des atomes d'oxygène, de soufre et d'azote, et les substituants des groupes énumérés ci-dessus pouvant être des substituants :

hydroxy, hydroxyalkyloxy, aminoalkyloxy, amidinoalkyloxy, alkyloxy, acyloxy, aryloxy, hétérocyclyloxy, carbamoyle, carbamoyloxy, thiocarbamoyle, thiocarbamoyloxy, alkylcarbamoyloxy, alkylthiocarbamoyloxy, mercapto, alkylthio, hydroxyalkylthio, aminoalkylthio, amidinoalkylthio, acylthio, arylthio, alkylhétéroarylthio, hydroxyalkylhétéroarylthio, hétérocyclylthio, carbamoylthio, alkylcarbamoylthio, thiocarbamoylthio, alkylthiocarbamoylthio, amino ou monoalkylamino, dialkylamino, oxo, oximino ou alkylimino, tétra-alkylammonium, cycloalkylamino, arylamino, hétérocyclylamino, acylamino, amidino, alkylamidino, guanidino, alkylguanidino, carbamoylamino, alkylcarbamoylamino, thiocarbamoylamino, alkylthiocarbamoylamino, nitro, chloro, bromo, fluoro, iodo, azido, cyano, alkylsulfinyle, alkylsulfonyle, sulfonamido, sulfamoyloxy, alkylsulfonyloxy ou sulfo, sulfoxy ou carboxy, les substituants apparaissant une ou plusieurs fois indépendamment les uns des autres et leur partie alkylique de molécule contenant 1 à 6 atomes de carbone, leur partie arylique de molécule contenant 6 à 10 atomes de carbone et la partie hétérocyclique de molécule étant monocyclique ou bicyclique et contenant 3 à 10 atomes de carbone dont l'un ou plusieurs sont choisis dans le groupe des atomes d'oxygène, de soufre et d'azote, et dans lesquelles $R^3$, $R^4$ et $R^5$ sont choisis indépendamment les uns des autres parmi les groupes acceptables du point de vue pharmaceutique mentionnés ci-dessus, attachés par des liaisons simples carbone-à-carbone à la partie restante de la molécule,

caractérisé en ce qu'on fait réagir un composé de formule

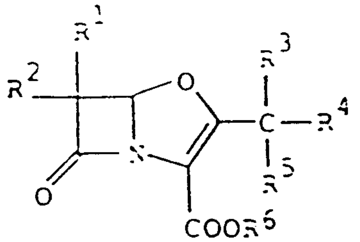

ou

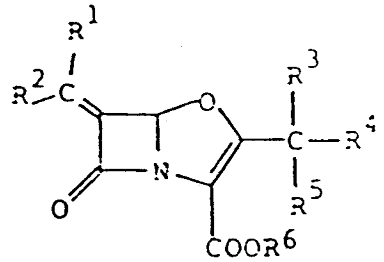

dans laquelle $R^1$ à $R^5$ ont la définition indiquée dans la revendication 1 et $R^6$ est un groupe benzyle, p-nitrobenzyle, méthyle, tertiobutyldiméthylsilyle, trichloréthyle ou pivaloyloxyméthyle, allyle, méthallyle, 2-oxo-éthyle, 2-oxo-propyle, 2-méthylthioéthyle ou butène-1-yle,

par élimination du groupe protecteur $R^6$ pour former un composé de formule (I) ou ses sels acceptables du point de vue pharmaceutique.

**2.** Procédé de production de composés de formule structurale (I) suivant la revendication 1, caractérisé en ce qu'on fait réagir une cétone de formule

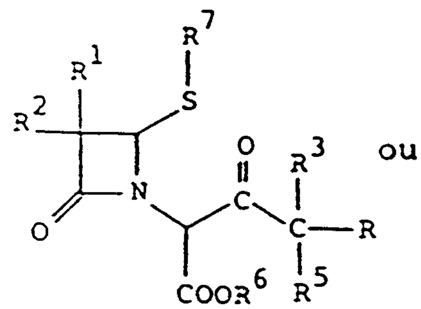

ou

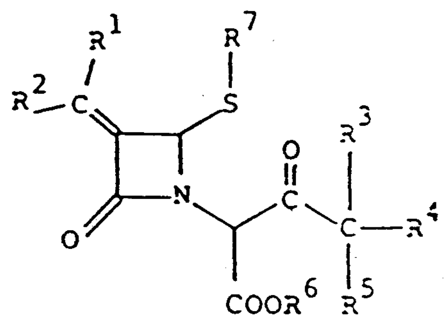

dans laquelle $R^7$ est un groupe, substitué ou non substitué, alkyle ramifié ou non ramifié, aryle, aralkyle, hétéroaryle ou hétéroaralkyle ayant jusqu'à 10 atomes de carbone, les substituants étant choisis dans le groupe des substituants alkyle inférieur, acyloxy, chloro, bromo, nitro, alkyloxy inférieur et cyano et les hétéro-atomes de la partie hétéroaryle ou hétéroaralkyle étant choisis dans le groupe des atomes d'oxygène, d'azote et de soufre, et la partie $R^6$ de la molécule d'ester ayant la définition indiquée dans la revendication 1, avec un sel de mercure (II) pour former un composé de formule

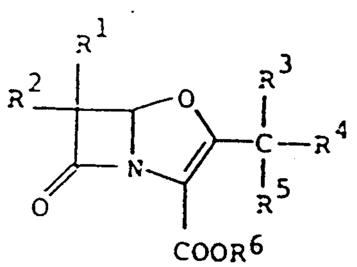

ou

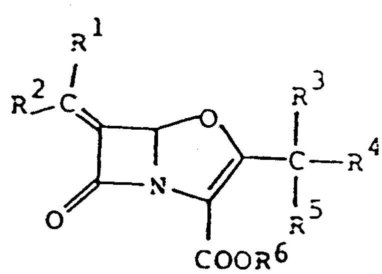

**3.** Procédé de production de composés de formule structurale (I) suivant la revendication 1, caractérisé en ce qu'on fait réagir un composé de formule

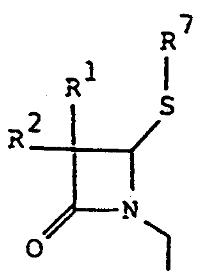

ou

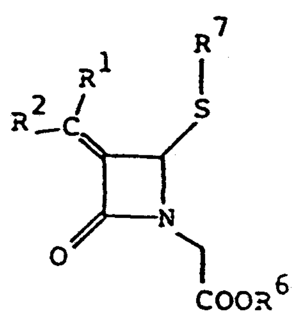

dans laquelle $R^1$ à $R^5$ ont la définition indiquée dans la revendication 1 et $R^7$ est un groupe, substitué ou non substitué, alkyle ramifié ou non ramifié, aryle, aralkyle, hétéroaryle ou hétéroaralkyle ayant jusqu'à 10 atomes de carbone, les substituants étant choisis dans le groupe des substituants alkyle inférieur, acyloxy, chloro, bromo, nitro, alkyloxy inférieur et cyano et les hétéro-atomes de la partie hétéroaryle ou hétéroaralkyle étant choisis dans le groupe dos atomes d'oxygène, d'azote et de soufre et la partie $R^6$ de la molécule d'ester étant un groupe benzyle, p-nitrobenzyle, méthyle, tertiobutyldiméthylsilyle, trichloréthyle ou pivaloyloxyméthyle, allyle, méthallyle, 2-oxo-éthyle, 2-oxo-propyle, 2-méthylthioéthyle ou butène-1-yl, avec une base forte et un halogénure d'acide de formule générale

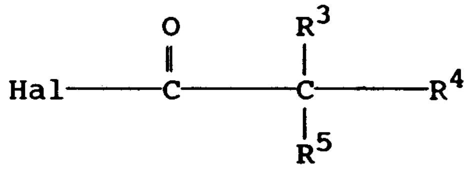

dans laquelle Hal représente le chlore, le brome ou l'iode et on obtient ainsi une cétone de formule

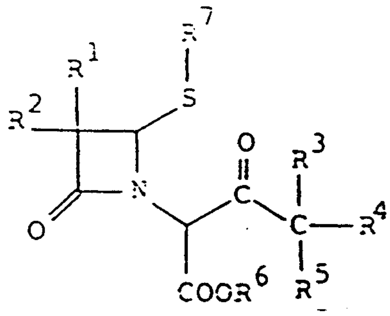

ou

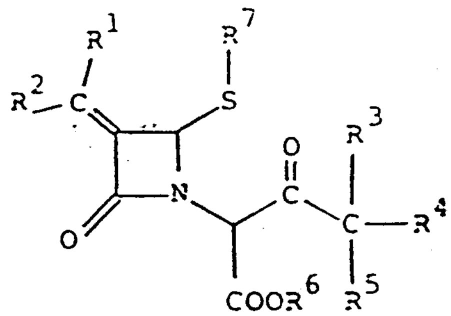

puis on fait réagir cette cétone avec un agent d'halogénation pour obtenir une cétone de formule

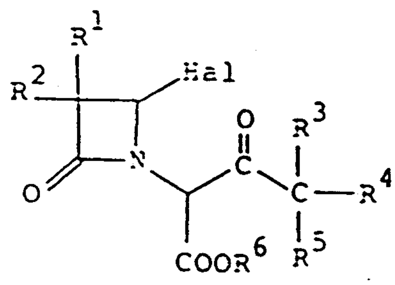

ou

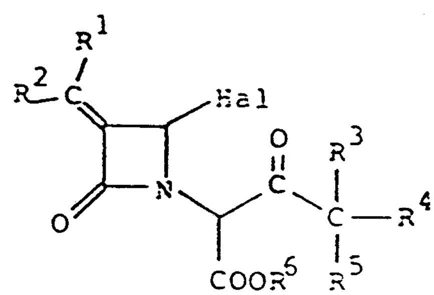

où

Hal représente le chlore, le brome ou l'iode et on fait ensuite réagir ce composé avec une base ou un sel de mercure (II) pour obtenir un composé de formule

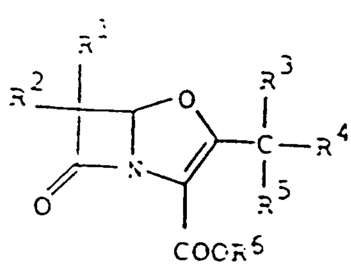

ou

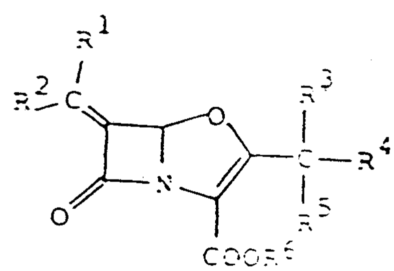

on élimine le groupe protecteur $R^6$ qui a la définition indiquée ci-dessus et on obtient un composé de formule (I) ou les sels acceptables du point de vue pharmaceutique de ce composé.